# EUROPEAN PATENT SPECIFICATION

(11) **EP 3 076 992 B1**
(45) Date of publication and mention of the grant of the patent: **06.04.2022**
(21) Application number: 14821379.6
(22) Date of filing: 05.12.2014
(51) Int. Cl.: A61K 39/00

(54) **FORMULATIONS FOR NEOPLASIA VACCINES**
ZUSAMMENSETZUNGEN VON IMPFSTOFFE GEGEN NEOPLASIE
FORMULATIONS DES VACCINS CONTRE LA NÉOPLASIE

(30) Priority: 06.12.2013 US 201361913172 P
(43) Date of publication of application: 12.10.2016
(73) Proprietor: The Broad Institute, Inc., Cambridge, MA 02142 (US)
(72) Inventor: FRITSCH, Edward, F., Concord, MA 01742 (US); NELLAIAPPAN, Kaliappanadar, Lexington, MA 02420 (US); JAVERI, Indu, North Andover, MA 01845 (US)
(74) Representative: Mathys & Squire
(86) International application number: PCT/US2014/068893
(87) International publication number: WO 2015/085233

(56) References cited:
- WO-A2-2010/045345
- WO-A2-2012/159754
- WO-A2-2014/168874
- US-B1- 6 923 973
- HACOHEN NIR ET AL: "Getting Personal with Neoantigen-Based Therapeutic Cancer Vaccines", CANCER IMMUNOLOGY RESEARCH, vol. 1, no. 1, July 2013 (2013-07), pages 11-15, XP002737816,
- MALAVOLTA LUCIANA ET AL: "Interpretation of the dissolution of insoluble peptide sequences based on the acid-base properties of the solvent.", PROTEIN SCIENCE : A PUBLICATION OF THE PROTEIN SOCIETY JUN 2006, vol. 15, no. 6, June 2006 (2006-06), pages 1476-1488, ISSN: 0961-8368

## Description

### RELATED APPLICATIONS

This application claims benefit of and priority to US provisional patent application Serial No. 61/913,172, filed December 6, 2013.

### FIELD OF THE INVENTION

The present invention relates to formulations for the treatment of neoplasia. More particularly, the present invention relates to the formulations for tumor vaccines for treatment of neoplasia in a subject.

### BACKGROUND OF THE INVENTION

Approximately 1.6 million Americans are diagnosed with neoplasia every year, and approximately 580,000 people in the United States are expected to die of the disease in 2013. Over the past few decades there been significant improvements in the detection, diagnosis, and treatment of neoplasia, which have significantly increased the survival rate for many types of neoplasia. However, only about 60% of people diagnosed with neoplasia are still alive 5 years after the onset of treatment, which makes neoplasia the second leading cause of death in the United States.

Currently, there are a number of different existing cancer therapies, including ablation techniques (e.g., surgical procedures, cryogenic/heat treatment, ultrasound, radiofrequency, and radiation) and chemical techniques (e.g., pharmaceutical agents, cytotoxic/chemotherapeutic agents, monoclonal antibodies, and various combinations thereof). Unfortunately, such therapies are frequently associated with serious risk, toxic side effects, and extremely high costs, as well as uncertain efficacy.

There is a growing interest in cancer therapies that seek to target cancerous cells with a patient's own immune system (e.g., cancer vaccines) because such therapies may mitigate/eliminate some of the herein-described disadvantages. Cancer vaccines are typically composed of tumor antigens and immunostimulatory molecules (e.g., cytokines or TLR ligands) that work together to induce antigen-specific cytotoxic T cells that target and destroy tumor cells. Current cancer vaccines typically contain shared tumor antigens, which are native proteins (i.e. - proteins encoded by the DNA of all the normal cells in the individual) that are selectively expressed or over-expressed in tumors found in many individuals. While such shared tumor antigens are useful in identifying particular types of tumors, they are not ideal as immunogens for targeting a T-cell response to a particular tumor type because they are subject to the immune dampening effects of self-tolerance. Vaccines containing tumor-specific and patient-specific neoantigens can overcome some of the disadvantages of vaccines containing shared tumor antigens.

In general, any vaccine should have a shelf-life long enough to ensure that the vaccine will not degrade or deteriorate before use. Storage stability also requires that the components of the vaccine should not precipitate from solution during storage. However, achieving adequate storage stability can be difficult. Accordingly, new formulations for vaccines are needed.

WO 2010/045345 A2 relates to therapies and pharmaceutical compositions for the treatment of pathological conditions, such as cancer. Luciana et al. (2006) Protein Sci Jun; 15(6); 1476-1488 relates to a strategy for the dissolution of insoluble peptides.

### SUMMARY OF THE INVENTION

Based on the disclosure that it contained herein, the present invention provides a pharmaceutical composition comprising: (a) at least one neo-antigenic peptide or a pharmaceutically acceptable salt thereof, wherein the at least one neo-antigenic peptide ranges from 5 to 50 amino acids in length; (b) a pH modifier that is a base, wherein the pH modifier is succinate or citrate and wherein the pH modifier is present in the composition at a concentration from 1 mM to 10 mM: and (c) a pharmaceutically acceptable carrier comprising i. water: ii. dextrose, sucrose or trehalose; and iii. 1-4% dimethylsulfoxide.

In another aspect, the present invention provides a method of preparing a neo-antigenic peptide solution for a neoplasia vaccine, the method comprising: (a) preparing a solution comprising at least one neo-antigenic peptide or a pharmaceutically acceptable salt thereof, wherein the at least one neo-antigenic peptide ranges from 5 to 50 amino acids in length; and (b) combining the solution comprising at least one neo-antigenic peptide or a pharmaceutically acceptable salt thereof with a solution comprising a pharmaceutically acceptable salt of succinic acid or citric acid , thereby preparing a peptide solution for a neoplasia vaccine, wherein the pharmaceutically acceptable salt of succinic acid or citric acid is present in the peptide solution at a concentration from 1 mM to 10 mM and wherein the peptide solution comprises water; dextrose, sucrose or trehalose; and 1-4% dimethylsulfoxide; and preferably (i) wherein the solution comprising at least one neo-antigenic peptide or a pharmaceutically acceptable salt thereof comprises at least two or at least three, or four, or five neo-antigenic peptides: (ii) wherein the peptide solution for a neoplasia vaccine comprises water, dextrose. 1 mM to 10 mM succinate, and 1-4% dimethylsulfoxide: or (iii) further comprising, after the step of combining, filtering the peptide solution for a neoplasia vaccine: and preferably wherein the peptide solution for a neoplasia vaccine is lyophilizable.

In a further aspect the present invention provides a method of preparing a neoplasia vaccine, the method comprising: (a) preparing a neo-antigenic peptide solution in accordance with the invention: and (b) combining the neo-antigenic peptide solution with a solution of an immunomodulator or adjuvant thereby preparing a neoplasia vaccine, preferably wherein the immunomodulator or adjuvant is selected from the group consisting of 1018 ISS, aluminum salts. Amplivax, AS15, BCG. CP-870,893, CpG7909, CyaA, dSLIM, GM-CSF, IC30. IC31. Imiquimod. ImuFact IMP321, IS Patch. ISS, ISCOMATRIX, JuvImmune, LipoVac, MF59, monophosphoryl lipid A, Montanide IMS 1312. Montanide ISA 206. Montanide ISA 50V, Montanide ISA-51, OK-432, OM-174, OM-197-MPEC, ONTAK, PepTel^{®}, vector system. PLGA microparticles, resiquimod, SRL172, Virosomes and other Virus-like particles. YF-17D. VEGF trap, R848, beta-glucan, Pam3Cys, and Aquila's QS21 stimulon.

In a yet further aspect, the present invention provides a pharmaceutical composition according to the invention for use in treating a neoplasia, wherein said treating comprises administering the pharmaceutical composition to a subject diagnosed as having a neoplasia, preferably further comprising administering a second, third or fourth pharmaceutical composition according to the invention to the subject.

In a yet further aspect, the present invention provides a vaccination or immunization kit comprising: (a) a composition according to the invention, wherein the composition is separately packaged and freeze-dried: and (b) a solution for the reconstitution of the freeze-dried composition, preferably wherein the solution contains an adjuvant.

### The present invention and embodiments thereof are set out in the appended claims.

The present invention relates to neoplasia vaccines or immunogenic compositions for the treatment of neoplasia, and more particularly to the vaccine formulations comprising a pool of tumor-specific and patient-specific neo-antigens for the treatment of tumors in a subject.

In certain embodiments, the pharmaceutical composition is a vaccine composition.

In certain embodiments, the pharmaceutical composition comprises at least two neoantigenic peptides. In certain embodiments, the pharmaceutical composition comprises at least three neo-antigenic peptides. In certain embodiments, the pharmaceutical composition comprises at least four neo-antigenic peptides. In certain embodiments, the pharmaceutical composition comprises at least five neo-antigenic peptides. The neoplasia vaccine or immunogenic composition advantageously comprises at least four different neoantigens (and by different antigens it is intended that each antigen has a different neoepitope), e.g., at least 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or more different neoantigens can be in the neoplasia vaccine or immunogenic composition.

According to the invention, the neoantigenic peptide ranges from 5 to 50 amino acids in length. In another related embodiment, the neoantigenic peptide ranges from about 15 to about 35 amino acids in length. Typically, the length is greater than about 15 or 20 amino acids, e.g., from 15 to 50 or about 75 amino acids.

In one instance , the neoplasia vaccine or immunogenic composition further comprises a pH modifier and a pharmaceutically acceptable carrier.

According to the invention, the pH modifier is a base. In certain instances, the pH modifier is a dicarboxylate or tricarboxylate salt. According to the invention, the pH modifier is succinate. or citrate.

In certain instances , the succinic acid or a pharmaceutically acceptable salt thereof comprises di sodium succinate.

In certain instances, succinate is present in the formulation at a concentration from about 1 mM to about 10 mM. In certain embodiments, succinate is present in the formulation at a concentration of about 2 mM to about 5 mM.

According to the invention, the pharmaceutically acceptable carrier comprises water. According to the invention, the pharmaceutically acceptable carrier comprises dextrose, sucrose or trehalose.

In certain embodiments, the pharmaceutically acceptable carrier comprises dextrose.

In certain embodiments, the pharmaceutically acceptable carrier comprises trehalose

In certain embodiments, the pharmaceutically acceptable carrier comprises sucrose.

According to the invention, the pharmaceutically acceptable carrier comprises dimethylsulfoxide.

In certain embodiments, the pharmaceutical composition further comprises an immunomodulator or adjuvant. In one instance , the method further comprises administration of an immunomodulator or adjuvant. In another related embodiment, the immunomodulator or adjuvant is selected from the group consisting of poly-ICLC, 1018 ISS, aluminum salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, JuvImmune, LipoVac, MF59, monophosphoryl lipid A, Montanide IMS 1312, Montanide ISA 206, Montanide ISA 50V, Montanide ISA-51, OK-432, OM-174, OM-197-MP-EC, ONTAK, PEPTEL, vector system, PLGA microparticles, resiquimod, SRL172, Virosomes and other Virus-like particles, YF-17D, VEGF trap, R848, beta-glucan, Pam3Cys, and Aquila's QS21 stimulon. In another further embodiment, the immunomodulator or adjuvant is poly-ICLC.

The dissolution of these polymers in water leads to an acid solution which is neutralized, preferably to physiological pH, in order to give the adjuvant solution into which the vaccine or immunogenic composition or antigen(s) or vector(s) thereof is incorporated. The carboxyl groups of the polymer are then partly in COO⁻.

Preferably, a solution of adjuvant according to the invention, especially of carbomer, is prepared in distilled water, preferably in the presence of sodium chloride, the solution obtained being at acidic pH. This stock solution is diluted by adding it to the required quantity (for obtaining the desired final concentration), or a substantial part thereof, of water charged with salt such as NaCl, preferably physiological saline (NaCl 9 g/l), all at once or in several portions with concomitant or subsequent neutralization (pH 7.3 to 7.4), preferably with a base such as NaOH. This solution at physiological pH is used as is to reconstitute the vaccine, especially stored in freeze-dried or lyophilized form.

The polymer concentration in the final vaccine composition is 0.01% to 2% w/v, more particularly 0.06 to 1% w/v, preferably 0.1 to 0.6% w/v.

In another embodiment, the invention provides a pharmaceutical composition which is a neoplasia vaccine, comprising: one to five neo-antigenic peptides or pharmaceutically acceptable salts thereof; 1-3% dimethylsulfoxide; 3.6- 3.7 % dextrose in water; 3.6- 3.7mM succinate acid or a salt thereof; 0.5 mg/ml poly I: poly C; 0.375 mg/ml poly-L-Lysine; 1.25 mg/ml sodium carboxymethylcellulose; and 0.225% sodium chloride. In certain embodiments, each of the one to five neo-antigenic peptides or pharmaceutically acceptable salts thereof are each present at a concentration of about 300 µg/ml.

In another instance, the disclosure provides a method of preparing a neo-antigenic peptide solution for a neoplasia vaccine, the method comprising: providing a solution comprising at least one neo-antigenic peptide or a pharmaceutically acceptable salt thereof; and combining the solution comprising at least one neo-antigenic peptide or a pharmaceutically acceptable salt thereof with a solution comprising succinic acid or a pharmaceutically acceptable salt thereof, thereby preparing a peptide solution for a neoplasia vaccine.

In certain embodiments, the solution comprising at least one neo-antigenic peptide or a pharmaceutically acceptable salt thereof comprises at least two (or 3, or 4, or 5) neo-antigenic peptides. In certain embodiments, the peptide solution for a neoplasia vaccine comprises water, dextrose or trehalose or sucrose, succinate, and dimethylsulfoxide. In certain embodiments, the method further comprises, after the step of combining, filtering the peptide solution for a neoplasia vaccine.

In another instance, the disclosure provides a method of preparing a neoplasia vaccine, the method comprising: providing a peptide solution for a neoplasia vaccine; and combining the peptide solution with a solution of an immunodulator or adjuvant, thereby preparing a neoplasia vaccine.

In another instance, the disclosure provides a neoplasia vaccine made by any method described herein (e.g., the method described above).

In another instance, the disclosure provides a neo-antigenic peptide solution for a neoplasia vaccine, comprising: at least one neo-antigenic peptide or a pharmaceutically acceptable salt thereof; and succinic acid or a pharmaceutically acceptable salt thereof.

In another instance, the disclosure provides a method of treating a subject diagnosed as having a neoplasia, the method comprising: administering a pharmaceutical composition of the invention to the subject, thereby treating the neoplasia.

In certain instances, the method further comprises administering a second pharmaceutical composition of the invention to the subject.

In certain instances, the method further comprises administering a third pharmaceutical composition of the invention to the subject.

In certain instances, the method further comprises administering a fourth pharmaceutical composition of the invention to the subject.

The administering of the neoplasia vaccine or immunogenic composition can be on one time schedule, e.g., weekly, biweekly, every three weeks, monthly, bimonthly, every quarter year (every three months), every third of a year (every four months), every five months, twice yearly (every six months), every seven months, every eight months, every nine months, every ten months, every eleven months, annually or the like.

The neoplasia vaccine or immunogenic composition can be administered via subcompositions, each containing a portion of the neoantigens, and sub-compositions can be administered to different places on the subject or patient; for instance, a composition comprising 20 different neoantigens, can be administered in four (4) subcompositions, each containing 5 of the 20 different neoantigens, and the four (4) subcompositions can be administered so as to endeavor to deliver each subcomposition at or near a draining lymph node of the patient, e.g., to each of the arms and legs (e.g., thigh or upper thigh or near buttocks or lower back on each side of the patient) so as to endeavor to deliver each subcomposition at or near a draining lymph node of the patient or subject. Of course, the number of locations and hence number of subcompositions can vary, e.g., the skilled practitioner could consider administration at or near the spleen to have a fifth point of administration, and the skilled practitioner can vary the locations such that only one, two or three are used (e.g., each arm and a leg, each of legs and one arm, each of the legs and no arms, or only both arms).

The vaccine or immunogenic composition administered at the aforementioned various intervals can be different formulations, and the subcompositions administered at different places on the subject or patient during a single administration can be different compositions. For instance, a first administration can be of a whole antigen vaccine or immunogenic composition and a next or later administration can be of a vector (e.g., viral vector or plasmid) that has expression of antigen(s) in vivo. Likewise, in the administration of different subcompositions to different locations on the patient or subject, some of the subcompositions can comprise a whole antigen and some of the subcompositions can comprise a vector (e.g., viral vector or plasmid) that has expression of antigen(s) in vivo. And some compositions and subcompositions can comprise both vector(s) (e.g., viral vector or plasmid) that has / have expression of antigen(s) in vivo and whole antigens. Some vectors (e.g., poxvirus) that have expression of antigen(s) in vivo can have an immunostimulatory or adjuvanting effect, and hence compositions or subcompositions that contain such vectors can be self-adjuvanting. Also, by changing up the nature of how the antigens are presented to the immune system, the administrations can "prime" and then "boost" the immune system. And in this text, when there is mention of a "vaccine" it is intended that the invention comprehends immunogenic compositions, and when there is mention of a patient or subject it is intended that such an individual is a patient or subject in need of the herein disclosed treatments, administrations, compositions, and generally the subject invention.

Moreover, the invention applies to the use of any type of expression vector, such as a viral expression vector, e.g., poxvirus (e.g., orthopoxvirus or avipoxvirus such as vaccinia virus, including Modified Vaccinia Ankara or MVA, MVA-BN, NYVAC according to WO-A-92/15672, fowlpox, e.g., TROVAX, canarypox, e.g., ALVAC (WO-A-95/27780 and WO-A-92/15672) pigeonpox, swinepox and the like), adenovirus, AAV herpesvirus, and lentivirus; or a plasmid or DNA or nucleic acid molecule vector. Some vectors that are cytoplasmic, such as poxvirus vectors, may be advantageous. However adenovirus, AAV and lentivirus can also be advantageous to use in the practice of the invention.

In a ready-for-use, especially reconstituted, vaccine or immunogenic composition, the vector, e.g., viral vector, is present in the quantities within the ambit of the skilled person from this disclosure and the knowledge in the art (such as in patent and scientific literature cited herein).

Whole antigen or vector, e.g., recombinant live vaccines generally exist in a freeze-dried form allowing their storage and are reconstituted immediately before use in a solvent or excipient, which can include an adjuvant as herein discussed.

The subject of the disclosure is therefore also a vaccination or immunization set or kit comprising, packaged separately, freeze-dried vaccine and a solution, advantageously including an adjuvant compound as herein discussed for the reconstitution of the freeze-dried vaccine.

The subject of the disclosure is also a method of vaccination or immunization comprising or consisting essentially of or consisting of administering, e.g., by the parenteral, preferably subcutaneous, intramuscular or intradermal, route or by the mucosal route a vaccine or immunogenic composition in accordance with the invention at the rate of one or more administrations. Optionally this method includes a preliminary step of reconstituting the freeze-dried vaccine or immunogenic composition (e.g., if lyophilized whole antigen or vector) in a solution, advantageously also including an adjuvant.

In one embodiment, the subject is suffering from a neoplasia selected from the group consisting of: Non-Hodgkin's Lymphoma (NHL), clear cell Renal Cell Carcinoma (ccRCC), melanoma, sarcoma, leukemia or a cancer of the bladder, colon, brain, breast, head and neck, endometrium, lung, ovary, pancreas or prostate. In another embodiment, the neoplasia is metastatic. In a further embodiment, the subject has no detectable neoplasia but is at high risk for disease recurrence. In a further related embodiment, the subject has previously undergone autologous hematopoietic stem cell transplant (AHSCT).

In one embodiment, administration of the neoplasia vaccine or immunogenic composition is in a prime/ boost dosing regimen. In another embodiment, administration of the neoplasia vaccine or immunogenic composition is at weeks 1, 2, 3 or 4 as a prime. In another further embodiment, administration of the neoplasia vaccine or immunogenic composition is at months 2, 3, 4 or 5 as a boost.

In one embodiment, the vaccine or immunogenic composition is administered at a dose of about 10 µg- 1 mg per 70 kg individual as to each neoantigenic peptide. In another embodiment, the vaccine or immunogenic composition is administered at an average weekly dose level of about 10 µg- 2000 µg per 70 kg individual as to each neoantigenic peptide.

In one embodiment, the vaccine or immunogenic composition is administered intravenously or subcutaneously.

In another instance, the disclosure provides a neo-antigenic peptide solution for a neoplasia vaccine, comprising: at least one neo-antigenic peptide or a pharmaceutically acceptable salt thereof; and succinic acid or a pharmaceutically acceptable salt thereof.

The invention comprehends performing methods as in U.S. patent application No. 20110293637 e.g., a method of identifying a plurality of at least 4 subject-specific peptides and preparing a subject-specific immunogenic composition that upon administration presents the plurality of at least 4 subject-specific peptides to the subject's immune system, wherein the subject has a tumor and the subject-specific peptides are specific to the subject and the subject's tumor, said method comprising:
(i) identifying, including through
   nucleic acid sequencing of a sample of the subject's tumor and
   nucleic acid sequencing of a non-tumor sample of the subject,
   a plurality of at least 4 tumor-specific non-silent mutations not present in the non-tumor sample; and
(ii) selecting from the identified non-silent mutations the plurality of at least 4 subject-specific peptides, each having a different tumor neo-epitope that is an epitope specific to the tumor of the subject, from the identified plurality of tumor specific mutations,
   wherein each neo-epitope is an expression product of a tumor-specific non-silent mutation not present in the non-tumor sample, each neo-epitope binds to a HLA protein of the subject, and selecting includes
   determining binding of the subject-specific peptides to the HLA protein, and
(iii) formulating the subject-specific immunogenic composition for administration to the subject so that upon administration the plurality of at least 4 subject-specific peptides are presented to the subject's immune system,
   wherein the selecting or formulating comprises at least one of:
   including in the subject-specific immunogenic composition a subject-specific peptide that includes an expression product of an identified neo-ORF, wherein a neo-ORF is a tumor-specific non-silent mutation not present in the non-tumor sample that creates a new open reading frame, and
   including in the subject-specific immunogenic composition a subject-specific peptide that includes an expression product of an identified point mutation and has a determined binding to the HLA protein of the subject with an IC50 less than 500 nM, whereby, the plurality of at least 4 subject-specific peptides are identified, and the subject-specific immunogenic composition that upon administration presents the plurality of at least 4 subject-specific peptides to the subject's immune system, wherein the subject-specific peptides are specific to the subject and the subject's tumor, is prepared; or a method of identifying a neoantigen comprising:
      a. identifying a tumor specific mutation in an expressed gene of a subject having cancer;
      b. wherein when said mutation identified in step (a) is a point mutation:
         i. identifying a mutant peptide having the mutation identified in step (a), wherein said mutant peptide binds to a class I HLA protein with a greater affinity than a wild-type peptide; and has an IC50 less than 500 nm;
      c. wherein when said mutation identified in step (a) is a splice-site, frameshift, read-through or gene-fusion mutation:
         i. identifying a mutant polypeptide encoded by the mutation identified in step (a), wherein said mutant polypeptide binds to a class I HLA protein; or a method of inducing a tumor specific immune response in a subject comprising administering one or more peptides or polypeptides identified and an adjuvant; or a method of vaccinating or treating a subject for cancer comprising:
            a. identifying a plurality of tumor specific mutations in an expressed gene of the subject wherein when said mutation identified is a:
               i. point mutation further identifying a mutant peptide having the point mutation; and/or
               ii. splice-site, frameshift, read-through or gene-fusion mutation further identifying a mutant polypeptide encoded by the mutation;
            b. selecting one or more mutant peptides or polypeptides identified in step (a) that binds to a class I HLA protein;
            c. selecting the one or more mutant peptides or polypeptides identified in step (b) that is capable of activating anti-tumor CD8 T-cells; and
            d. administering to the subject the one or more peptides or polypeptides, autologous dendritic cells or antigen presenting cells pulsed with the one or more peptides or polypeptides selected in step (c); or preparing a pharmaceutical composition comprising one identified peptide(s), and performing method(s) as herein discussed. Thus, the neoplasia vaccine or immunogenic composition herein can be as in U.S. patent application No. 20110293637.

Accordingly, it is an object of the invention to not encompass within the invention any previously known product, process of making the product, or method of using the product such that Applicants reserve the right and hereby disclose a disclaimer of any previously known product, process, or method. It is further noted that the invention does not intend to encompass within the scope of the invention any product, process, or making of the product or method of using the product, which does not meet the written description and enablement requirements of the USPTO (35 U.S.C. §112, first paragraph) or the EPO (Article 83 of the EPC), such that Applicants reserve the right and hereby disclose a disclaimer of any previously described product, process of making the product, or method of using the product.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

### BRIEF DESCRIPTION OF THE DRAWINGS

The patent or application file contains at least one drawing executed in color. Copies of this patent or patent application publication with color drawing(s) is provided by the Office upon request and payment of the necessary fee.

The following detailed description, given by way of example, but not intended to limit the invention solely to the specific embodiments described, may best be understood in conjunction with the accompanying drawings, wherein:

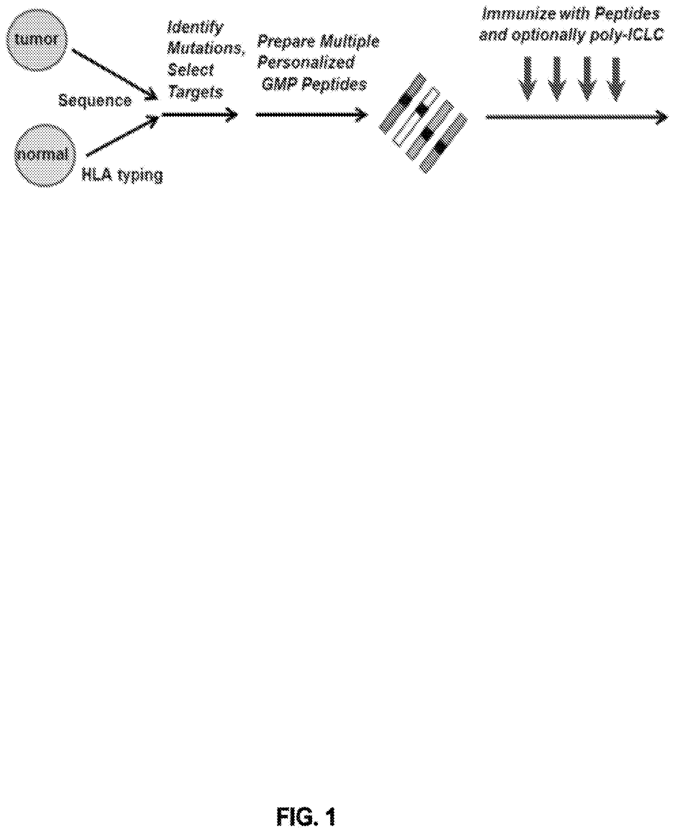

Figure 1 depicts a flow process for making a personalized cancer vaccine or immunogenic composition.
Figure 2 shows a flow process for pre-treatment steps for generating a cancer vaccine or immunogenic composition for a cancer patient.
Figure 3 illustrates an immunization schedule based on a prime boost strategy according to an exemplary embodiment of the present invention. Multiple immunizations may occur over the first -3 weeks to maintain an early high antigen exposure during the priming phase of immune response. Patients may then be rested for eight weeks to allow memory T cells to develop and these T cells will then be boosted in order to maintain a strong ongoing response.
Figure 4 shows a time line indicating the primary immunological endpoint according to an exemplary aspect of the invention.
Figure 5 shows a schematic depicting drug product processing of individual neoantigenic peptides into pools of 4 subgroups according to an exemplary embodiment of the invention.
Figure 6 shows the results of quantitative PCR to assess the levels of induction of a number of key immune markers after stimulation of mouse dendritic cells using a neoantigenic formulation.
Figure 7 shows MDSC analysis of 5% Dextrose and 0.8% DMSO.
Figure 8 shows MDSC analysis of 10% Trehalose and 0.8% DMSO.
Figure 9 shows MDSC analysis of 10% Sucrose and 0.8% DMSO.
Figure 10 shows the pressure profile of an exemplary lyophilization.
Figure 11 shows the temperature profile of an exemplary lyophilization.
Figure 12 shows the physical appearance of lyophilized cake using exemplary formulations of the disclosure .

### DETAILED DESCRIPTION OF THE INVENTION

### Definitions

To facilitate an understanding of the present invention, a number of terms and phrases are defined herein:

Unless specifically stated or obvious from context, as used herein, the term "about" is understood as within a range of normal tolerance in the art, for example within 2 standard deviations of the mean. About can be understood as within 50%, 45%, 40%, 35%, 30%, 25%, 20%, 15%, 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

Unless specifically stated or obvious from context, as used herein, the term "or" is understood to be inclusive. Unless specifically stated or obvious from context, as used herein, the terms "a," "an," and "the" are understood to be singular or plural.

By "agent" is meant any small molecule chemical compound, antibody, nucleic acid molecule, or polypeptide, or fragments thereof.

By "ameliorate" is meant decrease, suppress, attenuate, diminish, arrest, or stabilize the development or progression of a disease (e.g., a neoplasia, tumor, etc.).

By "alteration" is meant a change (increase or decrease) in the expression levels oractivity of a gene or polypeptide as detected by standard art known methods such as those described herein. As used herein, an alteration includes a 10% change in expression levels, preferably a 25% change, more preferably a 40% change, and most preferably a 50% or greater change in expression levels.

By "analog" is meant a molecule that is not identical, but has analogous functional or structural features. For example, a tumor specific neo-antigen polypeptide analog retains the biological activity of a corresponding naturally-occurring tumor specific neo-antigen polypeptide, while having certain biochemical modifications that enhance the analog's function relative to a naturally-occurring polypeptide. Such biochemical modifications could increase the analog's protease resistance, membrane permeability, or half-life, without altering, for example, ligand binding. An analog may include an unnatural amino acid.

The term "neoantigen" or "neoantigenic" means a class of tumor antigens that arises from a tumor-specific mutation(s) which alters the amino acid sequence of genome encoded proteins.

By "neoplasia" is meant any disease that is caused by or results in inappropriately high levels of cell division, inappropriately low levels of apoptosis, or both. For example, cancer is an example of a neoplasia. Examples of cancers include, without limitation, leukemia (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (e.g., Hodgkin's disease, non-Hodgkin's disease), Waldenstrom's macroglobulinemia, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, nile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodenroglioma, schwannoma, meningioma, melanoma, neuroblastoma, and retinoblastoma). Lymphoproliferative disorders are also considered to be proliferative diseases.

The term "neoplasia vaccine" is meant to refer to a pooled sample of neoplasia/tumor specific neoantigens, for example at least two, at least three, at least four, at least five, or more neoantigenic peptides. A "vaccine" is to be understood as meaning a composition for generating immunity for the prophylaxis and/or treatment of diseases (e.g., neoplasia/tumor). Accordingly, vaccines are medicaments which comprise antigens and are intended to be used in humans or animals for generating specific defense and protective substance by vaccination. A "neoplasia vaccine composition " can include a pharmaceutically acceptable excipient, carrier or diluent.

The term "pharmaceutically acceptable" refers to approved or approvable by a regulatory agency of the Federal or a state government or listed in the U.S. Pharmacopeia or other generally recognized pharmacopeia for use in animals, including humans.

A "pharmaceutically acceptable excipient, carrier or diluent" refers to an excipient, carrier or diluent that can be administered to a subject, together with an agent, and which does not destroy the pharmacological activity thereof and is nontoxic when administered in doses sufficient to deliver a therapeutic amount of the agent.

A "pharmaceutically acceptable salt" of pooled tumor specific neoantigens as recited herein may be an acid or base salt that is generally considered in the art to be suitable for use in contact with the tissues of human beings or animals without excessive toxicity, irritation, allergic response, or other problem or complication. Such salts include mineral and organic acid salts of basic residues such as amines, as well as alkali or organic salts of acidic residues such as carboxylic acids. Specific pharmaceutical salts include, but are not limited to, salts of acids such as hydrochloric, phosphoric, hydrobromic, malic, glycolic, fumaric, sulfuric, sulfamic, sulfanilic, formic, toluenesulfonic, methanesulfonic, benzene sulfonic, ethane disulfonic, 2-hydroxyethylsulfonic, nitric, benzoic, 2-acetoxybenzoic, citric, tartaric, lactic, stearic, salicylic, glutamic, ascorbic, pamoic, succinic, fumaric, maleic, propionic, hydroxymaleic, hydroiodic, phenylacetic, alkanoic such as acetic, HOOC-(CH2)n-COOH where n is 0-4, and the like. Similarly, pharmaceutically acceptable cations include, but are not limited to sodium, potassium, calcium, aluminum, lithium and ammonium. Those of ordinary skill in the art will recognize from this disclosure and the knowledge in the art that further pharmaceutically acceptable salts for the pooled tumor specific neoantigens provided herein, including those listed by Remington's Pharmaceutical Sciences, 17th ed., Mack Publishing Company, Easton, PA, p. 1418 (1985). In general, a pharmaceutically acceptable acid or base salt can be synthesized from a parent compound that contains a basic or acidic moiety by any conventional chemical method. Briefly, such salts can be prepared by reacting the free acid or base forms of these compounds with a stoichiometric amount of the appropriate base or acid in an appropriate solvent.

By a "polypeptide" or "peptide" is meant a polypeptide that has been separated from components that naturally accompany it. Typically, the polypeptide is isolated when it is at least 60%, by weight, free from the proteins and naturally-occurring organic molecules with which it is naturally associated. Preferably, the preparation is at least 75%, more preferably at least 90%, and most preferably at least 99%, by weight, a polypeptide. An isolated polypeptide may be obtained, for example, by extraction from a natural source, by expression of a recombinant nucleic acid encoding such a polypeptide; or by chemically synthesizing the protein. Purity can be measured by any appropriate method, for example, column chromatography, polyacrylamide gel electrophoresis, or by HPLC analysis.

As used herein, the terms "prevent," "preventing," "prevention," "prophylactic treatment," and the like, refer to reducing the probability of developing a disease or condition in a subject, who does not have, but is at risk of or susceptible to developing a disease or condition.

The term "prime/ boost" or "prime/ boost dosing regimen" is meant to refer to the successive administrations of a vaccine or immunogenic or immunological compositions. The priming administration (priming) is the administration of a first vaccine or immunogenic or immunological composition type and may comprise one, two or more administrations. The boost administration is the second administration of a vaccine or immunogenic or immunological composition type and may comprise one, two or more administrations, and, for instance, may comprise or consist essentially of annual administrations. In certain embodiments, administration of the neoplasia vaccine or immunogenic composition is in a prime/ boost dosing regimen.

Ranges provided herein are understood to be shorthand for all of the values within the range. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50, as well as all intervening decimal values between the aforementioned integers such as, for example, 1.1, 1.2, 1.3, 1.4, 1.5, 1.6, 1.7, 1.8, and 1.9. With respect to sub-ranges, "nested sub-ranges" that extend from either end point of the range are specifically contemplated. For example, a nested sub-range of an exemplary range of 1 to 50 may comprise 1 to 10, 1 to 20, 1 to 30, and 1 to 40 in one direction, or 50 to 40, 50 to 30, 50 to 20, and 50 to 10 in the other direction.

A "receptor" is to be understood as meaning a biological molecule or a molecule grouping capable of binding a ligand. A receptor may serve, to transmit information in a cell, a cell formation or an organism. The receptor comprises at least one receptor unit and frequently contains two or more receptor units, where each receptor unit may consist of a protein molecule, in particular a glycoprotein molecule. The receptor has a structure that complements the structure of a ligand and may complex the ligand as a binding partner. Signaling information may be transmitted by conformational changes of the receptor following binding with the ligand on the surface of a cell. According to the disclosure, a receptor may refer to particular proteins of MHC classes I and II capable of forming a receptor/ligand complex with a ligand, in particular a peptide or peptide fragment of suitable length.

A "receptor/ligand complex" is also to be understood as meaning a "receptor/peptide complex" or "receptor/peptide fragment complex," in particular a peptide- or peptide fragment-presenting MHC molecule of class I or of class II.

By "reduces" is meant a negative alteration of at least 10%, 25%, 50%, 75%, or 100%.

By "reference" is meant a standard or control condition.

A "reference sequence" is a defined sequence used as a basis for sequence comparison. A reference sequence may be a subset of, or the entirety of, a specified sequence; for example, a segment of a full-length cDNA or genomic sequence, or the complete cDNA or genomic sequence. For polypeptides, the length of the reference polypeptide sequence will generally be at least about 10-2,000 amino acids, 10-1,500, 10-1,000, 10-500, or 10-100. Preferably, the length of the reference polypeptide sequence may be at least about 10-50 amino acids, more preferably at least about 10-40 amino acids, and even more preferably about 10-30 amino acids, about 10-20 amino acids, about 15-25 amino acids, or about 20 amino acids. For nucleic acids, the length of the reference nucleic acid sequence will generally be at least about 50 nucleotides, preferably at least about 60 nucleotides, more preferably at least about 75 nucleotides, and even more preferably about 100 nucleotides or about 300 nucleotides or any integer thereabout or there between.

By "specifically binds" is meant a compound or antibody that recognizes and binds a polypeptide, but which does not substantially recognize and bind other molecules in a sample, for example, a biological sample.

Nucleic acid molecules useful in the methods of the invention include any nucleic acid molecule that encodes a polypeptide of the disclosure or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double-stranded nucleic acid molecule. By "hybridize" is meant pair to form a double-stranded molecule between complementary polynucleotide sequences (e.g., a gene described herein), or portions thereof, under various conditions of stringency. (See, e.g., Wahl, G. M. and S. L. Berger (1987) Methods Enzymol. 152:399; Kimmel, A. R. (1987) Methods Enzymol. 152:507).

For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30°C, more preferably of at least about 37°C, and most preferably of at least about 42°C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred: instance , hybridization will occur at 30°C in 750 mM NaCl, 75 mM trisodium citrate, and 1% SDS. In a more preferred instance , hybridization will occur at 37° C in 500 mM NaCl, 50 mM trisodium citrate, 1% SDS, 35% formamide, and 100 µg/ml denatured salmon sperm DNA (ssDNA). In a most preferred instance , hybridization will occur at 42°C in 250 mM NaCl, 25 mM trisodium citrate, 1% SDS, 50% formamide, and 200 µg/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25°C, more preferably of at least about 42°C, and even more preferably of at least about 68°C. In a preferred instance , wash steps will occur at 25°C in 30 mM NaCl, 3 mM trisodium citrate, and 0.1% SDS. In a more preferred instance , wash steps will occur at 42°C in 15 mM NaCl, 1.5 mM trisodium citrate, and 0.1% SDS. In a more preferred instance , wash steps will occur at 68°C in 15 mM NaCl, 1.5 mM trisodium citrate, and 0.1% SDS. Additional variations on these conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

The term "subject" refers to an animal which is the object of treatment, observation, or experiment. By way of example only, a subject includes, but is not limited to, a mammal, including, but not limited to, a human or a non-human mammal, such as a non-human primate, bovine, equine, canine, ovine, or feline.

By "substantially identical" is meant a polypeptide or nucleic acid molecule exhibiting at least 50% identity to a reference amino acid sequence (for example, any one of the amino acid sequences described herein) or nucleic acid sequence (for example, any one of the nucleic acid sequences described herein). Preferably, such a sequence is at least 60%, more preferably 80% or 85%, and more preferably 90%, 95% or even 99% identical at the amino acid level or nucleic acid to the sequence used for comparison.

Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between e⁻³ and e⁻¹⁰⁰ indicating a closely related sequence.

A "T-cell epitope" is to be understood as meaning a peptide sequence that can be bound by MHC molecules of class I or II in the form of a peptide-presenting MHC molecule or MHC complex and then, in this form, be recognized and bound by naive T-cells, cytotoxic T-lymphocytes or T-helper cells.

The terms "treat," "treated," "treating," "treatment," and the like are meant to refer to reducing or ameliorating a disorder and/or symptoms associated therewith (e.g., a neoplasia or tumor). "Treating" includes the concepts of "alleviating", which refers to lessening the frequency of occurrence or recurrence, or the severity, of any symptoms or other ill effects related to a cancer and/or the side effects associated with cancer therapy. The term "treating" also encompasses the concept of "managing" which refers to reducing the severity of a particular disease or disorder in a patient or delaying its recurrence, e.g., lengthening the period of remission in a patient who had suffered from the disease. It is appreciated that, although not precluded, treating a disorder or condition does not require that the disorder, condition, or symptoms associated therewith be completely eliminated.

The term "therapeutic effect" refers to some extent of relief of one or more of the symptoms of a disorder (e.g., a neoplasia or tumor) or its associated pathology. "Therapeutically effective amount" as used herein refers to an amount of an agent which is effective, upon single or multiple dose administration to the cell or subject, in prolonging the survivability of the patient with such a disorder, reducing one or more signs or symptoms of the disorder, preventing or delaying, and the like beyond that expected in the absence of such treatment. "Therapeutically effective amount" is intended to qualify the amount required to achieve a therapeutic effect. A physician or veterinarian having ordinary skill in the art can readily determine and prescribe the "therapeutically effective amount" (e.g., ED50) of the pharmaceutical composition required. For example, the physician or veterinarian could start doses of the compounds of the disclosure employed in a pharmaceutical composition at levels lower than that required in order to achieve the desired therapeutic effect and gradually increase the dosage until the desired effect is achieved.

The pharmaceutical compositions typically should provide a dosage of from about 0.0001 mg to about 200 mg of compound per kilogram of body weight per day. For example, dosages for systemic administration to a human patient can range from 0.01-10 µg/kg, 20-80 µg/kg, 5-50 µg/kg, 75-150 µg/kg, 100-500 µg/kg, 250-750 µg/kg, 500-1000 µg/kg, 1-10 mg/kg, 5-50 mg/kg, 25-75 mg/kg, 50-100 mg/kg, 100-250 mg/kg, 50-100 mg/kg, 250-500 mg/kg, 500-750 mg/kg, 750-1000 mg/kg, 1000-1500 mg/kg, 1500-2000 mg/kg, 5 mg/kg, 20 mg/kg, 50 mg/kg, 100 mg/kg, of 200 mg/kg. Pharmaceutical dosage unit forms are prepared to provide from about 0.001 mg to about 5000 mg, for example from about 100 to about 2500 mg of the compound or a combination of essential ingredients per dosage unit form.

A "vaccine" is to be understood as meaning a composition for generating immunity for the prophylaxis and/or treatment of diseases (e.g., neoplasia/tumor). Accordingly, vaccines are medicaments which comprise antigens and are intended to be used in humans or animals for generating specific defense and protective substance by vaccination.

The recitation of a listing of chemical groups in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

The present disclosure relates to vaccines and methods for the treatment of neoplasia, and more particularly tumors, by administering a therapeutically effective amount of a pharmaceutical composition (e.g., a cancer vaccine) comprising a plurality of neoplasia/tumor specific neo-antigens to a subject (e.g., a mammal such as a human). As described in more detail herein, whole genome/exome sequencing may be used to identify all, or nearly all, mutated neoantigens that are uniquely present in a neoplasia/tumor of an individual patient, and that this collection of mutated neoantigens may be analyzed to identify a specific, optimized subset of neoantigens for use as a personalized cancer vaccine or immunogenic composition for treatment of the patient's neoplasia/tumor. For example, a population of neoplasia/tumor specific neoantigens may be identified by sequencing the neoplasia/tumor and normal DNA of each patient to identify tumor-specific mutations, and the patient's HLA allotype can be identified. The population of neoplasia/tumor specific neoantigens and their cognate native antigens may then be subject to bioinformatic analysis using validated algorithms to predict which tumor-specific mutations create epitopes that could bind to the patient's HLA allotype. Based on this analysis, a plurality of peptides corresponding to a subset of these mutations may be designed and synthesized for each patient, and pooled together for use as a cancer vaccine or immunogenic composition in immunizing the patient. The neo-antigens peptides may be combined with an adjuvant (e.g., poly-ICLC) or another anti-neoplastic agent. Without being bound by theory, these neo-antigens are expected to bypass central thymic tolerance (thus allowing stronger anti-tumor T cell response), while reducing the potential for autoimmunity (e.g., by avoiding targeting of normal self-antigens).

The immune system can be classified into two functional subsystems: the innate and the acquired immune system. The innate immune system is the first line of defense against infections, and most potential pathogens are rapidly neutralized by this system before they can cause, for example, a noticeable infection. The acquired immune system reacts to molecular structures, referred to as antigens, of the intruding organism. There are two types of acquired immune reactions, which include the humoral immune reaction and the cell-mediated immune reaction. In the humoral immune reaction, antibodies secreted by B cells into bodily fluids bind to pathogen-derived antigens, leading to the elimination of the pathogen through a variety of mechanisms, e.g. complement-mediated lysis. In the cell-mediated immune reaction, T-cells capable of destroying other cells are activated. For example, if proteins associated with a disease are present in a cell, they are fragmented proteolytically to peptides within the cell. Specific cell proteins then attach themselves to the antigen or peptide formed in this manner and transport them to the surface of the cell, where they are presented to the molecular defense mechanisms, in particular T-cells, of the body. Cytotoxic T cells recognize these antigens and kill the cells that harbor the antigens.

The molecules that transport and present peptides on the cell surface are referred to as proteins of the major histocompatibility complex (MHC). MHC proteins are classified into two types, referred to as MHC class I and MHC class II. The structures of the proteins of the two MHC classes are very similar; however, they have very different functions. Proteins of MHC class I are present on the surface of almost all cells of the body, including most tumor cells. MHC class I proteins are loaded with antigens that usually originate from endogenous proteins or from pathogens present inside cells, and are then presented to naïve or cytotoxic T-lymphocytes (CTLs). MHC class II proteins are present on dendritic cells, B- lymphocytes, macrophages and other antigen-presenting cells. They mainly present peptides, which are processed from external antigen sources, i.e. outside of the cells, to T-helper (Th) cells. Most of the peptides bound by the MHC class I proteins originate from cytoplasmic proteins produced in the healthy host cells of an organism itself, and do not normally stimulate an immune reaction. Accordingly, cytotoxic T-lymphocytes that recognize such self-peptide-presenting MHC molecules of class I are deleted in the thymus (central tolerance) or, after their release from the thymus, are deleted or inactivated, i.e. tolerized (peripheral tolerance). MHC molecules are capable of stimulating an immune reaction when they present peptides to non-tolerized T-lymphocytes. Cytotoxic T-lymphocytes have both T-cell receptors (TCR) and CD8 molecules on their surface. T-Cell receptors are capable of recognizing and binding peptides complexed with the molecules of MHC class I. Each cytotoxic T-lymphocyte expresses a unique T-cell receptor which is capable of binding specific MHC/peptide complexes.

The peptide antigens attach themselves to the molecules of MHC class I by competitive affinity binding within the endoplasmic reticulum, before they are presented on the cell surface. Here, the affinity of an individual peptide antigen is directly linked to its amino acid sequence and the presence of specific binding motifs in defined positions within the amino acid sequence. If the sequence of such a peptide is known, it is possible to manipulate the immune system against diseased cells using, for example, peptide vaccines.

One of the critical barriers to developing curative and tumor-specific immunotherapy is the identification and selection of highly specific and restricted tumor antigens to avoid autoimmunity. Tumor neoantigens, which arise as a result of genetic change (e.g., inversions, translocations, deletions, missense mutations, splice site mutations, etc.) within malignant cells, represent the most tumor-specific class of antigens. Neoantigens have rarely been used in cancer vaccine or immunogenic compositions due to technical difficulties in identifying them, selecting optimized neoantigens, and producing neoantigens for use in a vaccine or immunogenic composition. These problems may be addressed by:
- identifying all, or nearly all, mutations in the neoplasia/tumor at the DNA level using whole genome, whole exome (e.g., only captured exons), or RNA sequencing of tumor versus matched germline samples from each patient;
- analyzing the identified mutations with one or more peptide-MHC binding prediction algorithms to generate a plurality of candidate neoantigen T cell epitopes that are expressed within the neoplasia/tumor and may bind patient HLA alleles; and
- synthesizing the plurality of candidate neoantigen peptides selected from the sets of all neoORF peptides and predicted binding peptides for use in a cancer vaccine or immunogenic composition.

For example, translating sequencing information into a therapeutic vaccine may include:
*(1) Prediction of personal mutated peptides that can bind to HLA molecules of the individual.* Efficiently choosing which particular mutations to utilize as immunogen requires identification of the patient HLA type and the ability to predict which mutated peptides would efficiently bind to the patient's HLA alleles. Recently, neural network based learning approaches with validated binding and non-binding peptides have advanced the accuracy of prediction algorithms for the major HLA-A and -B alleles.
*(2) Formulating the drug as a multi-epitope vaccine of long peptides.* Targeting as many mutated epitopes as practically possible takes advantage of the enormous capacity of the immune system, prevents the opportunity for immunological escape by down-modulation of a particular immune targeted gene product, and compensates for the known inaccuracy of epitope prediction approaches. Synthetic peptides provide a particularly useful means to prepare multiple immunogens efficiently and to rapidly translate identification of mutant epitopes to an effective vaccine. Peptides can be readily synthesized chemically and easily purified utilizing reagents free of contaminating bacteria or animal substances. The small size allows a clear focus on the mutated region of the protein and also reduces irrelevant antigenic competition from other components (unmutated protein or viral vector antigens).
*(3) Combination with a strong vaccine adjuvant.* Effective vaccines require a strong adjuvant to initiate an immune response. As described below, poly-ICLC, an agonist of TLR3 and the RNA helicase -domains of MDA5 and RIG3, has shown several desirable properties for a vaccine adjuvant. These properties include the induction of local and systemic activation of immune cells *in vivo,* production of stimulatory chemokines and cytokines, and stimulation of antigen-presentation by DCs. Furthermore, poly-ICLC can induce durable CD4+ and CD8+ responses in humans. Importantly, striking similarities in the upregulation of transcriptional and signal transduction pathways were seen in subjects vaccinated with poly-ICLC and in volunteers who had received the highly effective, replication-competent yellow fever vaccine. Furthermore, >90% of ovarian carcinoma patients immunized with poly-ICLC in combination with a NY-ES0-1 peptide vaccine (in addition to Montanide) showed induction of CD4+ and CD8+ T cell, as well as antibody responses to the peptide in a recent phase 1 study. At the same time, polyICLC has been extensively tested in more than 25 clinical trials to date and exhibited a relatively benign toxicity profile. The advantages of the invention are described further herein.

As described herein, there is a large body of evidence in both animals and humans that mutated epitopes are effective in inducing an immune response and that cases of spontaneous tumor regression or long term survival correlate with CD8+ T-cell responses to mutated epitopes (Buckwalter and Srivastava PK. "It is the antigen(s), stupid" and other lessons from over a decade of vaccitherapy of human cancer. Seminars in immunology 20:296-300 (2008); Karanikas et al, High frequency of cytolytic T lymphocytes directed against a tumor-specific mutated antigen detectable with HLA tetramers in the blood of a lung carcinoma patient with long survival. Cancer Res. 61:3718-3724 (2001); Lennerz et al, The response of autologous T cells to a human melanoma is dominated by mutated neoantigens. Proc Natl Acad Sci U S A.102:16013 (2005)) and that "immunoediting" can be tracked to alterations in expression of dominant mutated antigens in mice and man (Matsushita et al, Cancer exome analysis reveals a T-cell-dependent mechanism of cancer immunoediting Nature 482:400 (2012); DuPage et al, Expression of tumor-specific antigens underlies cancer immunoediting Nature 482:405 (2012); and Sampson et al, Immunologic escape after prolonged progression-free survival with epidermal growth factor receptor variant III peptide vaccination in patients with newly diagnosed glioblastoma J Clin Oncol. 28:4722-4729 (2010)). In one embodiment, the mutated epitopes of a cancer patient are determined.

In one embodiment mutated epitopes are determined by sequencing the genome and/or exome of tumor tissue and healthy tissue from a cancer patient using next generation sequencing technologies. In another embodiment genes that are selected based on their frequency of mutation and ability to act as a neoantigen are sequenced using next generation sequencing technology. Next-generation sequencing applies to genome sequencing, genome resequencing, transcriptome profiling (RNA-Seq), DNA-protein interactions (ChIP-sequencing), and epigenome characterization (de Magalhães JP, Finch CE, Janssens G (2010). "Next-generation sequencing in aging research: emerging applications, problems, pitfalls and possible solutions". Ageing Research Reviews 9 (3): 315-323; Hall N (May 2007). "Advanced sequencing technologies and their wider impact in microbiology". J. Exp. Biol. 209 (Pt 9): 1518-1525; Church GM (January 2006). "Genomes for all". Sci. Am. 294 (1): 46-54; ten Bosch JR, Grody WW (2008). "Keeping Up with the Next Generation". The Journal of Molecular Diagnostics 10 (6): 484-492; Tucker T, Marra M, Friedman JM (2009). "Massively Parallel Sequencing: The Next Big Thing in Genetic Medicine". The American Journal of Human Genetics 85 (2): 142-154). Next-generation sequencing can now rapidly reveal the presence of discrete mutations such as coding mutations in individual tumors, most commonly single amino acid changes (e.g., missense mutations) and less frequently novel stretches of amino acids generated by frame-shift insertions/deletions/gene fusions, read-through mutations in stop codons, and translation of improperly spliced introns (e.g., neoORFs). NeoORFs are particularly valuable as immunogens because the entirety of their sequence is completely novel to the immune system and so are analogous to a viral or bacterial foreign antigen. Thus, neoORFs: (1) are highly specific to the tumor (i.e. there is no expression in any normal cells); and (2) can bypass central tolerance, thereby increasing the precursor frequency of neoantigen-specific CTLs. For example, the power of utilizing analogous foreign sequences in a therapeutic anticancer vaccine or immunogenic composition was recently demonstrated with peptides derived from human papilloma virus (HPV). ~50% of the 19 patients with pre-neoplastic, viral-induced disease who received 3 - 4 vaccinations of a mix of HPV peptides derived from the viral oncogenes E6 and E7 maintained a complete response for ≥24 months (Kenter et a, Vaccination against HPV-16 Oncoproteins for Vulvar Intraepithelial Neoplasia NEJM 361:1838 (2009)).

Sequencing technology has revealed that each tumor contains multiple, patient-specific mutations that alter the protein coding content of a gene. Such mutations create altered proteins, ranging from single amino acid changes (caused by missense mutations) to addition of long regions of novel amino acid sequence due to frame shifts, read-through of termination codons or translation of intron regions (novel open reading frame mutations; neoORFs). These mutated proteins are valuable targets for the host's immune response to the tumor as, unlike native proteins, they are not subject to the immune-dampening effects of self-tolerance. Therefore, mutated proteins are more likely to be immunogenic and are also more specific for the tumor cells compared to normal cells of the patient.

An alternative method for identifying tumor specific neoantigens is direct protein sequencing. Protein sequencing of enzymatic digests using multidimensional MS techniques (MSn) including tandem mass spectrometry (MS/MS)) can also be used to identify neoantigens of the disclosure. Such proteomic approaches permit rapid, highly automated analysis (see, e.g., K. Gevaert and J. Vandekerckhove, Electrophoresis 21:1145-1154 (2000)). It is further contemplated within the scope of the invention that high-throughput methods for de novo sequencing of unknown proteins may be used to analyze the proteome of a patient's tumor to identify expressed neoantigens. For example, meta shotgun protein sequencing may be used to identify expressed neoantigens (see e.g., Guthals et al. (2012) Shotgun Protein Sequencing with Meta-contig Assembly, Molecular and Cellular Proteomics 11(10):1084-96).

Tumor specific neoantigens may also be identified using MHC multimers to identify neoantigen-specific T-cell responses. For example, high-throughput analysis of neoantigen-specific T-cell responses in patient samples may be performed using MHC tetramer-based screening techniques (see e.g., Hombrink et al. (2011) High-Throughput Identification of Potential Minor Histocompatibility Antigens by MHC Tetramer-Based Screening: Feasibility and Limitations 6(8):1-11; Hadrup et al. (2009) Parallel detection of antigen-specific T-cell responses by multidimensional encoding of MHC multimers, Nature Methods, 6(7):520-26; van Rooij et al. (2013) Tumor exome analysis reveals neoantigen-specific T-cell reactivity in an Ipilimumab-responsive melanoma, Journal of Clinical Oncology, 31:1-4; and Heemskerk et al. (2013) The cancer antigenome, EMBO Journal, 32(2):194-203). Such tetramer-based screening techniques may be used for the initial identification of tumor specific neoantigens, or alternatively as a secondary screening protocol to assess what neoantigens a patient may have already been exposed to, thereby facilitating the selection of candidate neoantigens for the invention.

In one embodiment the sequencing data derived from determining the presence of mutations in a cancer patient is analysed to predict personal mutated peptides that can bind to HLA molecules of the individual. In one embodiment the data is analysed using a computer. In another embodiment the sequence data is analysed for the presence of neoantigens. In one embodiment neoantigens are determined by their affinity to MHC molecules. Efficiently choosing which particular mutations to utilize as immunogen requires identification of the patient HLA type and the ability to predict which mutated peptides would efficiently bind to the patient's HLA alleles. Recently, neural network based learning approaches with validated binding and non-binding peptides have advanced the accuracy of prediction algorithms for the major HLA-A and -B alleles. Utilizing the recently improved algorithms for predicting which missense mutations create strong binding peptides to the patient's cognate MHC molecules, a set of peptides representative of optimal mutated epitopes (both neoORF and missense) for each patient may be identified and prioritized (Zhang et al, Machine learning competition in immunology - Prediction of HLA class I binding peptides J Immunol Methods 374:1 (2011); Lundegaard et al Prediction of epitopes using neural network based methods J Immunol Methods 374:26 (2011)).

Targeting as many mutated epitopes as practically possible takes advantage of the enormous capacity of the immune system, prevents the opportunity for immunological escape by down-modulation of a particular immune targeted gene product, and compensates for the known inaccuracy of epitope prediction approaches. Synthetic peptides provide a particularly useful means to prepare multiple immunogens efficiently and to rapidly translate identification of mutant epitopes to an effective vaccine or immunogenic composition. Peptides can be readily synthesized chemically and easily purified utilizing reagents free of contaminating bacteria or animal substances. The small size allows a clear focus on the mutated region of the protein and also reduces irrelevant antigenic competition from other components (unmutated protein or viral vector antigens).

In one embodiment the drug formulation is a multi-epitope vaccine or immunogenic composition of long peptides. Such "long" peptides undergo efficient internalization, processing and cross-presentation in professional antigen-presenting cells such as dendritic cells, and have been shown to induce CTLs in humans (Melief and van der Burg, Immunotherapy of established (pre) malignant disease by synthetic long peptide vaccines Nature Rev Cancer 8:351 (2008)). In one embodiment at least 1 peptide is prepared for immunization. In a prefered embodiment 20 or more peptides are prepared for immunization. According to the invention the neoantigenic peptide ranges from 5 to 50 amino acids in length. In another embodiment peptides from about 15 to about 35 amino acids in length is synthesized. In prefered embodiment the neoantigenic peptide ranges from about 20 to about 35 amino acids in length.

### Production of Tumor Specific Neoantigens

The present invention is based, at least in part, on the ability to present the immune system of the patient with a pool of tumor specific neoantigens. One of skill in the art from this disclosure and the knowledge in the art will appreciate that there are a variety of ways in which to produce such tumor specific neoantigens. In general, such tumor specific neoantigens may be produced either in vitro or in vivo. Tumor specific neoantigens may be produced in vitro as peptides or polypeptides, which may then be formulated into a personalized neoplasia vaccine or immunogenic composition and administered to a subject. As described in further detail herein, such in vitro production may occur by a variety of methods known to one of skill in the art such as, for example, peptide synthesis or expression of a peptide/polypeptide from a DNA or RNA molecule in any of a variety of bacterial, eukaryotic, or viral recombinant expression systems, followed by purification of the expressed peptide/polypeptide. Alternatively, tumor specific neoantigens may be produced in vivo by introducing molecules (e.g., DNA, RNA, viral expression systems, and the like) that encode tumor specific neoantigens into a subject, whereupon the encoded tumor specific neoantigens are expressed. The methods of in vitro and in vivo production of neoantigens is also further described herein as it relates to pharmaceutical compositions and methods of delivery.

### In Vitro Peptide/Polypeptide Synthesis

Proteins or peptides may be made by any technique known to those of skill in the art, including the expression of proteins, polypeptides or peptides through standard molecular biological techniques, the isolation of proteins or peptides from natural sources, in vitro translation, or the chemical synthesis of proteins or peptides. The nucleotide and protein, polypeptide and peptide sequences corresponding to various genes have been previously disclosed, and may be found at computerized databases known to those of ordinary skill in the art. One such database is the National Center for Biotechnology Information's Genbank and GenPept databases located at the National Institutes of Health website. The coding regions for known genes may be amplified and/or expressed using the techniques disclosed herein or as would be known to those of ordinary skill in the art. Alternatively, various commercial preparations of proteins, polypeptides and peptides are known to those of skill in the art.

Peptides can be readily synthesized chemically utilizing reagents that are free of contaminating bacterial or animal substances (Merrifield RB: Solid phase peptide synthesis. I. The synthesis of a tetrapeptide. J. Am. Chem. Soc. 85:2149-54, 1963). In certain embodiments, neoantigenic peptides are prepared by (1) parallel solid-phase synthesis on multi-channel instruments using uniform synthesis and cleavage conditions; (2) purification over a RP-HPLC column with column stripping; and re-washing, but not replacement, between peptides; followed by (3) analysis with a limited set of the most informative assays. The Good Manufacturing Practices (GMP) footprint can be defined around the set of peptides for an individual patient, thus requiring suite changeover procedures only between syntheses of peptides for different patients.

Alternatively, a nucleic acid (e.g., a polynucleotide) encoding a neoantigenic peptide of the disclosure may be used to produce the neoantigenic peptide in vitro. The polynucleotide may be, e.g., DNA, cDNA, PNA, CNA, RNA, either single- and/or double-stranded, or native or stabilized forms of polynucleotides, such as e.g. polynucleotides with a phosphorothiate backbone, or combinations thereof and it may or may not contain introns so long as it codes for the peptide. In one embodiment in vitro translation is used to produce the peptide. Many exemplary systems exist that one skilled in the art could utilize (e.g., Retic Lysate IVT Kit, Life Technologies, Waltham, MA).

An expression vector capable of expressing a polypeptide can also be prepared. Expression vectors for different cell types are well known in the art and can be selected without undue experimentation. Generally, the DNA is inserted into an expression vector, such as a plasmid, in proper orientation and correct reading frame for expression. If necessary, the DNA may be linked to the appropriate transcriptional and translational regulatory control nucleotide sequences recognized by the desired host (e.g., bacteria), although such controls are generally available in the expression vector. The vector is then introduced into the host bacteria for cloning using standard techniques (see, e.g., Sambrook et al. (1989) Molecular Cloning, A Laboratory Manual, Cold Spring Harbor Laboratory, Cold Spring Harbor, N.Y.).

Expression vectors comprising the isolated polynucleotides, as well as host cells containing the expression vectors, are also contemplated. The neoantigenic peptides may be provided in the form of RNA or cDNA molecules encoding the desired neoantigenic peptides. One or more neoantigenic peptides of the disclosure may be encoded by a single expression vector.

The term "polynucleotide encoding a polypeptide" encompasses a polynucleotide which includes only coding sequences for the polypeptide as well as a polynucleotide which includes additional coding and/or non-coding sequences. Polynucleotides can be in the form of RNA or in the form of DNA. DNA includes cDNA, genomic DNA, and synthetic DNA; and can be double-stranded or single-stranded, and if single stranded can be the coding strand or non-coding (anti-sense) strand.

In embodiments, the polynucleotides may comprise the coding sequence for the tumor specific neoantigenic peptide fused in the same reading frame to a polynucleotide which aids, for example, in expression and/or secretion of a polypeptide from a host cell (e.g., a leader sequence which functions as a secretory sequence for controlling transport of a polypeptide from the cell). The polypeptide having a leader sequence is a preprotein and can have the leader sequence cleaved by the host cell to form the mature form of the polypeptide.

In embodiments, the polynucleotides can comprise the coding sequence for the tumor specific neoantigenic peptide fused in the same reading frame to a marker sequence that allows, for example, for purification of the encoded polypeptide, which may then be incorporated into the personalized neoplasia vaccine or immunogenic composition. For example, the marker sequence can be a hexa-histidine tag supplied by a pQE-9 vector to provide for purification of the mature polypeptide fused to the marker in the case of a bacterial host, or the marker sequence can be a hemagglutinin (HA) tag derived from the influenza hemagglutinin protein when a mammalian host (e.g., COS-7 cells) is used. Additional tags include, but are not limited to, Calmodulin tags, FLAG tags, Myc tags, S tags, SBP tags, Softag 1, Softag 3, V5 tag, Xpress tag, Isopeptag, SpyTag, Biotin Carboxyl Carrier Protein (BCCP) tags, GST tags, fluorescent protein tags (e.g., green fluorescent protein tags), maltose binding protein tags, Nus tags, Strep-tag, thioredoxin tag, TC tag, Ty tag, and the like.

In embodiments, the polynucleotides may comprise the coding sequence for one or more of the tumor specific neoantigenic peptides fused in the same reading frame to create a single concatamerized neoantigenic peptide construct capable of producing multiple neoantigenic peptides.

In certain instances , isolated nucleic acid molecules having a nucleotide sequence at least 60% identical, at least 65% identical, at least 70% identical, at least 75% identical, at least 80% identical, at least 85% identical, at least 90% identical, at least 95% identical, or at least 96%, 97%, 98% or 99% identical to a polynucleotide encoding a tumor specific neoantigenic peptide of the present invention, can be provided.

By a polynucleotide having a nucleotide sequence at least, for example, 95% "identical" to a reference nucleotide sequence is intended that the nucleotide sequence of the polynucleotide is identical to the reference sequence except that the polynucleotide sequence can include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence can be deleted or substituted with another nucleotide, or a number of nucleotides up to 5% of the total nucleotides in the reference sequence can be inserted into the reference sequence. These mutations of the reference sequence can occur at the amino- or carboxy-terminal positions of the reference nucleotide sequence or anywhere between those terminal positions, interspersed either individually among nucleotides in the reference sequence or in one or more contiguous groups within the reference sequence.

As a practical matter, whether any particular nucleic acid molecule is at least 80% identical, at least 85% identical, at least 90% identical, and in some instances , at least 95%, 96%, 97%, 98%, or 99% identical to a reference sequence can be determined conventionally using known computer programs such as the Bestfit program (Wisconsin Sequence Analysis Package, Version 8 for Unix, Genetics Computer Group, University Research Park, 575 Science Drive, Madison, WI 53711). Bestfit uses the local homology algorithm of Smith and Waterman, Advances in Applied Mathematics 2:482-489 (1981), to find the best segment of homology between two sequences. When using Bestfit or any other sequence alignment program to determine whether a particular sequence is, for instance, 95% identical to a reference sequence according to the present disclosure , the parameters are set such that the percentage of identity is calculated over the full length of the reference nucleotide sequence and that gaps in homology of up to 5% of the total number of nucleotides in the reference sequence are allowed.

The isolated tumor specific neoantigenic peptides described herein can be produced in vitro (e.g., in the laboratory) by any suitable method known in the art. Such methods range from direct protein synthetic methods to constructing a DNA sequence encoding isolated polypeptide sequences and expressing those sequences in a suitable transformed host. In some embodiments, a DNA sequence is constructed using recombinant technology by isolating or synthesizing a DNA sequence encoding a wild-type protein of interest. Optionally, the sequence can be mutagenized by site-specific mutagenesis to provide functional analogs thereof. See, e.g. Zoeller et al., Proc. Nat'l. Acad. Sci. USA 81:5662-5066 (1984) and U.S. Pat. No. 4,588,585.

In embodiments, a DNA sequence encoding a polypeptide of interest would be constructed by chemical synthesis using an oligonucleotide synthesizer. Such oligonucleotides can be designed based on the amino acid sequence of the desired polypeptide and selecting those codons that are favored in the host cell in which the recombinant polypeptide of interest is produced. Standard methods can be applied to synthesize an isolated polynucleotide sequence encoding an isolated polypeptide of interest. For example, a complete amino acid sequence can be used to construct a back-translated gene. Further, a DNA oligomer containing a nucleotide sequence coding for the particular isolated polypeptide can be synthesized. For example, several small oligonucleotides coding for portions of the desired polypeptide can be synthesized and then ligated. The individual oligonucleotides typically contain 5' or 3' overhangs for complementary assembly.

Once assembled (e.g., by synthesis, site-directed mutagenesis, or another method), the polynucleotide sequences encoding a particular isolated polypeptide of interest is inserted into an expression vector and optionally operatively linked to an expression control sequence appropriate for expression of the protein in a desired host. Proper assembly can be confirmed by nucleotide sequencing, restriction mapping, and expression of a biologically active polypeptide in a suitable host. As well known in the art, in order to obtain high expression levels of a transfected gene in a host, the gene can be operatively linked to transcriptional and translational expression control sequences that are functional in the chosen expression host.

Recombinant expression vectors may be used to amplify and express DNA encoding the tumor specific neoantigenic peptides. Recombinant expression vectors are replicable DNA constructs which have synthetic or cDNA-derived DNA fragments encoding a tumor specific neoantigenic peptide or a bioequivalent analog operatively linked to suitable transcriptional or translational regulatory elements derived from mammalian, microbial, viral or insect genes. A transcriptional unit generally comprises an assembly of (1) a genetic element or elements having a regulatory role in gene expression, for example, transcriptional promoters or enhancers, (2) a structural or coding sequence which is transcribed into mRNA and translated into protein, and (3) appropriate transcription and translation initiation and termination sequences, as described in detail herein. Such regulatory elements can include an operator sequence to control transcription. The ability to replicate in a host, usually conferred by an origin of replication, and a selection gene to facilitate recognition of transformants can additionally be incorporated. DNA regions are operatively linked when they are functionally related to each other. For example, DNA for a signal peptide (secretory leader) is operatively linked to DNA for a polypeptide if it is expressed as a precursor which participates in the secretion of the polypeptide; a promoter is operatively linked to a coding sequence if it controls the transcription of the sequence; or a ribosome binding site is operatively linked to a coding sequence if it is positioned so as to permit translation. Generally, operatively linked means contiguous, and in the case of secretory leaders, means contiguous and in reading frame. Structural elements intended for use in yeast expression systems include a leader sequence enabling extracellular secretion of translated protein by a host cell. Alternatively, where recombinant protein is expressed without a leader or transport sequence, it can include an N-terminal methionine residue. This residue can optionally be subsequently cleaved from the expressed recombinant protein to provide a final product.

Useful expression vectors for eukaryotic hosts, especially mammals or humans include, for example, vectors comprising expression control sequences from SV40, bovine papilloma virus, adenovirus and cytomegalovirus. Useful expression vectors for bacterial hosts include known bacterial plasmids, such as plasmids from Escherichia coli, including pCR 1, pBR322, pMB9 and their derivatives, wider host range plasmids, such as M13 and filamentous single-stranded DNA phages.

Suitable host cells for expression of a polypeptide include prokaryotes, yeast, insect or higher eukaryotic cells under the control of appropriate promoters. Prokaryotes include gram negative or gram positive organisms, for example E. coli or bacilli. Higher eukaryotic cells include established cell lines of mammalian origin. Cell-free translation systems could also be employed. Appropriate cloning and expression vectors for use with bacterial, fungal, yeast, and mammalian cellular hosts are well known in the art (see Pouwels et al., Cloning Vectors: A Laboratory Manual, Elsevier, N.Y., 1985).

Various mammalian or insect cell culture systems are also advantageously employed to express recombinant protein. Expression of recombinant proteins in mammalian cells can be performed because such proteins are generally correctly folded, appropriately modified and completely functional. Examples of suitable mammalian host cell lines include the COS-7 lines of monkey kidney cells, described by Gluzman (Cell 23:175, 1981), and other cell lines capable of expressing an appropriate vector including, for example, L cells, C127, 3T3, Chinese hamster ovary (CHO), 293, HeLa and BHK cell lines. Mammalian expression vectors can comprise nontranscribed elements such as an origin of replication, a suitable promoter and enhancer linked to the gene to be expressed, and other 5' or 3' flanking nontranscribed sequences, and 5' or 3' nontranslated sequences, such as necessary ribosome binding sites, a polyadenylation site, splice donor and acceptor sites, and transcriptional termination sequences. Baculovirus systems for production of heterologous proteins in insect cells are reviewed by Luckow and Summers, Bio/Technology 6:47 (1988).

The proteins produced by a transformed host can be purified according to any suitable method. Such standard methods include chromatography (e.g., ion exchange, affinity and sizing column chromatography, and the like), centrifugation, differential solubility, or by any other standard technique for protein purification. Affinity tags such as hexahistidine, maltose binding domain, influenza coat sequence, glutathione-S-transferase, and the like can be attached to the protein to allow easy purification by passage over an appropriate affinity column. Isolated proteins can also be physically characterized using such techniques as proteolysis, nuclear magnetic resonance and x-ray crystallography.

For example, supernatants from systems which secrete recombinant protein into culture media can be first concentrated using a commercially available protein concentration filter, for example, an Amicon or Millipore Pellicon ultrafiltration unit. Following the concentration step, the concentrate can be applied to a suitable purification matrix. Alternatively, an anion exchange resin can be employed, for example, a matrix or substrate having pendant diethylaminoethyl (DEAE) groups. The matrices can be acrylamide, agarose, dextran, cellulose or other types commonly employed in protein purification. Alternatively, a cation exchange step can be employed. Suitable cation exchangers include various insoluble matrices comprising sulfopropyl or carboxymethyl groups. Finally, one or more reversed-phase high performance liquid chromatography (RP-HPLC) steps employing hydrophobic RP-HPLC media, e.g., silica gel having pendant methyl or other aliphatic groups, can be employed to further purify a cancer stem cell protein-Fc composition. Some or all of the foregoing purification steps, in various combinations, can also be employed to provide a homogeneous recombinant protein.

Recombinant protein produced in bacterial culture can be isolated, for example, by initial extraction from cell pellets, followed by one or more concentration, salting-out, aqueous ion exchange or size exclusion chromatography steps. High performance liquid chromatography (HPLC) can be employed for final purification steps. Microbial cells employed in expression of a recombinant protein can be disrupted by any convenient method, including freeze-thaw cycling, sonication, mechanical disruption, or use of cell lysing agents.

### In Vivo Peptide/Polypeptide Synthesis

The present disclosure also contemplates the use of nucleic acid molecules as vehicles for delivering neoantigenic peptides/polypeptides to the subject in need thereof, in vivo, in the form of, e.g., DNA/RNA vaccines (see, e.g., WO2012/159643, and WO2012/159754 ).

In one instance neoantigens may be administered to a patient in need thereof by use of a plasmid. These are plasmids which usually consist of a strong viral promoter to drive the in vivo transcription and translation of the gene (or complementary DNA) of interest (Mor, et al., (1995). The Journal of Immunology 155 (4): 2039-2046). Intron A may sometimes be included to improve mRNA stability and hence increase protein expression (Leitner et al. (1997).The Journal of Immunology 159 (12): 6112-6119). Plasmids also include a strong polyadenylation/transcriptional termination signal, such as bovine growth hormone or rabbit beta-globulin polyadenylation sequences (Alarcon et al., (1999). Adv. Parasitol. Advances in Parasitology 42: 343-410; Robinson et al., (2000). Adv. Virus Res. Advances in Virus Research 55: 1-74; Böhmet al., (1996). Journal of Immunological Methods 193 (1): 29-40.). Multicistronic vectors are sometimes constructed to express more than one immunogen, or to express an immunogen and an immunostimulatory protein (Lewis et al., (1999). Advances in Virus Research (Academic Press) 54: 129-88).

Because the plasmid is the "vehicle" from which the immunogen is expressed, optimising vector design for maximal protein expression is essential (Lewis et al., (1999). Advances in Virus Research (Academic Press) 54: 129-88). One way of enhancing protein expression is by optimising the codon usage of pathogenic mRNAs for eukaryotic cells. Another consideration is the choice of promoter. Such promoters may be the SV40 promoter or Rous Sarcoma Virus (RSV).

Plasmids may be introduced into animal tissues by a number of different methods. The two most popular approaches are injection of DNA in saline, using a standard hypodermic needle, and gene gun delivery. A schematic outline of the construction of a DNA vaccine plasmid and its subsequent delivery by these two methods into a host is illustrated at Scientific American (Weiner et al., (1999) Scientific American 281 (1): 34-41). Injection in saline is normally conducted intramuscularly (IM) in skeletal muscle, or intradermally (ID), with DNA being delivered to the extracellular spaces. This can be assisted by electroporation by temporarily damaging muscle fibres with myotoxins such as bupivacaine; or by using hypertonic solutions of saline or sucrose (Alarcon et al., (1999). Adv. Parasitol. Advances in Parasitology 42: 343-410). Immune responses to this method of delivery can be affected by many factors, including needle type, needle alignment, speed of injection, volume of injection, muscle type, and age, sex and physiological condition of the animal being injected(Alarcon et al., (1999). Adv. Parasitol. Advances in Parasitology 42: 343-410).

Gene gun delivery, the other commonly used method of delivery, ballistically accelerates plasmid DNA (pDNA) that has been adsorbed onto gold or tungsten microparticles into the target cells, using compressed helium as an accelerant (Alarcon et al., (1999). Adv. Parasitol. Advances in Parasitology 42: 343-410; Lewis et al., (1999). Advances in Virus Research (Academic Press) 54: 129-88).

Alternative delivery methods may include aerosol instillation of naked DNA on mucosal surfaces, such as the nasal and lung mucosa, (Lewis et al., (1999). Advances in Virus Research (Academic Press) 54: 129-88) and topical administration of pDNA to the eye and vaginal mucosa (Lewis et al., (1999) Advances in Virus Research (Academic Press) 54: 129-88). Mucosal surface delivery has also been achieved using cationic liposome-DNA preparations, biodegradable microspheres, attenuated Shigella or Listeria vectors for oral administration to the intestinal mucosa, and recombinant adenovirus vectors.

The method of delivery determines the dose of DNA required to raise an effective immune response. Saline injections require variable amounts of DNA, from 10 µg-1 mg, whereas gene gun deliveries require 100 to 1000 times less DNA than intramuscular saline injection to raise an effective immune response. Generally, 0.2 µg - 20 µg are required, although quantities as low as 16 ng have been reported. These quantities vary from species to species, with mice, for example, requiring approximately 10 times less DNA than primates. Saline injections require more DNA because the DNA is delivered to the extracellular spaces of the target tissue (normally muscle), where it has to overcome physical barriers (such as the basal lamina and large amounts of connective tissue, to mention a few) before it is taken up by the cells, while gene gun deliveries bombard DNA directly into the cells, resulting in less "wastage" (See e.g., Sedegah et al., (1994). Proceedings of the National Academy of Sciences of the United States of America 91 (21): 9866-9870; Daheshiaet al., (1997). The Journal of Immunology 159 (4): 1945-1952; Chen et al., (1998). The Journal of Immunology 160 (5): 2425-2432; Sizemore (1995) Science 270 (5234): 299-302; Fynan et al., (1993) Proc. Natl. Acad. Sci. U.S.A. 90 (24): 11478-82).

In one instance , a neoplasia vaccine or immunogenic composition may include separate DNA plasmids encoding, for example, one or more neoantigenic peptides/polypeptides as identified in according to the invention. As discussed herein, the exact choice of expression vectors can depend upon the peptide/polypeptides to be expressed, and is well within the skill of the ordinary artisan. The expected persistence of the DNA constructs (e.g., in an episomal, non-replicating, non-integrated form in the muscle cells) is expected to provide an increased duration of protection.

One or more neoantigenic peptides of the disclosure may be encoded and expressed in vivo using a viral based system (e.g., an adenovirus system, an adeno associated virus (AAV) vector, a poxvirus, or a lentivirus). In one instance , the neoplasia vaccine or immunogenic composition may include a viral based vector for use in a human patient in need thereof, such as, for example, an adenovirus (see, e.g., Baden et al. First-in-human evaluation of the safety and immunogenicity of a recombinant adenovirus serotype 26 HIV-1 Env vaccine (IPCAVD 001). J Infect Dis. 2013 Jan 15;207(2):240-7 ). Plasmids that can be used for adeno associated virus, adenovirus, and lentivirus delivery have been described previously (see e.g., U.S. Patent Nos. 6,955,808 and 6,943,019, and U.S. Patent application No. 20080254008).

Among vectors that may be used in the practice of the disclosure , integration in the host genome of a cell is possible with retrovirus gene transfer methods, often resulting in long term expression of the inserted transgene. In a preferred instance the retrovirus is a lentivirus. Additionally, high transduction efficiencies have been observed in many different cell types and target tissues. The tropism of a retrovirus can be altered by incorporating foreign envelope proteins, expanding the potential target population of target cells. A retrovirus can also be engineered to allow for conditional expression of the inserted transgene, such that only certain cell types are infected by the lentivirus. Cell type specific promoters can be used to target expression in specific cell types. Lentiviral vectors are retroviral vectors (and hence both lentiviral and retroviral vectors may be used in the practice of the disclosure ). Moreover, lentiviral vectors are preferred as they are able to transduce or infect non-dividing cells and typically produce high viral titers. Selection of a retroviral gene transfer system may therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the desired nucleic acid into the target cell to provide permanent expression. Widely used retroviral vectors that may be used in the practice of the disclosure include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., (1992) J. Virol. 66:2731-2739; Johann et al., (1992) J. Virol. 66:1635-1640; Sommnerfelt et al., (1990) Virol. 176:58-59; Wilson et al., (1998) J. Virol. 63:2374-2378; Miller et al., (1991) J. Virol. 65:2220-2224; PCT/US94/05700). Zou et al. administered about 10 µl of a recombinant lentivirus having a titer of 1 × 10⁹ transducing units (TU)/ml by an intrathecal catheter. These sort of dosages can be adapted or extrapolated to use of a retroviral or lentiviral vector in the present disclosure .

Also useful in the practice of the disclosure is a minimal non-primate lentiviral vector, such as a lentiviral vector based on the equine infectious anemia virus (EIAV) (see, e.g., Balagaan, (2006) J Gene Med; 8: 275 - 285, Published online 21 November 2005 in Wiley InterScience (www.interscience.wiley.com). DOI: 10.1002/jgm.845). The vectors may have cytomegalovirus (CMV) promoter driving expression of the target gene. Accordingly, the disclosure contemplates amongst vector(s) useful in the practice of the disclosure : viral vectors, including retroviral vectors and lentiviral vectors.

Also useful in the practice of the disclosure is an adenovirus vector. One advantage is the ability of recombinant adenoviruses to efficiently transfer and express recombinant genes in a variety of mammalian cells and tissues in vitro and in vivo, resulting in the high expression of the transferred nucleic acids. Further, the ability to productively infect quiescent cells, expands the utility of recombinant adenoviral vectors. In addition, high expression levels ensure that the products of the nucleic acids will be expressed to sufficient levels to generate an immune response (see e.g., U.S. Patent No. 7,029,848).

In an instance herein the delivery is via an adenovirus, which may be at a single booster dose containing at least 1 × 10⁵ particles (also referred to as particle units, pu) of adenoviral vector. In an embodiment herein, the dose preferably is at least about 1 × 10⁶ particles (for example, about 1 × 10⁶-1 × 10¹² particles), more preferably at least about 1 × 10⁷ particles, more preferably at least about 1 × 10⁸ particles (e.g., about 1 × 10⁸-1 × 10¹¹ particles or about 1 × 10⁸-1 × 10¹² particles), and most preferably at least about 1 × 10⁹ particles (e.g., about 1 × 10⁹-1 × 10¹⁰ particles or about 1 × 10⁹-1 × 10¹² particles), or even at least about 1 × 10¹⁰ particles (e.g., about 1 × 10¹⁰-1 × 10¹² particles) of the adenoviral vector. Alternatively, the dose comprises no more than about 1 × 10¹⁴ particles, preferably no more than about 1 × 10¹³ particles, even more preferably no more than about 1 × 10¹² particles, even more preferably no more than about 1 × 10¹¹ particles, and most preferably no more than about 1 × 10¹⁰ particles (e.g., no more than about 1 × 10⁹ articles). Thus, the dose may contain a single dose of adenoviral vector with, for example, about 1 × 10⁶ particle units (pu), about 2 × 10⁶ pu, about 4 × 10⁶ pu, about 1 × 10⁷ pu, about 2 × 10⁷ pu, about 4 × 10⁷ pu, about 1 × 10⁸ pu, about 2 × 10⁸ pu, about 4 × 10⁸ pu, about 1 × 10⁹ pu, about 2 × 10⁹ pu, about 4 × 10⁹ pu, about 1 × 10¹⁰ pu, about 2 × 10¹⁰ pu, about 4 × 10¹⁰ pu, about 1 × 10¹¹ pu, about 2 × 10¹¹ pu, about 4 × 10¹¹ pu, about 1 × 10¹² pu, about 2 × 10¹² pu, or about 4 × 10¹² pu of adenoviral vector. See, for example, the adenoviral vectors in U.S. Patent No. 8,454,972 B2 to Nabel, et. al., granted on June 4, 2013; and the dosages at col 29, lines 36-58 thereof. In an instance herein, the adenovirus is delivered via multiple doses.

In terms of in vivo delivery, AAV is advantageous over other viral vectors due to low toxicity and low probability of causing insertional mutagenesis because it doesn't integrate into the host genome. AAV has a packaging limit of 4.5 or 4.75 Kb. Constructs larger than 4.5 or 4.75 Kb result in significantly reduced virus production. There are many promoters that can be used to drive nucleic acid molecule expression. AAV ITR can serve as a promoter and is advantageous for eliminating the need for an additional promoter element. For ubiquitous expression, the following promoters can be used: CMV, CAG, CBh, PGK, SV40, Ferritin heavy or light chains, etc. For brain expression, the following promoters can be used: SynapsinI for all neurons, CaMKIIalpha for excitatory neurons, GAD67 or GAD65 or VGAT for GABAergic neurons, etc. Promoters used to drive RNA synthesis can include: Pol III promoters such as U6 or HI. The use of a Pol II promoter and intronic cassettes can be used to express guide RNA (gRNA).

As to AAV, the AAV can be AAV1, AAV2, AAV5 or any combination thereof. One can select the AAV with regard to the cells to be targeted; e.g., one can select AAV serotypes 1, 2, 5 or a hybrid capsid AAV1, AAV2, AAV5 or any combination thereof for targeting brain or neuronal cells; and one can select AAV4 for targeting cardiac tissue. AAV8 is useful for delivery to the liver. The above promoters and vectors are preferred individually.

In an instance herein, the delivery is via an AAV. A therapeutically effective dosage for in vivo delivery of the AAV to a human is believed to be in the range of from about 20 to about 50 ml of saline solution containing from about 1 × 10¹⁰ to about 1 × 10⁵⁰ functional AAV/ml solution. The dosage may be adjusted to balance the therapeutic benefit against any side effects. In an instance herein, the AAV dose is generally in the range of concentrations of from about 1 × 10⁵ to 1 × 10⁵⁰ genomes AAV, from about 1 × 10⁸ to 1 × 10²⁰ genomes AAV, from about 1 × 10¹⁰ to about 1 × 10¹⁶ genomes, or about 1 × 10¹¹ to about 1 × 10¹⁶ genomes AAV. A human dosage may be about 1 × 10¹³ genomes AAV. Such concentrations may be delivered in from about 0.001 ml to about 100 ml, about 0.05 to about 50 ml, or about 10 to about 25 ml of a carrier solution. In a preferred instance , AAV is used with a titer of about 2 × 10¹³ viral genomes/milliliter, and each of the striatal hemispheres of a mouse receives one 500 nanoliter injection. Other effective dosages can be readily established by one of ordinary skill in the art through routine trials establishing dose response curves. See, for example, U.S. Patent No. 8,404,658 B2 to Hajjar, et al., granted on March 26, 2013, at col. 27, lines 45-60.

In another instance effectively activating a cellular immune response for a neoplasia vaccine or immunogenic composition can be achieved by expressing the relevant neoantigens in a vaccine or immunogenic composition in a non-pathogenic microorganism. Well-known examples of such microorganisms are Mycobacterium bovis BCG, Salmonella and Pseudomona (See, U.S. Patent No. 6,991,797).

In another instance a Poxvirus is used in the neoplasia vaccine or immunogenic composition. These include orthopoxvirus, avipox, vaccinia, MVA, NYVAC, canarypox, ALVAC, fowlpox, TROVAC, etc. (see e.g., Verardiet al., Hum Vaccin Immunother. 2012 Jul;8(7):961-70; and Moss, Vaccine. 2013; 31(39): 4220-4222). Poxvirus expression vectors were described in 1982 and quickly became widely used for vaccine development as well as research in numerous fields. Advantages of the vectors include simple construction, ability to accommodate large amounts of foreign DNA and high expression levels.

In another instance the vaccinia virus is used in the neoplasia vaccine or immunogenic composition to express a neoantigen. (Rolph et al., Recombinant viruses as vaccines and immunological tools. Curr Opin Immunol 9:517-524, 1997). The recombinant vaccinia virus is able to replicate within the cytoplasm of the infected host cell and the polypeptide of interest can therefore induce an immune response. Moreover, Poxviruses have been widely used as vaccine or immunogenic composition vectors because of their ability to target encoded antigens for processing by the major histocompatibility complex class I pathway by directly infecting immune cells, in particular antigen-presenting cells, but also due to their ability to self-adjuvant.

In another instance ALVAC is used as a vector in a neoplasia vaccine or immunogenic composition. ALVAC is a canarypox virus that can be modified to express foreign transgenes and has been used as a method for vaccination against both prokaryotic and eukaryotic antigens (Horig H, Lee DS, Conkright W, et al. Phase I clinical trial of a recombinant canarypoxvirus (ALVAC) vaccine expressing human carcinoembryonic antigen and the B7.1 co-stimulatory molecule. Cancer Immunol Immunother 2000;49:504-14; von Mehren M, Arlen P, Tsang KY, et al. Pilot study of a dual gene recombinant avipox vaccine containing both carcinoembryonic antigen (CEA) and B7.1 transgenes in patients with recurrent CEA-expressing adenocarcinomas. Clin Cancer Res 2000;6:2219-28; Musey L, Ding Y, Elizaga M, et al. HIV-1 vaccination administered intramuscularly can induce both systemic and mucosal T cell immunity in HIV-1-uninfected individuals. J Immunol 2003;171:1094-101; Paoletti E. Applications of pox virus vectors to vaccination: an update. Proc Natl Acad Sci U S A 1996;93:11349-53; U.S. Patent No. 7,255,862). In a phase I clinical trial, an ALVAC virus expressing the tumor antigen CEA showed an excellent safety profile and resulted in increased CEA-specific T-cell responses in selected patients; objective clinical responses, however, were not observed (Marshall JL, Hawkins MJ, Tsang KY, et al. Phase I study in cancer patients of a replication-defective avipox recombinant vaccine that expresses human carcinoembryonic antigen. J Clin Oncol 1999;17:332-7).

In another instance a Modified Vaccinia Ankara (MVA) virus may be used as a viral vector for a neoantigen vaccine or immunogenic composition. MVA is a member of the Orthopoxvirus family and has been generated by about 570 serial passages on chicken embryo fibroblasts of the Ankara strain of Vaccinia virus (CVA) (for review see Mayr, A., et al., Infection 3, 6-14, 1975). As a consequence of these passages, the resulting MVA virus contains 31 kilobases less genomic information compared to CVA, and is highly host-cell restricted (Meyer, H. et al., J. Gen. Virol. 72, 1031-1038, 1991). MVA is characterized by its extreme attenuation, namely, by a diminished virulence or infectious ability, but still holds an excellent immunogenicity. When tested in a variety of animal models, MVA was proven to be avirulent, even in immuno-suppressed individuals. Moreover, MVA-BN^{®}-HER2 is a candidate immunotherapy designed for the treatment of HER-2-positive breast cancer and is currently in clinical trials. (Mandl et al., Cancer Immunol Immunother. Jan 2012; 61(1): 19-29). Methods to make and use recombinant MVA has been described (e.g., see U.S. Patent Nos. 8,309,098 and 5,185,146).

In another instance the modified Copenhagen strain of vaccinia virus, NYVAC and NYVAC variations are used as a vector (see U.S. Patent No. 7,255,862; PCT WO 95/30018; U.S. Pat. Nos. 5,364,773 and 5,494,807).

In one instance recombinant viral particles of the vaccine or immunogenic composition are administered to patients in need thereof. Dosages of expressed neoantigen can range from a few to a few hundred micrograms, e.g., 5 to 500 .mu.g. The vaccine or immunogenic composition can be administered in any suitable amount to achieve expression at these dosage levels. The viral particles can be administered to a patient in need thereof or transfected into cells in an amount of about at least 10^{3.5} pfu; thus, the viral particles are preferably administered to a patient in need thereof or infected or transfected into cells in at least about 10⁴ pfu to about 10⁶ pfu; however, a patient in need thereof can be administered at least about 10⁸ pfu such that a more preferred amount for administration can be at least about 10⁷ pfu to about 10⁹ pfu. Doses as to NYVAC are applicable as to ALVAC, MVA, MVA-BN, and avipoxes, such as canarypox and fowlpox.

### Vaccine or Immunogenic Composition Adjuvant

Effective vaccine or immunogenic compositions advantageously include a strong adjuvant to initiate an immune response. As described herein, poly-ICLC, an agonist of TLR3 and the RNA helicase -domains of MDA5 and RIG3, has shown several desirable properties for a vaccine or immunogenic composition adjuvant. These properties include the induction of local and systemic activation of immune cells in vivo, production of stimulatory chemokines and cytokines, and stimulation of antigen-presentation by DCs. Furthermore, poly-ICLC can induce durable CD4+ and CD8+ responses in humans. Importantly, striking similarities in the upregulation of transcriptional and signal transduction pathways were seen in subjects vaccinated with poly-ICLC and in volunteers who had received the highly effective, replication-competent yellow fever vaccine. Furthermore, >90% of ovarian carcinoma patients immunized with poly-ICLC in combination with a NY-ESO-1 peptide vaccine (in addition to Montanide) showed induction of CD4+ and CD8+ T cell, as well as antibody responses to the peptide in a recent phase 1 study. At the same time, poly-ICLC has been extensively tested in more than 25 clinical trials to date and exhibited a relatively benign toxicity profile. In addition to a powerful and specific immunogen the neoantigen peptides may be combined with an adjuvant (e.g., poly-ICLC) or another anti-neoplastic agent. Without being bound by theory, these neoantigens are expected to bypass central thymic tolerance (thus allowing stronger anti-tumor T cell response), while reducing the potential for autoimmunity (e.g., by avoiding targeting of normal self-antigens). An effective immune response advantageously includes a strong adjuvant to activate the immune system (Speiser and Romero, Molecularly defined vaccines for cancer immunotherapy, and protective T cell immunity Seminars in Immunol 22:144 (2010)). For example, Toll-like receptors (TLRs) have emerged as powerful sensors of microbial and viral pathogen "danger signals", effectively inducing the innate immune system, and in turn, the adaptive immune system (Bhardwaj and Gnjatic, TLR AGONISTS: Are They Good Adjuvants? Cancer J. 16:382-391 (2010)). Among the TLR agonists, poly-ICLC (a synthetic double-stranded RNA mimic) is one of the most potent activators of myeloid-derived dendritic cells. In a human volunteer study, poly-ICLC has been shown to be safe and to induce a gene expression profile in peripheral blood cells comparable to that induced by one of the most potent live attenuated viral vaccines, the yellow fever vaccine YF-17D (Caskey et al, Synthetic double-stranded RNA induces innate immune responses similar to a live viral vaccine in humans J Exp Med 208:2357 (2011)). In a preferred embodiment Hiltonol^{®}, a GMP preparation of poly-ICLC prepared by Oncovir, Inc, is utilized as the adjuvant. In other embodiments, other adjuvants described herein are envisioned. For instance oil-in-water, water-in-oil or multiphasic W/O/W; see, e.g., US 7,608,279 and Aucouturier et al, Vaccine 19 (2001), 2666-2672, and documents cited therein.

### Indications

Examples of cancers and cancer conditions that can be treated with the immunogenic composition or vaccine of this document include, but are not limited to a patient in need thereof that has been diagnosed as having cancer, or at risk of developing cancer. The subject may have a solid tumor such as breast, ovarian, prostate, lung, kidney, gastric, colon, testicular, head and neck, pancreas, brain, melanoma, and other tumors of tissue organs and hematological tumors, such as lymphomas and leukemias, including acute myelogenous leukemia, chronic myelogenous leukemia, chronic lymphocytic leukemia, T cell lymphocytic leukemia, and B cell lymphomas, tumors of the brain and central nervous system (e.g., tumors of the meninges, brain, spinal cord, cranial nerves and other parts of the CNS, such as glioblastomas or medulla blastomas); head and/or neck cancer, breast tumors, tumors of the circulatory system (e.g., heart, mediastinum and pleura, and other intrathoracic organs, vascular tumors, and tumor-associated vascular tissue); tumors of the blood and lymphatic system (e.g., Hodgkin's disease, Non-Hodgkin's disease lymphoma, Burkitt's lymphoma, AIDS-related lymphomas, malignant immunoproliferative diseases, multiple myeloma, and malignant plasma cell neoplasms, lymphoid leukemia, myeloid leukemia, acute or chronic lymphocytic leukemia, monocytic leukemia, other leukemias of specific cell type, leukemia of unspecified cell type, unspecified malignant neoplasms of lymphoid, hematopoietic and related tissues, such as diffuse large cell lymphoma, T-cell lymphoma or cutaneous T-cell lymphoma); tumors of the excretory system (e.g., kidney, renal pelvis, ureter, bladder, and other urinary organs); tumors of the gastrointestinal tract (e.g., esophagus, stomach, small intestine, colon, colorectal, rectosigmoid junction, rectum, anus, and anal canal); tumors involving the liver and intrahepatic bile ducts, gall bladder, and other parts of the biliary tract, pancreas, and other digestive organs; tumors of the oral cavity (e.g., lip, tongue, gum, floor of mouth, palate, parotid gland, salivary glands, tonsil, oropharynx, nasopharynx, puriform sinus, hypopharynx, and other sites of the oral cavity); tumors of the reproductive system (e.g., vulva, vagina, Cervix uteri, uterus, ovary, and other sites associated with female genital organs, placenta, penis, prostate, testis, and other sites associated with male genital organs); tumors of the respiratory tract (e.g., nasal cavity, middle ear, accessory sinuses, larynx, trachea, bronchus and lung, such as small cell lung cancer and non-small cell lung cancer); tumors of the skeletal system (e.g., bone and articular cartilage of limbs, bone articular cartilage and other sites); tumors of the skin (e.g., malignant melanoma of the skin, non-melanoma skin cancer, basal cell carcinoma of skin, squamous cell carcinoma of skin, mesothelioma, Kaposi's sarcoma); and tumors involving other tissues including peripheral nerves and autonomic nervous system, connective and soft tissue, retroperitoneoum and peritoneum, eye, thyroid, adrenal gland, and other endocrine glands and related structures, secondary and unspecified malignant neoplasms of lymph nodes, secondary malignant neoplasm of respiratory and digestive systems and secondary malignant neoplasm of other sites.

Of special interest is the treatment of Non-Hodgkin's Lymphoma (NHL), clear cell Renal Cell Carcinoma (ccRCC), metastatic melanoma, sarcoma, leukemia or a cancer of the bladder, colon, brain, breast, head and neck, endometrium, lung, ovary, pancreas or prostate. In certain embodiments, the melanoma is high risk melanoma.

Cancers that can be treated using this immunogenic composition or vaccine may include among others cases which are refractory to treatment with other chemotherapeutics. The term "refractory, as used herein refers to a cancer (and/or metastases thereof), which shows no or only weak antiproliferative response (e.g., no or only weak inhibition of tumor growth) after treatment with another chemotherapeutic agent. These are cancers that cannot be treated satisfactorily with other chemotherapeutics. Refractory cancers encompass not only (i) cancers where one or more chemotherapeutics have already failed during treatment of a patient, but also (ii) cancers that can be shown to be refractory by other means, e.g., biopsy and culture in the presence of chemotherapeutics.

The immunogenic composition or vaccine described herein is also applicable to the treatment of patients in need thereof who have not been previously treated.

The immunogenic composition or vaccine described herein is also applicable where the subject has no detectable neoplasia but is at high risk for disease recurrence.

Also of special interest is the treatment of patients in need thereof who have undergone Autologous Hematopoietic Stem Cell Transplant (AHSCT), and in particular patients who demonstrate residual disease after undergoing AHSCT. The post-AHSCT setting is characterized by a low volume of residual disease, the infusion of immune cells to a situation of homeostatic expansion, and the absence of any standard relapse-delaying therapy. These features provide a unique opportunity to use the described neoplastic vaccine or immunogenic composition to delay disease relapse.

### Pharmaceutical Compositions/Methods of Delivery

The present disclosure is also directed to pharmaceutical compositions comprising an effective amount of one or more compounds according to the present disclosure (including a pharmaceutically acceptable salt, thereof), optionally in combination with a pharmaceutically acceptable carrier, excipient or additive.

While the tumor specific neo-antigenic peptides can be administered as the sole active pharmaceutical agent, they can also be used in combination with one or more other agents and/or adjuvants. When administered as a combination, the therapeutic agents can be formulated as separate compositions that are given at the same time or different times, or the therapeutic agents can be given as a single composition.

The compositions may be administered once daily, twice daily, once every two days, once every three days, once every four days, once every five days, once every six days, once every seven days, once every two weeks, once every three weeks, once every four weeks, once every two months, once every six months, or once per year. The dosing interval can be adjusted according to the needs of individual patients. For longer intervals of administration, extended release or depot formulations can be used.

The compositions of the invention can be used to treat diseases and disease conditions that are acute, and may also be used for treatment of chronic conditions. In particular, the compositions of the invention are used in methods to treat or prevent a neoplasia. In certain instances, the compounds of the disclosure are administered for time periods exceeding two weeks, three weeks, one month, two months, three months, four months, five months, six months, one year, two years, three years, four years, or five years, ten years, or fifteen years; or for example, any time period range in days, months or years in which the low end of the range is any time period between 14 days and 15 years and the upper end of the range is between 15 days and 20 years (e.g., 4 weeks and 15 years, 6 months and 20 years). In some cases, it may be advantageous for the compounds of the disclosure to be administered for the remainder of the patient's life. In preferred embodiments, the patient is monitored to check the progression of the disease or disorder, and the dose is adjusted accordingly. In preferred embodiments, treatment according to the invention is effective for at least two weeks, three weeks, one month, two months, three months, four months, five months, six months, one year, two years, three years, four years, or five years, ten years, fifteen years, twenty years, or for the remainder of the subject's life.

The tumor specific neo-antigenic peptides may be administered by injection, orally, parenterally, by inhalation spray, rectally, vaginally, or topically in dosage unit formulations containing conventional pharmaceutically acceptable carriers, adjuvants, and vehicles. The term parenteral as used herein includes, into a lymph node or nodes, subcutaneous, intravenous, intramuscular, intrastemal, infusion techniques, intraperitoneally, eye or ocular, intravitreal, intrabuccal, transdermal, intranasal, into the brain, including intracranial and intradural, into the joints, including ankles, knees, hips, shoulders, elbows, wrists, directly into tumors, and the like, and in suppository form.

Surgical resection uses surgery to remove abnormal tissue in cancer, such as mediastinal, neurogenic, or germ cell tumors, or thymoma. In certain embodiments, administration of the neoplasia vaccine or immunogenic composition is initiated 1, 2, 3, 4, 5, 6, 7, 8, 9, 10, 11, 12, 13, 14, 15 or more weeks after tumor resection. Preferably, administration of the neoplasia vaccine or immunogenic composition is initiated 4, 5, 6, 7, 8, 9, 10, 11 or 12 weeks after tumor resection.

Prime/ boost regimens refer to the successive administrations of a vaccine or immunogenic or immunological compositions. In certain embodiments, administration of the neoplasia vaccine or immunogenic composition is in a prime/ boost dosing regimen, for example administration of the neoplasia vaccine or immunogenic composition at weeks 1, 2, 3 or 4 as a prime and administration of the neoplasia vaccine or immunogenic composition is at months 2, 3 or 4 as a boost. In another embodiment heterologous prime-boost strategies are used to ellicit a greater cytotoxic T-cell response (see Schneider et al., Induction of CD8+ T cells using heterologous prime-boost immunisation strategies, Immunological Reviews Volume 170, Issue 1, pages 29-38, August 1999). In another instance DNA encoding neoantigens is used to prime followed by a protein boost. In another instance protein is used to prime followed by boosting with a virus encoding the neoantigen. In another instance a virus encoding the neoantigen is used to prime and another virus is used to boost. In another instance protein is used to prime and DNA is used to boost. In a preferred instance a DNA vaccine or immunogenic composition is used to prime a T-cell response and a recombinant viral vaccine or immunogenic composition is used to boost the response. In another preferred instance a viral vaccine or immunogenic composition is coadministered with a protein or DNA vaccine or immunogenic composition to act as an adjuvant for the protein or DNA vaccine or immunogenic composition. The patient can then be boosted with either the viral vaccine or immunogenic composition, protein, or DNA vaccine or immunogenic composition (see Hutchings et al., Combination of protein and viral vaccines induces potent cellular and humoral immune responses and enhanced protection from murine malaria challenge. Infect Immun. 2007 Dec;75(12):5819-26. Epub 2007 Oct 1).

The pharmaceutical compositions can be processed in accordance with conventional methods of pharmacy to produce medicinal agents for administration to patients in need thereof, including humans and other mammals.

Modifications of the neoantigenic peptides can affect the solubility, bioavailability and rate of metabolism of the peptides, thus providing control over the delivery of the active species. Solubility can be assessed by preparing the neoantigenic peptide and testing according to known methods well within the routine practitioner's skill in the art.

It has been unexpectedly found that a pharmaceutical composition comprising succinic acid or a pharmaceutically acceptable salt thereof (succinate) can provide improved solubility for the neoantigenic peptides. Thus, in one instance the disclosure provides a pharmaceutical composition comprising: at least one neoantigenic peptide or a pharmaceutically acceptable salt thereof; a pH modifier (such as a base, such as a dicarboxylate or tricarboxylate salt, for example, a pharmaceutically acceptable salt of succinic acid or citric acid); and a pharmaceutically acceptable carrier. Such pharmaceutical compositions can be prepared by combining a solution comprising at least one neoantigenic peptide with a base, such as a dicarboxylate or tricarboxylate salt, such as a pharmaceutically acceptable salt of succinic acid or citric acid (such as sodium succinate), or by combining a solution comprising at least one neoantigenic peptide with a solution comprising a base, such as a dicarboxylate or tricarboxylate salt, such as a pharmaceutically acceptable salt of succinic acid or citric acid (including, e.g., a succinate buffer solution). In certain embodiments, the pharmaceutical composition comprises sodium succinate. In certain instances , the pH modifier (such as citrate or succinate) is present in the composition at a concentration from about 1 mM to about 10 mM, and, in certain instances , at a concentration from about 1.5 mM to about 7.5 mM, or about 2.0 to about 6.0 mM, or about 3.75 to about 5.0 mM. According to the invention, the pH modifier is succinate or citrate at a concentration from 1 mM to 10 mM.

According to the invention, the pharmaceutically acceptable carrier comprises water. In certain embodiments, the pharmaceutically acceptable carrier further comprises dextrose. According to the invention, the pharmaceutically acceptable carrier further comprises 1-4% dimethylsulfoxide. In certain embodiments, the pharmaceutical composition further comprises an immunomodulator or adjuvant. In certain embodiments, the immunodulator or adjuvant is selected from the group consisting of poly-ICLC, 1018 ISS, aluminum salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, JuvImmune, LipoVac, MF59, monophosphoryl lipid A, Montanide IMS 1312, Montanide ISA 206, Montanide ISA 50V, Montanide ISA-51, OK-432, OM-174, OM-197-MP-EC, ONTAK, PEPTEL, vector system, PLGA microparticles, resiquimod, SRL172, Virosomes and other Virus-like particles, YF-17D, VEGF trap, R848, beta-glucan, Pam3Cys, and Aquila's QS21 stimulon. In certain embodiments, the immunomodulator or adjuvant comprises poly-ICLC.

Xanthenone derivatives such as, for example, Vadimezan or AsA404 (also known as 5,6-dimethylaxanthenone-4-acetic acid (DMXAA)), may also be used as adjuvants according to embodiments of the invention. Alternatively, such derivatives may also be administered in parallel to the vaccine or immunogenic composition of the invention, for example via systemic or intratumoral delivery, to stimulate immunity at the tumor site. Without being bound by theory, it is believed that such xanthenone derivatives act by stimulating interferon (IFN) production via the stimulator of IFN gene ISTING) receptor (see e.g., Conlon et al. (2013) Mouse, but not Human STING, Binds and Signals in Response to the Vascular Disrupting Agent 5,6-Dimethylxanthenone-4-Acetic Acid, Journal of Immunology, 190:5216-25 and Kim et al. (2013) Anticancer Flavonoids are Mouse-Selective STING Agonists, 8:1396-1401).

The vaccine or immunological composition may also include an adjuvant compound chosen from the acrylic or methacrylic polymers and the copolymers of maleic anhydride and an alkenyl derivative. It is in particular a polymer of acrylic or methacrylic acid cross-linked with a polyalkenyl ether of a sugar or polyalcohol (carbomer), in particular cross-linked with an allyl sucrose or with allylpentaerythritol. It may also be a copolymer of maleic anhydride and ethylene cross-linked, for example, with divinyl ether (see U.S. Patent No. 6,713,068

In certain embodiments, the pH modifier can stabilize the adjuvant or immunomodulator as described herein.

In certain instances, a pharmaceutical composition comprises: one to five peptides, dimethylsulfoxide (DMSO), dextrose (or trehalose or sucrose), water, succinate, poly I: poly C, poly-L-lysine, carboxymethylcellulose, and chloride. In certain embodiments, each of the one to five peptides is present at a concentration of 300 µg/ml. In certain instances, the pharmaceutical composition comprises ≤ 3% DMSO by volume. In certain embodiments, the pharmaceutical composition comprises 3.6 - 3.7 % dextrose in water. In certain embodiments, the pharmaceutical composition comprises 3.6 - 3.7 mM succinate (e.g., as disodium succinate) or a salt thereof. In certain embodiments, the pharmaceutical composition comprises 0.5 mg/ml poly I: poly C. In certain embodiments, the pharmaceutical composition comprises 0.375 mg/ml poly-L-Lysine. In certain embodiments, the pharmaceutical composition comprises 1.25 mg/ml sodium carboxymethylcellulose. In certain embodiments, the pharmaceutical composition comprises 0.225% sodium chloride.

Pharmaceutical compositions comprise the herein-described tumor specific neoantigenic peptides in a therapeutically effective amount for treating diseases and conditions (e.g., a neoplasia/tumor), which have been described herein, optionally in combination with a pharmaceutically acceptable additive, carrier and/or excipient. One of ordinary skill in the art from this disclosure and the knowledge in the art will recognize that a therapeutically effective amount of one of more compounds according to the present invention may vary with the condition to be treated, its severity, the treatment regimen to be employed, the pharmacokinetics of the agent used, as well as the patient (animal or human) treated.

To prepare the pharmaceutical compositions according to the present invention, a therapeutically effective amount of one or more of the compounds according to the present disclosure is preferably intimately admixed with a pharmaceutically acceptable carrier according to conventional pharmaceutical compounding techniques to produce a dose. A carrier may take a wide variety of forms depending on the form of preparation desired for administration, e.g., ocular, oral, topical or parenteral, including gels, creams ointments, lotions and time released implantable preparations, among numerous others. In preparing pharmaceutical compositions in oral dosage form, any of the usual pharmaceutical media may be used. Thus, for liquid oral preparations such as suspensions, elixirs and solutions, suitable carriers and additives including water, glycols, oils, alcohols, flavoring agents, preservatives, coloring agents and the like may be used. For solid oral preparations such as powders, tablets, capsules, and for solid preparations such as suppositories, suitable carriers and additives including starches, sugar carriers, such as dextrose, mannitol, lactose and related carriers, diluents, granulating agents, lubricants, binders, disintegrating agents and the like may be used. If desired, the tablets or capsules may be entericcoated or sustained release by standard techniques.

The active compound is included in the pharmaceutically acceptable carrier or diluent in an amount sufficient to deliver to a patient a therapeutically effective amount for the desired indication, without causing serious toxic effects in the patient treated.

Oral compositions generally include an inert diluent or an edible carrier. They may be enclosed in gelatin capsules or compressed into tablets. For the purpose of oral therapeutic administration, the active compound or its prodrug derivative can be incorporated with excipients and used in the form of tablets, troches, or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a dispersing agent such as alginic acid or corn starch; a lubricant such as magnesium stearate; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material herein discussed, a liquid carrier such as a fatty oil. In addition, dosage unit forms can contain various other materials which modify the physical form of the dosage unit, for example, coatings of sugar, shellac, or enteric agents.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil emulsion and as a bolus, etc.

A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface-active or dispersing agent. Molded tablets may be made by molding in a suitable machine a mixture of the powdered compound moistened with an inert liquid diluent. The tablets optionally may be coated or scored and may be formulated so as to provide slow or controlled release of the active ingredient therein.

Methods of formulating such slow or controlled release compositions of pharmaceutically active ingredients, are known in the art and described in several issued US Patents, some of which include, but are not limited to, US Patent Nos. 3,870,790; 4,226,859; 4,369,172; 4,842,866 and 5,705,190. Coatings can be used for delivery of compounds to the intestine (see, e.g., U.S. Patent Nos. 6,638,534, 5,541,171, 5,217,720, and 6,569,457, and references cited therein).

The active compound or pharmaceutically acceptable salt thereof may also be administered as a component of an elixir, suspension, syrup, wafer, chewing gum or the like. A syrup may contain, in addition to the active compounds, sucrose or fructose as a sweetening agent and certain preservatives, dyes and colorings and flavors.

Solutions or suspensions used for ocular, parenteral, intradermal, subcutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates; and agents for the adjustment of tonicity such as sodium chloride or dextrose.

In certain instances the pharmaceutically acceptable carrier is an aqueous solvent, i.e., a solvent comprising water, optionally with additional co-solvents. Exemplary pharmaceutically acceptable carriers include water, buffer solutions in water (such as phosphatebuffered saline (PBS), and 5% dextrose in water (D5W) or 10% trehalose or 10% sucrose. In certain instances , the aqueous solvent further comprises dimethyl sulfoxide (DMSO), e.g., in an amount of about 1-4%, or 1-3%. In certain embodiments, the pharmaceutically acceptable carrier is isotonic (i.e., has substantially the same osmotic pressure as a body fluid such as plasma).

In one embodiment, the active compounds are prepared with carriers that protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid, and polylactic-co-glycolic acid (PLGA). Methods for preparation of such formulations are within the ambit of the skilled artisan in view of this disclosure and the knowledge in the art.

A skilled artisan from this disclosure and the knowledge in the art recognizes that in addition to tablets, other dosage forms can be formulated to provide slow or controlled release of the active ingredient. Such dosage forms include, but are not limited to, capsules, granulations and gel-caps.

Liposomal suspensions may also be pharmaceutically acceptable carriers. These may be prepared according to methods known to those skilled in the art. For example, liposomal formulations may be prepared by dissolving appropriate lipid(s) in an inorganic solvent that is then evaporated, leaving behind a thin film of dried lipid on the surface of the container. An aqueous solution of the active compound are then introduced into the container. The container is then swirled by hand to free lipid material from the sides of the container and to disperse lipid aggregates, thereby forming the liposomal suspension. Other methods of preparation well known by those of ordinary skill may also be used in this aspect of the present invention.

The formulations may conveniently be presented in unit dosage form and may be prepared by conventional pharmaceutical techniques. Such techniques include the step of bringing into association the active ingredient and the pharmaceutical carrier(s) or excipient(s). In general, the formulations are prepared by uniformly and intimately bringing into association the active ingredient with liquid carriers or finely divided solid carriers or both, and then, if necessary, shaping the product.

Formulations and compositions suitable for topical administration in the mouth include lozenges comprising the ingredients in a flavored basis, usually sucrose and acacia or tragacanth; pastilles comprising the active ingredient in an inert basis such as gelatin and glycerin, or sucrose and acacia; and mouthwashes comprising the ingredient to be administered in a suitable liquid carrier.

Formulations suitable for topical administration to the skin may be presented as ointments, creams, gels and pastes comprising the ingredient to be administered in a pharmaceutical acceptable carrier. A preferred topical delivery system is a transdermal patch containing the ingredient to be administered.

Formulations for rectal administration may be presented as a suppository with a suitable base comprising, for example, cocoa butter or a salicylate.

Formulations suitable for nasal administration, wherein the carrier is a solid, include a coarse powder having a particle size, for example, in the range of 20 to 500 microns which is administered in the manner in which snuff is administered, i.e., by rapid inhalation through the nasal passage from a container of the powder held close up to the nose. Suitable formulations, wherein the carrier is a liquid, for administration, as for example, a nasal spray or as nasal drops, include aqueous or oily solutions of the active ingredient.

Formulations suitable for vaginal administration may be presented as pessaries, tampons, creams, gels, pastes, foams or spray formulations containing in addition to the active ingredient such carriers as are known in the art to be appropriate.

The parenteral preparation can be enclosed in ampoules, disposable syringes or multiple dose vials made of glass or plastic. If administered intravenously, preferred carriers include, for example, physiological saline or phosphate buffered saline (PBS).

For parenteral formulations, the carrier usually comprises sterile water or aqueous sodium chloride solution, though other ingredients including those which aid dispersion may be included. Of course, where sterile water is to be used and maintained as sterile, the compositions and carriers are also sterilized. Injectable suspensions may also be prepared, in which case appropriate liquid carriers, suspending agents and the like may be employed.

Formulations suitable for parenteral administration include aqueous and non-aqueous sterile injection solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze-dried (lyophilized) condition requiring only the addition of the sterile liquid carrier, for example, water for injections, immediately prior to use. Extemporaneous injection solutions and suspensions may be prepared from sterile powders, granules and tablets of the kind previously described.

Administration of the active compound may range from continuous (intravenous drip) to several oral administrations per day (for example, Q.I.D.) and may include oral, topical, eye or ocular, parenteral, intramuscular, intravenous, sub-cutaneous, transdermal (which may include a penetration enhancement agent), buccal and suppository administration, among other routes of administration, including through an eye or ocular route.

The neoplasia vaccine or immunogenic composition may be administered by injection, orally, parenterally, by inhalation spray, rectally, vaginally, or topically in dosage unit formulations containing conventional pharmaceutically acceptable carriers, adjuvants, and vehicles. The term parenteral as used herein includes, into a lymph node or nodes, subcutaneous, intravenous, intramuscular, intrasternal, infusion techniques, intraperitoneally, eye or ocular, intravitreal, intrabuccal, transdermal, intranasal, into the brain, including intracranial and intradural, into the joints, including ankles, knees, hips, shoulders, elbows, wrists, directly into tumors, and the like, and in suppository form.

Various techniques can be used for providing the subject compositions at the site of interest, such as injection, use of catheters, trocars, projectiles, pluronic gel, stents, sustained drug release polymers or other device which provides for internal access. Where an organ or tissue is accessible because of removal from the patient, such organ or tissue may be bathed in a medium containing the subject compositions, the subject compositions may be painted onto the organ, or may be applied in any convenient way.

The tumor specific neoantigenic peptides may be administered through a device suitable for the controlled and sustained release of a composition effective in obtaining a desired local or systemic physiological or pharmacological effect. The method includes positioning the sustained released drug delivery system at an area wherein release of the agent is desired and allowing the agent to pass through the device to the desired area of treatment.

The tumor specific neoantigenic peptides may be utilized in combination with at least one known other therapeutic agent, or a pharmaceutically acceptable salt of said agent. Examples of known therapeutic agents which can be used include, but are not limited to, corticosteroids (e.g., cortisone, prednisone, dexamethasone), non-steroidal anti-inflammatory drugs (NSAIDS) (e.g., ibuprofen, celecoxib, aspirin, indomethicin, naproxen), alkylating agents such as busulfan, cis-platin, mitomycin C, and carboplatin; antimitotic agents such as colchicine, vinblastine, paclitaxel, and docetaxel; topo I inhibitors such as camptothecin and topotecan; topo II inhibitors such as doxorubicin and etoposide; and/or RNA/DNA antimetabolites such as 5-azacytidine, 5-fluorouracil and methotrexate; DNA antimetabolites such as 5-fluoro-2'-deoxy-uridine, ara-C, hydroxyurea and thioguanine; antibodies such as HERCEPTIN and RITUXAN.

It should be understood that in addition to the ingredients particularly mentioned herein, the formulations of the present invention may include other agents conventional in the art having regard to the type of formulation in question, for example, those suitable for oral administration may include flavoring agents.

Pharmaceutically acceptable salt forms may be the preferred chemical form of compounds according to the present invention for inclusion in pharmaceutical compositions according to the present invention.

The present compounds or their derivatives, including prodrug forms of these agents, can be provided in the form of pharmaceutically acceptable salts. As used herein, the term pharmaceutically acceptable salts or complexes refers to appropriate salts or complexes of the active compounds according to the present disclosure which retain the desired biological activity of the parent compound and exhibit limited toxicological effects to normal cells. Nonlimiting examples of such salts are (a) acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, and polyglutamic acid, among others; (b) base addition salts formed with metal cations such as zinc, calcium, sodium, potassium, and the like, among numerous others.

The compounds herein are commercially available or can be synthesized. As can be appreciated by the skilled artisan, further methods of synthesizing the compounds of the formulae herein is evident to those of ordinary skill in the art. Additionally, the various synthetic steps may be performed in an alternate sequence or order to give the desired compounds. Synthetic chemistry transformations and protecting group methodologies (protection and deprotection) useful in synthesizing the compounds described herein are known in the art and include, for example, those such as described in R. Larock, Comprehensive Organic Transformations, 2nd. Ed., Wiley-VCH Publishers (1999); T.W. Greene and P.G.M. Wuts, Protective Groups in Organic Synthesis, 3rd. Ed., John Wiley and Sons (1999); L. Fieser and M. Fieser, Fieser and Fieser's Reagents for Organic Synthesis, John Wiley and Sons (1999); and L. Paquette, ed., Encyclopedia of Reagents for Organic Synthesis, John Wiley and Sons (1995), and subsequent editions thereof.

The additional agents that may be included with the tumor specific neo-antigenic peptides of this disclosure may contain one or more asymmetric centers and thus occur as racemates and racemic mixtures, single enantiomers, individual diastereomers and diastereomeric mixtures. All such isomeric forms of these compounds are expressly included in the present disclosure . The compounds of this disclosure may also be represented in multiple tautomeric forms, in such instances, the disclosure expressly includes all tautomeric forms of the compounds described herein (e.g., alkylation of a ring system may result in alkylation at multiple sites, the invention expressly includes all such reaction products). All such isomeric forms of such compounds are expressly included in the present disclosure . All crystal forms of the compounds described herein are expressly included in the present disclosure .

### Dosage

When the agents described herein are administered as pharmaceuticals to humans or animals, they can be given per se or as a pharmaceutical composition containing active ingredient in combination with a pharmaceutically acceptable carrier, excipient, or diluent.

Actual dosage levels and time course of administration of the active ingredients in the pharmaceutical compositions of the invention can be varied so as to obtain an amount of the active ingredient which is effective to achieve the desired therapeutic response for a particular patient, composition, and mode of administration, without being toxic to the patient. Generally, agents or pharmaceutical compositions of the disclosure 1 are administered in an amount sufficient to reduce or eliminate symptoms associated with viral infection and/or autoimmune disease.

A preferred dose of an agent is the maximum that a patient can tolerate and not develop serious or unacceptable side effects. Exemplary dose ranges include 0.01 mg to 250 mg per day, 0.01 mg to 100 mg per day, 1 mg to 100 mg per day, 10 mg to 100 mg per day, 1 mg to 10 mg per day, and 0.01 mg to 10 mg per day. A preferred dose of an agent is the maximum that a patient can tolerate and not develop serious or unacceptable side effects. In embodiments, the agent is administered at a concentration of about 10 micrograms to about 100 mg per kilogram of body weight per day, about 0.1 to about 10 mg/kg per day, or about 1.0 mg to about 10 mg/kg of body weight per day.

In embodiments, the pharmaceutical composition comprises an agent in an amount ranging between 1 and 10 mg, such as 1, 2, 3, 4, 5, 6, 7, 8, 9, or 10 mg.

In embodiments, the therapeutically effective dosage produces a serum concentration of an agent of from about 0.1 ng/ml to about 50-100 mglml. The pharmaceutical compositions 5 typically should provide a dosage of from about 0.001 mg to about 2000 mg of compound per kilogram of body weight per day. For example, dosages for systemic administration to a human patient can range from 1-10 mglkg, 20-80 mglkg, 5-50 mg/kg, 75-150 mg/kg, 100-500 mglkg, 250-750 mglkg, 500-1000 mglkg, 1-10 mg/kg, 5-50 mg/kg, 25-75 mg/kg, 50-100 mg/kg, 100-250 mg/kg, 50-100 mg/kg, 250-500 mg/kg, 500-750 mg/kg, 750-1000 mg/kg, 1000-1500 mg/kg, 10 1500-2000 mg/kg, 5 mg/kg, 20 mg/kg, 50 mg/kg, 100 mg/kg, 500 mg/kg, 1000 mg/kg, 1500 mg/kg, or 2000 mg/kg. Pharmaceutical dosage unit forms are prepared to provide from about 1 mg to about 5000 mg, for example from about 100 to about 2500 mg of the compound or a combination of essential ingredients per dosage unit form.

In embodiments, about 50 nM to about 1µM of an agent is administered to a subject. In related embodiments, about 50-100 nM, 50-250 nM, 100-500 nM, 250-500 nM, 250-750 nM, 500-750 nM, 500 nM to 1µM, or 750 nM to 1µM of an agent is administered to a subject.

Determination of an effective amount is well within the capability of those skilled in the art, especially in light of the detailed disclosure provided herein. Generally, an efficacious or effective amount of an agent is determined by first administering a low dose of the agent(s) and then incrementally increasing the administered dose or dosages until a desired effect (e.g., reduce or eliminate symptoms associated with viral infection or autoimmune disease) is observed in the treated subject, with minimal or acceptable toxic side effects. Applicable methods for determining an appropriate dose and dosing schedule for administration of a pharmaceutical composition of the present invention are described, for example, in Goodman and Gilman's The Pharmacological Basis of Therapeutics, Goodman et al., eds., 11th Edition, McGraw-Hill 2005, and Remington: The Science and Practice of Pharmacy, 20th and 21st Editions, Gennaro and University of the Sciences in Philadelphia, Eds., Lippencott Williams & Wilkins (2003 and 2005) .

Preferred unit dosage formulations are those containing a daily dose or unit, daily sub-dose, as herein discussed, or an appropriate fraction thereof, of the administered ingredient.

The dosage regimen for treating a disorder or a disease with the tumor specific neoantigenic peptides of this disclosure and/or compositions of this invention is based on a variety of factors, including the type of disease, the age, weight, sex, medical condition of the patient, the severity of the condition, the route of administration, and the particular compound employed. Thus, the dosage regimen may vary widely, but can be determined routinely using standard methods.

The amounts and dosage regimens administered to a subject can depend on a number of factors, such as the mode of administration, the nature of the condition being treated, the body weight of the subject being treated and the judgment of the prescribing physician; all such factors being within the ambit of the skilled artisan from this disclosure and the knowledge in the art.

The amount of compound included within therapeutically active formulations according to the present invention is an effective amount for treating the disease or condition. In general, a therapeutically effective amount of the present preferred compound in dosage form usually ranges from slightly less than about 0.025 mg/kg/day to about 2.5 g/kg/day, preferably about 0.1 mg/kg/day to about 100 mg/kg/day of the patient or considerably more, depending upon the compound used, the condition or infection treated and the route of administration, although exceptions to this dosage range may be contemplated by the present invention. In its most preferred form, compounds according to the present disclosure are administered in amounts ranging from about 1 mg/kg/day to about 100 mg/kg/day. The dosage of the compound can depend on the condition being treated, the particular compound, and other clinical factors such as weight and condition of the patient and the route of administration of the compound. It is to be understood that the present invention has application for both human and veterinary use.

For oral administration to humans, a dosage of between approximately 0.1 to 100 mg/kg/day, preferably between approximately 1 and 100 mg/kg/day, is generally sufficient.

Where drug delivery is systemic rather than topical, this dosage range generally produces effective blood level concentrations of active compound ranging from less than about 0.04 to about 400 micrograms/cc or more of blood in the patient. The compound is conveniently administered in any suitable unit dosage form, including but not limited to one containing 0.001 to 3000 mg, preferably 0.05 to 500 mg of activeingredient per unit dosage form. An oral dosage of 10-250 mg is usually convenient.

According to certain exemplary embodiments, the vaccine or immunogenic composition is administered at a dose of about 10 µg- 1 mg per neoantigenic peptide. According to certain exemplary embodiments, the vaccine or immunogenic composition is administered at an average weekly dose level of about 10 µg- 2000 µg per neoantigenic peptide.

The concentration of active compound in the drug composition will depend on absorption, distribution, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

The invention provides for pharmaceutical compositions containing at least one tumor specific neoantigen described herein. In embodiments, the pharmaceutical compositions contain a pharmaceutically acceptable carrier, excipient, or diluent, which includes any pharmaceutical agent that does not itself induce the production of an immune response harmful to a subject receiving the composition, and which may be administered without undue toxicity. As used herein, the term "pharmaceutically acceptable" means being approved by a regulatory agency of the Federal or a state government or listed in the U.S. Pharmacopia, European Pharmacopia or other generally recognized pharmacopia for use in mammals, and more particularly in humans. These compositions can be useful for treating and/or preventing viral infection and/or autoimmune disease.

A thorough discussion of pharmaceutically acceptable carriers, diluents, and other excipients is presented in Remington's Pharmaceutical Sciences (17th ed., Mack Publishing Company) and Remington: The Science and Practice of Pharmacy (21st ed., Lippincott Williams & Wilkins) . The formulation of the pharmaceutical composition should suit the mode of administration. In embodiments, the pharmaceutical composition is suitable for administration to humans, and can be sterile, non-particulate and/or non-pyrogenic.

Pharmaceutically acceptable carriers, excipients, or diluents include, but are not limited, to saline, buffered saline, dextrose, water, glycerol, ethanol, sterile isotonic aqueous buffer, and combinations thereof.

Wetting agents, emulsifiers and lubricants, such as sodium lauryl sulfate and magnesium stearate, as well as coloring agents, release agents, coating agents, sweetening, flavoring and perfuming agents, preservatives, and antioxidants can also be present in the compositions.

Examples of pharmaceutically-acceptable antioxidants include, but are not limited to: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

In embodiments, the pharmaceutical composition is provided in a solid form, such as a lyophilized powder suitable for reconstitution, a liquid solution, suspension, emulsion, tablet, pill, capsule, sustained release formulation, or powder.

In embodiments, the pharmaceutical composition is supplied in liquid form, for example, in a sealed container indicating the quantity and concentration of the active ingredient in the pharmaceutical composition. In related embodiments, the liquid form of the pharmaceutical composition is supplied in a hermetically sealed container.

Methods for formulating the pharmaceutical compositions of the present invention are conventional and well known in the art (see Remington and Remington's). One of skill in the art can readily formulate a pharmaceutical composition having the desired characteristics (e.g., route of administration, biosafety, and release profile).

Methods for preparing the pharmaceutical compositions include the step of bringing into association the active ingredient with a pharmaceutically acceptable carrier and, optionally, one or more accessory ingredients. The pharmaceutical compositions can be prepared by uniformly and intimately bringing into association the active ingredient with liquid carriers, or finely divided solid carriers, or both, and then, if necessary, shaping the product. Additional methodology for preparing the pharmaceutical compositions, including the preparation of multilayer dosage forms, are described in Ansel's Pharmaceutical Dosage Forms and Drug Delivery Systems (9th ed., Lippincott Williams & Wilkins) .

Pharmaceutical compositions suitable for oral administration can be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound(s) described herein, a derivative thereof, or a pharmaceutically acceptable salt or prodrug thereof as the active ingredient(s). The active ingredient can also be administered as a bolus, electuary, or paste.

In solid dosage forms for oral administration (e.g., capsules, tablets, pills, dragees, powders, granules and the like), the active ingredient is mixed with one or more pharmaceutically acceptable carriers, excipients, or diluents, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, acetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets, and pills, the pharmaceutical compositions can also comprise buffering agents. Solid compositions of a similar type can also be prepared using fillers in soft and hard-filled gelatin capsules, and excipients such as lactose or milk sugars, as well as high molecular weight polyethylene glycols and the like.

A tablet can be made by compression or molding, optionally with one or more accessory ingredients. Compressed tablets can be prepared using binders (for example, gelatin or hydroxypropylmethyl cellulose), lubricants, inert diluents, preservatives, disintegrants (for example, sodium starch glycolate or cross-linked sodium carboxymethyl cellulose), surfaceactives, and/ or dispersing agents. Molded tablets can be made by molding in a suitable machine a mixture of the powdered active ingredient moistened with an inert liquid diluent.

The tablets and other solid dosage forms, such as dragees, capsules, pills, and granules, can optionally be scored or prepared with coatings and shells, such as enteric coatings and other coatings well known in the art.

In some embodiments, in order to prolong the effect of an active ingredient, it is desirable to slow the absorption of the compound from subcutaneous or intramuscular injection. This can be accomplished by the use of a liquid suspension of crystalline or amorphous material having poor water solubility. The rate of absorption of the active ingredient then depends upon its rate of dissolution which, in turn, can depend upon crystal size and crystalline form. Alternatively, delayed absorption of a parenterally-administered active ingredient is accomplished by dissolving or suspending the compound in an oil vehicle. In addition, prolonged absorption of the injectable pharmaceutical form can be brought about by the inclusion of agents that delay absorption such as aluminum monostearate and gelatin.

Controlled release parenteral compositions can be in form of aqueous suspensions, microspheres, microcapsules, magnetic microspheres, oil solutions, oil suspensions, emulsions, or the active ingredient can be incorporated in biocompatible carrier(s), liposomes, nanoparticles, implants or infusion devices.

Materials for use in the preparation of microspheres and/or microcapsules include biodegradable/bioerodible polymers such as polyglactin, poly-(isobutyl cyanoacrylate), poly(2-hydroxyethyl-L-glutamine) and poly(lactic acid).

Biocompatible carriers which can be used when formulating a controlled release parenteral formulation include carbohydrates such as dextrans, proteins such as albumin, lipoproteins or antibodies.

Materials for use in implants can be non-biodegradable, e.g., polydimethylsiloxane, or biodegradable such as, e.g., poly(caprolactone), poly(lactic acid), poly(glycolic acid) or poly(ortho esters).

In embodiments, the active ingredient(s) are administered by aerosol. This is accomplished by preparing an aqueous aerosol, liposomal preparation, or solid particles containing the compound. A nonaqueous (e.g., fluorocarbon propellant) suspension can be used. The pharmaceutical composition can also be administered using a sonic nebulizer, which would minimize exposing the agent to shear, which can result in degradation of the compound.

Ordinarily, an aqueous aerosol is made by formulating an aqueous solution or suspension of the active ingredient(s) together with conventional pharmaceutically-acceptable carriers and stabilizers. The carriers and stabilizers vary with the requirements of the particular compound, but typically include nonionic surfactants (Tweens, Pluronics, or polyethylene glycol), innocuous proteins like serum albumin, sorbitan esters, oleic acid, lecithin, amino acids such as glycine, buffers, salts, sugars or sugar alcohols. Aerosols generally are prepared from isotonic solutions.

Dosage forms for topical or transdermal administration of an active ingredient(s) includes powders, sprays, ointments, pastes, creams, lotions, gels, solutions, patches and inhalants. The active ingredient(s) can be mixed under sterile conditions with a pharmaceutically acceptable carrier, and with any preservatives, buffers, or propellants as appropriate.

Transdermal patches suitable for use in the present invention are disclosed in Transdermal Drug Delivery: Developmental Issues and Research Initiatives (Marcel Dekker Inc., 1989) and U.S. Pat. Nos. 4,743,249, 4,906,169, 5,198,223, 4,816,540, 5,422,119, 5,023,084 . The transdermal patch can also be any transdermal patch well known in the art, including transscrotal patches. Pharmaceutical compositions in such transdermal patches can contain one or more absorption enhancers or skin permeation enhancers well known in the art (see, e.g., U.S. Pat. Nos. 4,379,454 and 4,973,468, which are hereby incorporated by reference). Transdermal therapeutic systems for use in the present invention can be based on iontophoresis, diffusion, or a combination of these two effects.

Transdermal patches have the added advantage of providing controlled delivery of active ingredient(s) to the body. Such dosage forms can be made by dissolving or dispersing the active ingredient(s) in a proper medium. Absorption enhancers can also be used to increase the flux of the active ingredient across the skin. The rate of such flux can be controlled by either providing a rate controlling membrane or dispersing the active ingredient(s) in a polymer matrix or gel.

Such pharmaceutical compositions can be in the form of creams, ointments, lotions, liniments, gels, hydrogels, solutions, suspensions, sticks, sprays, pastes, plasters and other kinds of transdermal drug delivery systems. The compositions can also include pharmaceutically acceptable carriers or excipients such as emulsifying agents, antioxidants, buffering agents, preservatives, humectants, penetration enhancers, chelating agents, gel-forming agents, ointment bases, perfumes, and skin protective agents.

Examples of emulsifying agents include, but are not limited to, naturally occurring gums, e.g. gum acacia or gum tragacanth, naturally occurring phosphatides, e.g. soybean lecithin and sorbitan monooleate derivatives.

Examples of antioxidants include, but are not limited to, butylated hydroxy anisole (BHA), ascorbic acid and derivatives thereof, tocopherol and derivatives thereof, and cysteine.

Examples of preservatives include, but are not limited to, parabens, such as methyl or propyl p-hydroxybenzoate and benzalkonium chloride.

Examples of humectants include, but are not limited to, glycerin, propylene glycol, sorbitol and urea.

Examples of penetration enhancers include, but are not limited to, propylene glycol, DMSO, triethanolamine, N,N-dimethylacetamide, N,N-dimethylformamide, 2-pyrrolidone and derivatives thereof, tetrahydrofurfuryl alcohol, propylene glycol, diethylene glycol monoethyl or monomethyl ether with propylene glycol monolaurate or methyl laurate, eucalyptol, lecithin, TRANSCUTOL, and AZONE.

Examples of chelating agents include, but are not limited to, sodium EDTA, citric acid and phosphoric acid.

Examples of gel forming agents include, but are not limited to, Carbopol, cellulose derivatives, bentonite, alginates, gelatin and polyvinylpyrrolidone.

In addition to the active ingredient(s), the ointments, pastes, creams, and gels of the present disclosure can contain excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain excipients such as lactose, talc, silicic acid, aluminum hydroxide, calcium silicates and polyamide powder, or mixtures of these substances. Sprays can additionally contain customary propellants, such as chlorofluorohydrocarbons, and volatile unsubstituted hydrocarbons, such as butane and propane.

Injectable depot forms are made by forming microencapsule matrices of compound(s) of the disclosure in biodegradable polymers such as polylactide-polyglycolide. Depending on the ratio of compound to polymer, and the nature of the particular polymer employed, the rate of compound release can be controlled. Examples of other biodegradable polymers include poly(orthoesters) and poly(anhydrides). Depot injectable formulations are also prepared by entrapping the drug in liposomes or microemulsions which are compatible with body tissue.

Subcutaneous implants are well known in the art and are suitable for use in the present invention. Subcutaneous implantation methods are preferably non-irritating and mechanically resilient. The implants can be of matrix type, of reservoir type, or hybrids thereof. In matrix type devices, the carrier material can be porous or non-porous, solid or semi-solid, and permeable or impermeable to the active compound or compounds. The carrier material can be biodegradable or may slowly erode after administration. In some instances, the matrix is nondegradable but instead relies on the diffusion of the active compound through the matrix for the carrier material to degrade. Alternative subcutaneous implant methods utilize reservoir devices where the active compound or compounds are surrounded by a rate controlling membrane, e.g., a membrane independent of component concentration (possessing zero-order kinetics). Devices consisting of a matrix surrounded by a rate controlling membrane also suitable for use.

Both reservoir and matrix type devices can contain materials such as polydimethylsiloxane, such as SILASTIC, or other silicone rubbers. Matrix materials can be insoluble polypropylene, polyethylene, polyvinyl chloride, ethylvinyl acetate, polystyrene and polymethacrylate, as well as glycerol esters of the glycerol palmitostearate, glycerol stearate, and glycerol behenate type. Materials can be hydrophobic or hydrophilic polymers and optionally contain solubilizing agents.

Subcutaneous implant devices can be slow-release capsules made with any suitable polymer, e.g., as described in U.S. Pat. Nos. 5,035,891 and 4,210,644 .

In general, at least four different approaches are applicable in order to provide rate control over the release and transdermal permeation of a drug compound. These approaches are: membrane-moderated systems, adhesive diffusion-controlled systems, matrix dispersion-type systems and microreservoir systems. It is appreciated that a controlled release percutaneous and/or topical composition can be obtained by using a suitable mixture of these approaches.

In a membrane-moderated system, the active ingredient is present in a reservoir which is totally encapsulated in a shallow compartment molded from a drug-impermeable laminate, such as a metallic plastic laminate, and a rate-controlling polymeric membrane such as a microporous or a non-porous polymeric membrane, e.g., ethylene-vinyl acetate copolymer. The active ingredient is released through the rate controlling polymeric membrane. In the drug reservoir, the active ingredient can either be dispersed in a solid polymer matrix or suspended in an unleachable, viscous liquid medium such as silicone fluid. On the external surface of the polymeric membrane, a thin layer of an adhesive polymer is applied to achieve an intimate contact of the transdermal system with the skin surface. The adhesive polymer is preferably a polymer which is hypoallergenic and compatible with the active drug substance.

In an adhesive diffusion-controlled system, a reservoir of the active ingredient is formed by directly dispersing the active ingredient in an adhesive polymer and then by, e.g., solvent casting, spreading the adhesive containing the active ingredient onto a flat sheet of substantially drug-impermeable metallic plastic backing to form a thin drug reservoir layer.

A matrix dispersion-type system is characterized in that a reservoir of the active ingredient is formed by substantially homogeneously dispersing the active ingredient in a hydrophilic or lipophilic polymer matrix. The drug-containing polymer is then molded into disc with a substantially well-defined surface area and controlled thickness. The adhesive polymer is spread along the circumference to form a strip of adhesive around the disc.

A microreservoir system can be considered as a combination of the reservoir and matrix dispersion type systems. In this case, the reservoir of the active substance is formed by first suspending the drug solids in an aqueous solution of water-soluble polymer and then dispersing the drug suspension in a lipophilic polymer to form a multiplicity of unleachable, microscopic spheres of drug reservoirs.

Any of the herein-described controlled release, extended release, and sustained release compositions can be formulated to release the active ingredient in about 30 minutes to about 1 week, in about 30 minutes to about 72 hours, in about 30 minutes to 24 hours, in about 30 minutes to 12 hours, in about 30 minutes to 6 hours, in about 30 minutes to 4 hours, and in about 3 hours to 10 hours. In embodiments, an effective concentration of the active ingredient(s) is sustained in a subject for 4 hours, 6 hours, 8 hours, 10 hours, 12 hours, 16 hours, 24 hours, 48 hours, 72 hours, or more after administration of the pharmaceutical compositions to the subject.

### Vaccine or immunogenic compositions

The present invention is directed to an immunogenic composition, e.g., a neoplasia vaccine or immunogenic composition capable of raising a specific T-cell response. The neoplasia vaccine or immunogenic composition comprises neoantigenic peptides and/or neoantigenic polypeptides corresponding to tumor specific neoantigens identified by the methods described herein.

A suitable neoplasia vaccine or immunogenic composition can preferably contain a plurality of tumor specific neoantigenic peptides. In an embodiment, the vaccine or immunogenic composition can include between 1 and 100 sets of peptides, more preferably between 1 and 50 such peptides, even more preferably between 10 and 30 sets peptides, even more preferably between 15 and 25 peptides. According to another preferred embodiment, the vaccine or immunogenic composition can include at least one peptides, more preferably 2, 3, 4, or 5 peptides, In certain embodiments, the vaccine or immunogenic composition can comprise 5, 6, 7, 8, 9, 10, 11, 12, 13, 14, 15, 16, 17, 18, 19, 20, 21, 22, 23, 24, 25, 26, 27, 28, 29, or 30 different peptides.

The optimum amount of each peptide to be included in the vaccine or immunogenic composition and the optimum dosing regimen can be determined by one skilled in the art without undue experimentation. For example, the peptide or its variant may be prepared for intravenous (i.v.) injection, sub-cutaneous (s.c.) injection, intradermal (i.d.) injection, intraperitoneal (i.p.) injection, intramuscular (i.m.) injection. Preferred methods of peptide injection include s.c, i.d., i.p., i.m., and i.v. Preferred methods of DNA injection include i.d., i.m., s.c, i.p. and i.v. For example, doses of between 1 and 500 mg 50 µg and 1.5 mg, preferably 10 µg to 500 µg, of peptide or DNA may be given and can depend from the respective peptide or DNA. Doses of this range were successfully used in previous trials (Brunsvig P F, et al., Cancer Immunol Immunother. 2006; 55(12): 1553- 1564; M. Staehler, et al., ASCO meeting 2007; Abstract No 3017). Other methods of administration of the vaccine or immunogenic composition are known to those skilled in the art.

In one instance of the present disclosure the different tumor specific neoantigenic peptides and/or polypeptides are selected for use in the neoplasia vaccine or immunogenic composition so as to maximize the likelihood of generating an immune attack against the neoplasia/tumor of the patient. Without being bound by theory, it is believed that the inclusion of a diversity of tumor specific neoantigenic peptides can generate a broad scale immune attack against a neoplasia/tumor. In one embodiment, the selected tumor specific neoantigenic peptides/polypeptides are encoded by missense mutations. In a second embodiment, the selected tumor specific neoantigenic peptides/polypeptides are encoded by a combination of missense mutations and neoORF mutations. In a third embodiment, the selected tumor specific neoantigenic peptides/polypeptides are encoded by neoORF mutations.

In one embodiment in which the selected tumor specific neoantigenic peptides/polypeptides are encoded by missense mutations, the peptides and/or polypeptides are chosen based on their capability to associate with the particular MHC molecules of the patient. Peptides/polypeptides derived from neoORF mutations can also be selected on the basis of their capability to associate with the particular MHC molecules of the patient, but can also be selected even if not predicted to associate with the particular MHC molecules of the patient.

The vaccine or immunogenic composition is capable of raising a specific cytotoxic T-cells response and/or a specific helper T-cell response.

The vaccine or immunogenic composition can further comprise an adjuvant and/or a carrier. Examples of useful adjuvants and carriers are given herein herein. The peptides and/or polypeptides in the composition can be associated with a carrier such as, e.g., a protein or an antigen-presenting cell such as e.g. a dendritic cell (DC) capable of presenting the peptide to a T-cell.

Adjuvants are any substance whose admixture into the vaccine or immunogenic composition increases or otherwise modifies the immune response to the mutant peptide. Carriers are scaffold structures, for example a polypeptide or a polysaccharide, to which the neoantigenic peptides, is capable of being associated. Optionally, adjuvants are conjugated covalently or non-covalently to the peptides or polypeptides of the disclosure.

The ability of an adjuvant to increase the immune response to an antigen is typically manifested by a significant increase in immune-mediated reaction, or reduction in disease symptoms. For example, an increase in humoral immunity is typically manifested by a significant increase in the titer of antibodies raised to the antigen, and an increase in T-cell activity is typically manifested in increased cell proliferation, or cellular cytotoxicity, or cytokine secretion. An adjuvant may also alter an immune response, for example, by changing a primarily humoral or Th2 response into a primarily cellular, or Th1 response.

Suitable adjuvants include, but are not limited to 1018 ISS, aluminum salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, JuvImmune, LipoVac, MF59, monophosphoryl lipid A, Montanide IMS 1312, Montanide ISA 206, Montanide ISA 50V, Montanide ISA-51, OK-432, OM-174, OM-197-MP-EC, ONTAK, PEPTEL. vector system, PLG microparticles, resiquimod, SRL172, Virosomes and other Virus-like particles, YF-17D, VEGF trap, R848, beta-glucan, Pam3Cys, Aquila's QS21 stimulon (Aquila Biotech, Worcester, Mass., USA) which is derived from saponin, mycobacterial extracts and synthetic bacterial cell wall mimics, and other proprietary adjuvants such as Ribi's Detox. Quil or Superfos. Several immunological adjuvants (e.g., MF59) specific for dendritic cells and their preparation have been described previously (Dupuis M, et al., Cell Immunol. 1998; 186(1): 18-27; Allison A C; Dev Biol Stand. 1998; 92:3-11). Also cytokines may be used. Several cytokines have been directly linked to influencing dendritic cell migration to lymphoid tissues (e.g., TNF-alpha), accelerating the maturation of dendritic cells into efficient antigen-presenting cells for T-lymphocytes (e.g., GM-CSF, IL-1 and IL-4) (U.S. Pat. No. 5,849,589 ) and acting as immunoadjuvants (e.g., IL-12) (Gabrilovich D I, et al., J Immunother Emphasis Tumor Immunol. 1996 (6):414-418).

Toll like receptors (TLRs) may also be used as adjuvants, and are important members of the family of pattern recognition receptors (PRRs) which recognize conserved motifs shared by many micro-organisms, termed "pathogen-associated molecular patterns" (PAMPS). Recognition of these "danger signals" activates multiple elements of the innate and adaptive immune system. TLRs are expressed by cells of the innate and adaptive immune systems such as dendritic cells (DCs), macrophages, T and B cells, mast cells, and granulocytes and are localized in different cellular compartments, such as the plasma membrane, lysosomes, endosomes, and endolysosomes. Different TLRs recognize distinct PAMPS. For example, TLR4 is activated by LPS contained in bacterial cell walls, TLR9 is activated by unmethylated bacterial or viral CpG DNA, and TLR3 is activated by double stranded RNA. TLR ligand binding leads to the activation of one or more intracellular signaling pathways, ultimately resulting in the production of many key molecules associated with inflammation and immunity (particularly the transcription factor NF-κB and the Type-I interferons). TLR mediated DC activation leads to enhanced DC activation, phagocytosis, upregulation of activation and co-stimulation markers such as CD80, CD83, and CD86, expression of CCR7 allowing migration of DC to draining lymph nodes and facilitating antigen presentation to T cells, as well as increased secretion of cytokines such as type I interferons, IL-12, and IL-6. All of these downstream events are critical for the induction of an adaptive immune response.

Among the most promising cancer vaccine or immunogenic composition adjuvants currently in clinical development are the TLR9 agonist CpG and the synthetic double-stranded RNA (dsRNA) TLR3 ligand poly-ICLC. In preclinical studies poly-ICLC appears to be the most potent TLR adjuvant when compared to LPS and CpG due to its induction of pro-inflammatory cytokines and lack of stimulation of IL-10, as well as maintenance of high levels of costimulatory molecules in DCs1. Furthermore, poly-ICLC was recently directly compared to CpG in non-human primates (rhesus macaques) as adjuvant for a protein vaccine or immunogenic composition consisting of human papillomavirus (HPV)16 capsomers (Stahl-Hennig C, Eisenblatter M, Jasny E, et al. Synthetic double-stranded RNAs are adjuvants for the induction of T helper 1 and humoral immune responses to human papillomavirus in rhesus macaques. PLoS pathogens. Apr 2009;5(4)).

CpG immuno stimulatory oligonucleotides have also been reported to enhance the effects of adjuvants in a vaccine or immunogenic composition setting. Without being bound by theory, CpG oligonucleotides act by activating the innate (non- adaptive) immune system via Toll-like receptors (TLR), mainly TLR9. CpG triggered TLR9 activation enhances antigen-specific humoral and cellular responses to a wide variety of antigens, including peptide or protein antigens, live or killed viruses, dendritic cell vaccines, autologous cellular vaccines and polysaccharide conjugates in both prophylactic and therapeutic vaccines. More importantly, it enhances dendritic cell maturation and differentiation, resulting in enhanced activation of Th1 cells and strong cytotoxic T- lymphocyte (CTL) generation, even in the absence of CD4 T-cell help. The Th1 bias induced by TLR9 stimulation is maintained even in the presence of vaccine adjuvants such as alum or incomplete Freund's adjuvant (IFA) that normally promote a Th2 bias. CpG oligonucleotides show even greater adjuvant activity when formulated or co-administered with other adjuvants or in formulations such as microparticles, nano particles, lipid emulsions or similar formulations, which are especially necessary for inducing a strong response when the antigen is relatively weak. They also accelerate the immune response and enabled the antigen doses to be reduced by approximately two orders of magnitude, with comparable antibody responses to the full-dose vaccine without CpG in some experiments (Arthur M. Krieg, Nature Reviews, Drug Discovery, 5, Jun. 2006, 471-484). U.S. Pat. No. 6,406,705 B1 describes the combined use of CpG oligonucleotides, non-nucleic acid adjuvants and an antigen to induce an antigen- specific immune response. A commercially available CpG TLR9 antagonist is dSLIM (double Stem Loop Immunomodulator) by Mologen (Berlin, GERMANY), which is a preferred component of the pharmaceutical composition of the present invention. Other TLR binding molecules such as RNA binding TLR 7, TLR 8 and/or TLR 9 may also be used.

Other examples of useful adjuvants include, but are not limited to, chemically modified CpGs (e.g. CpR, Idera), Poly(I:C)(e.g. polyi:CI2U), non-CpG bacterial DNA or RNA as well as immunoactive small molecules and antibodies such as cyclophosphamide, sunitinib, bevacizumab, celebrex, NCX-4016, sildenafil, tadalafil, vardenafil, sorafinib, XL-999, CP-547632, pazopanib, ZD2171, AZD2171, ipilimumab, tremelimumab, and SC58175, which may act therapeutically and/or as an adjuvant. The amounts and concentrations of adjuvants and additives useful in the context of the present invention can readily be determined by the skilled artisan without undue experimentation. Additional adjuvants include colony- stimulating factors, such as Granulocyte Macrophage Colony Stimulating Factor (GM-CSF, sargramostim).

Poly-ICLC is a synthetically prepared double-stranded RNA consisting of polyI and polyC strands of average length of about 5000 nucleotides, which has been stabilized to thermal denaturation and hydrolysis by serum nucleases by the addition of polylysine and carboxymethylcellulose. The compound activates TLR3 and the RNA helicase-domain of MDA5, both members of the PAMP family, leading to DC and natural killer (NK) cell activation and production of a "natural mix" of type I interferons, cytokines, and chemokines. Furthermore, poly-ICLC exerts a more direct, broad host-targeted anti-infectious and possibly antitumor effect mediated by the two IFN-inducible nuclear enzyme systems, the 2'5'-OAS and the P1/eIF2a kinase, also known as the PKR (4-6), as well as RIG-I helicase and MDA5.

In rodents and non-human primates, poly-ICLC was shown to enhance T cell responses to viral antigens, cross-priming, and the induction of tumor-, virus-, and autoantigenspecific CD8+ T-cells. In a recent study in non-human primates, poly-ICLC was found to be essential for the generation of antibody responses and T-cell immunity to DC targeted or nontargeted HIV Gag p24 protein, emphasizing its effectiveness as a vaccine adjuvant.

In human subjects, transcriptional analysis of serial whole blood samples revealed similar gene expression profiles among the 8 healthy human volunteers receiving one single s.c. administration of poly-ICLC and differential expression of up to 212 genes between these 8 subjects versus 4 subjects receiving placebo. Remarkably, comparison of the poly-ICLC gene expression data to previous data from volunteers immunized with the highly effective yellow fever vaccine YF17D showed that a large number of transcriptional and signal transduction canonical pathways, including those of the innate immune system, were similarly upregulated at peak time points.

More recently, an immunologic analysis was reported on patients with ovarian, fallopian tube, and primary peritoneal cancer in second or third complete clinical remission who were treated on a phase 1 study of subcutaneous vaccination with synthetic overlapping long peptides (OLP) from the cancer testis antigen NY-ESO-1 alone or with Montanide-ISA-51, or with 1.4 mg poly-ICLC and Montanide. The generation of NY-ESO-1-specific CD4+ and CD8+ T-cell and antibody responses were markedly enhanced with the addition of poly-ICLC and Montanide compared to OLP alone or OLP and Montanide.

A vaccine or immunogenic composition according to the present invention may comprise more than one different adjuvant. Furthermore, the invention encompasses a therapeutic composition comprising any adjuvant substance including any of those herein discussed. It is also contemplated that the peptide or polypeptide, and the adjuvant can be administered separately in any appropriate sequence.

A carrier may be present independently of an adjuvant. The carrier may be covalently linked to the antigen. A carrier can also be added to the antigen by inserting DNA encoding the carrier in frame with DNA encoding the antigen. The function of a carrier can for example be to confer stability, to increase the biological activity, or to increase serum half-life. Extension of the half-life can help to reduce the number of applications and to lower doses, thus are beneficial for therapeutic but also economic reasons. Furthermore, a carrier may aid presenting peptides to T-cells. The carrier may be any suitable carrier known to the person skilled in the art, for example a protein or an antigen presenting cell. A carrier protein could be but is not limited to keyhole limpet hemocyanin, serum proteins such as transferrin, bovine serum albumin, human serum albumin, thyroglobulin or ovalbumin, immunoglobulins, or hormones, such as insulin or palmitic acid. For immunization of humans, the carrier may be a physiologically acceptable carrier acceptable to humans and safe. However, tetanus toxoid and/or diptheria toxoid are suitable carriers in one embodiment of the invention. Alternatively, the carrier may be dextrans for example sepharose.

Cytotoxic T-cells (CTLs) recognize an antigen in the form of a peptide bound to an MHC molecule rather than the intact foreign antigen itself. The MHC molecule itself is located at the cell surface of an antigen presenting cell. Thus, an activation of CTLs is only possible if a trimeric complex of peptide antigen, MHC molecule, and APC is present. Correspondingly, it may enhance the immune response if not only the peptide is used for activation of CTLs, but if additionally APCs with the respective MHC molecule are added. Therefore, in some embodiments the vaccine or immunogenic composition according to the present invention additionally contains at least one antigen presenting cell.

The antigen-presenting cell (or stimulator cell) typically has an MHC class I or II molecule on its surface, and in one embodiment is substantially incapable of itself loading the MHC class I or II molecule with the selected antigen. As is described in more detail herein, the MHC class I or II molecule may readily be loaded with the selected antigen in vitro.

CD8+ cell activity may be augmented through the use of CD4+ cells. The identification of CD4 T+ cell epitopes for tumor antigens has attracted interest because many immune based therapies against cancer may be more effective if both CD8+ and CD4+ T lymphocytes are used to target a patient's tumor. CD4+ cells are capable of enhancing CD8 T cell responses. Many studies in animal models have clearly demonstrated better results when both CD4+ and CD8+ T cells participate in anti-tumor responses (see e.g., Nishimura et al. (1999) Distinct role of antigen-specific T helper type 1 (TH1) and Th2 cells in tumor eradication in vivo. J Ex Med 190:617-27). Universal CD4+ T cell epitopes have been identified that are applicable to developing therapies against different types of cancer (see e.g., Kobayashi et al. (2008) Current Opinion in Immunology 20:221-27). For example, an HLA-DR restricted helper peptide from tetanus toxoid was used in melanoma vaccines to activate CD4+ T cells nonspecifically (see e.g., Slingluff et al. (2007) Immunologic and Clinical Outcomes of a Randomized Phase II Trial of Two Multipeptide Vaccines for Melanoma in the Adjuvant Setting, Clinical Cancer Research 13(21):6386-95). It is contemplated within the scope of the invention that such CD4+ cells may be applicable at three levels that vary in their tumor specificity: 1) a broad level in which universal CD4+ epitopes (e.g., tetanus toxoid) may be used to augment CD8+ cells; 2) an intermediate level in which native, tumor-associated CD4+ epitopes may be used to augment CD8+ cells; and 3) a patient specific level in which neoantigen CD4+ epitopes may be used to augment CD8+ cells in a patient specific manner.

CD8+ cell immunity may also be generated with neoantigen loaded dendritic cell (DC) vaccine. DCs are potent antigen-presenting cells that initiate T cell immunity and can be used as cancer vaccines when loaded with one or more peptides of interest, for example, by direct peptide injection. For example, patients that were newly diagnosed with metastatic melanoma were shown to be immunized against 3 HLA-A^{∗}0201-restricted gp100 melanoma antigen-derived peptides with autologous peptide pulsed CD40L/IFN-g-activated mature DCs via an IL-12p70-producing patient DC vaccine (see e.g., Carreno et al (2013) L-12p70-producing patient DC vaccine elicits Tc1-polarized immunity, Journal of Clinical Investigation, 123(8):3383-94 and Ali et al. (2009) In situ regulation of DC subsets and T cells mediates tumor regression in mice, Cancer Immunotherapy, 1(8): 1-10). It is contemplated within the scope of the invention that neoantigen loaded DCs may be prepared using the synthetic TLR 3 agonist Polyinosinic-Polycytidylic Acid-poly-L-lysine Carboxymethylcellulose (Poly-ICLC) to stimulate the DCs. Poly-ICLC is a potent individual maturation stimulus for human DCs as assessed by an upregulation of CD83 and CD86, induction of interleukin-12 (IL-12), tumor necrosis factor (TNF), interferon gamma-induced protein 10 (IP-10), interleukin 1 (IL-1), and type I interferons (IFN), and minimal interleukin 10 (IL-10) production. DCs may be differentiated from frozen peripheral blood mononuclear cells (PBMCs) obtained by leukapheresis, while PBMCs may be isolated by Ficoll gradient centrifugation and frozen in aliquots.

Illustratively, the following 7 day activation protocol may be used. Day 1-PBMCs are thawed and plated onto tissue culture flasks to select for monocytes which adhere to the plastic surface after 1-2 hr incubation at 37°C in the tissue culture incubator. After incubation, the lymphocytes are washed off and the adherent monocytes are cultured for 5 days in the presence of interleukin-4 (IL-4) and granulocyte macrophage-colony stimulating factor (GM-CSF) to differentiate to immature DCs. On Day 6, immature DCs are pulsed with the keyhole limpet hemocyanin (KLH) protein which serves as a control for the quality of the vaccine and may boost the immunogenicity of the vaccine. The DCs are stimulated to mature, loaded with peptide antigens, and incubated overnight. On Day 7, the cells are washed, and frozen in 1 ml aliquots containing 4-20 x 10(6) cells using a controlled-rate freezer. Lot release testing for the batches of DCs may be performed to meet minimum specifications before the DCs are injected into patients (see e.g., Sabado et al. (2013) Preparation of tumor antigen-loaded mature dendritic cells for immunotherapy, J. Vis Exp. Aug 1;(78). doi: 10.3791/50085).

A DC vaccine may be incorporated into a scaffold system to facilitate delivery to a patient. Therapeutic treatment of a patients neoplasia with a DC vaccine may utilize a biomaterial system that releases factors that recruit host dendritic cells into the device, differentiates the resident, immature DCs by locally presenting adjuvants (e.g., danger signals) while releasing antigen, and promotes the release of activated, antigen loaded DCs to the lymph nodes (or desired site of action) where the DCs may interact with T cells to generate a potent cytotoxic T lymphocyte response to the cancer neoantigens. Implantable biomaterials may be used to generate a potent cytotoxic T lymphocyte response against a neoplasia in a patient specific manner. The biomaterial-resident dendritic cells may then be activated by exposing them to danger signals mimicking infection, in concert with release of antigen from the biomaterial. The activated dendritic cells then migrate from the biomaterials to lymph nodes to induce a cytotoxic T effector response. This approach has previously been demonstrated to lead to regression of established melanoma in preclinical studies using a lysate prepared from tumor biopsies (see e.g., Ali et al. (2209) In situ regulation of DC subsets and T cells mediates tumor regression in mice, Cancer Immunotherapy 1(8):1-10; Ali et al. (2009) Infection-mimicking materials to program dendritic cells in situ. Nat Mater 8:151-8), and such a vaccine is currently being tested in a Phase I clinical trial recently initiated at the Dana-Farber Cancer Institute. This approach has also been shown to lead to regression of glioblastoma, as well as the induction of a potent memory response to prevent relapse, using the C6 rat glioma model.24 in the current proposal. The ability of such an implantable, biomatrix vaccine delivery scaffold to amplify and sustain tumor specific dendritic cell activation may lead to more robust anti-tumor immunosensitization than can be achieved by traditional subcutaneous or intra-nodal vaccine administrations.

Preferably, the antigen presenting cells are dendritic cells. Suitably, the dendritic cells are autologous dendritic cells that are pulsed with the neoantigenic peptide. The peptide may be any suitable peptide that gives rise to an appropriate T-cell response. T-cell therapy using autologous dendritic cells pulsed with peptides from a tumor associated antigen is disclosed in Murphy et al. (1996) The Prostate 29, 371-380 and Tjua et al. (1997) The Prostate 32, 272-278.

Thus, in one embodiment of the present invention the vaccine or immunogenic composition containing at least one antigen presenting cell is pulsed or loaded with one or more peptides of the present disclosure. Alternatively, peripheral blood mononuclear cells (PBMCs) isolated from a patient may be loaded with peptides ex vivo and injected back into the patient. As an alternative the antigen presenting cell comprises an expression construct encoding a peptide of the present disclosure. The polynucleotide may be any suitable polynucleotide and it is preferred that it is capable of transducing the dendritic cell, thus resulting in the presentation of a peptide and induction of immunity.

The inventive pharmaceutical composition may be compiled so that the selection, number and/or amount of peptides present in the composition is/are tissue, cancer, and/or patient-specific. For instance, the exact selection of peptides can be guided by expression patterns of the parent proteins in a given tissue to avoid side effects. The selection may be dependent on the specific type of cancer, the status of the disease, earlier treatment regimens, the immune status of the patient, and, of course, the HLA-haplotype of the patient. Furthermore, the vaccine or immunogenic composition according to the invention can contain individualized components, according to personal needs of the particular patient. Examples include varying the amounts of peptides according to the expression of the related neoantigen in the particular patient, unwanted side-effects due to personal allergies or other treatments, and adjustments for secondary treatments following a first round or scheme of treatment.

Pharmaceutical compositions comprising the peptide of the disclosure may be administered to an individual already suffering from cancer. In therapeutic applications, compositions are administered to a patient in an amount sufficient to elicit an effective CTL response to the tumor antigen and to cure or at least partially arrest symptoms and/or complications. An amount adequate to accomplish this is defined as "therapeutically effective dose." Amounts effective for this use can depend on, e.g., the peptide composition, the manner of administration, the stage and severity of the disease being treated, the weight and general state of health of the patient, and the judgment of the prescribing physician, but generally range for the initial immunization (that is for therapeutic or prophylactic administration) from about 1.0 µg to about 50,000 µg of peptide for a 70 kg patient, followed by boosting dosages or from about 1.0 µg to about 10,000 µg of peptide pursuant to a boosting regimen over weeks to months depending upon the patient's response and condition and possibly by measuring specific CTL activity in the patient's blood. It should be kept in mind that the peptide and compositions of the present disclosure may generally be employed in serious disease states, that is, life-threatening or potentially life threatening situations, especially when the cancer has metastasized. For therapeutic use, administration should begin as soon as possible after the detection or surgical removal of tumors. This is followed by boosting doses until at least symptoms are substantially abated and for a period thereafter.

The pharmaceutical compositions (e.g., vaccine compositions) for therapeutic treatment are intended for parenteral, topical, nasal, oral or local administration. Preferably, the pharmaceutical compositions are administered parenterally, e.g., intravenously, subcutaneously, intradermally, or intramuscularly. The compositions may be administered at the site of surgical excision to induce a local immune response to the tumor. The invention provides compositions for parenteral administration which comprise a solution of the peptides and vaccine or immunogenic compositions are dissolved or suspended in an acceptable carrier, preferably an aqueous carrier. A variety of aqueous carriers may be used, e.g., water, buffered water, 0.9% saline, 0.3% glycine, hyaluronic acid and the like. These compositions may be sterilized by conventional, well known sterilization techniques, or may be sterile filtered. The resulting aqueous solutions may be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

A liposome suspension containing a peptide may be administered intravenously, locally, topically, etc. in a dose which varies according to, inter alia, the manner of administration, the peptide being delivered, and the stage of the disease being treated. For targeting to the immune cells, a ligand, such as, e.g., antibodies or fragments thereof specific for cell surface determinants of the desired immune system cells, can be incorporated into the liposome.

For solid compositions, conventional or nanoparticle nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient, that is, one or more peptides of the disclosure, and more preferably at a concentration of 25%-75%.

For aerosol administration, the immunogenic peptides are preferably supplied in finely divided form along with a surfactant and propellant. Typical percentages of peptides are 0.01 %-20% by weight, preferably 1%-10%. The surfactant can, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A carrier can also be included as desired, as with, e.g., lecithin for intranasal delivery.

The peptides and polypeptides of the disclosure can be readily synthesized chemically utilizing reagents that are free of contaminating bacterial or animal substances (Merrifield RB: Solid phase peptide synthesis. I. The synthesis of a tetrapeptide. J. Am. Chem. Soc. 85:2149-54, 1963).

The peptides and polypeptides of the disclosure ] can also be expressed by a vector, e.g., a nucleic acid molecule as herein-discussed, e.g., RNA or a DNA plasmid, a viral vector such as a poxvirus, e.g., orthopox virus, avipox virus, or adenovirus, AAV or lentivirus. This approach involves the use of a vector to express nucleotide sequences that encode the peptide of the disclosure. Upon introduction into an acutely or chronically infected host or into a noninfected host, the vector expresses the immunogenic peptide, and thereby elicits a host CTL response.

For therapeutic or immunization purposes, nucleic acids encoding the peptide of the disclosure and optionally one or more of the peptides described herein can also be administered to the patient. A number of methods are conveniently used to deliver the nucleic acids to the patient. For instance, the nucleic acid can be delivered directly, as "naked DNA". This approach is described, for instance, in Wolff et al., Science 247: 1465-1468 (1990) as well as U.S. Patent Nos. 5,580,859 and 5,589,466. The nucleic acids can also be administered using ballistic delivery as described, for instance, in U.S. Patent No. 5,204,253. Particles comprised solely of DNA can be administered. Alternatively, DNA can be adhered to particles, such as gold particles. Generally, a plasmid for a vaccine or immunological composition can comprise DNA encoding an antigen (e.g., one or more neoantigens) operatively linked to regulatory sequences which control expression or expression and secretion of the antigen from a host cell, e.g., a mammalian cell; for instance, from upstream to downstream, DNA for a promoter, such as a mammalian virus promoter (e.g., a CMV promoter such as an hCMV or mCMV promoter, e.g., an early-intermediate promoter, or an SV40 promoter--see documents cited herein for useful promoters), DNA for a eukaryotic leader peptide for secretion (e.g., tissue plasminogen activator), DNA for the neoantigen(s), and DNA encoding a terminator (e.g., the 3' UTR transcriptional terminator from the gene encoding Bovine Growth Hormone or bGH polyA). A composition can contain more than one plasmid or vector, whereby each vector contains and expresses a different neoantigen. Mention is also made of Wasmoen U.S. Pat. No. 5,849,303, and Dale U.S. Pat. No. 5,811,104, whose text may be useful. DNA or DNA plasmid formulations can be formulated with or inside cationic lipids; and, as to cationic lipids, as well as adjuvants, mention is also made of Loosmore U.S. Patent Application 2003/0104008. Also, teachings in Audonnet U.S. Pat. Nos. 6,228,846 and 6,159,477 may be relied upon for DNA plasmid teachings that can be employed in constructing and using DNA plasmids that contain and express in vivo.

The nucleic acids can also be delivered complexed to cationic compounds, such as cationic lipids. Lipid-mediated gene delivery methods are described, for instance, in WO1996/18372; WO 1993/24640; Mannino & Gould-Fogerite , BioTechniques 6(7): 682-691 (1988); U.S. Patent No. 5,279,833; WO 1991/06309; and Feigner et al., Proc. Natl. Acad. Sci. USA 84: 7413-7414 (1987).

RNA encoding the peptide of interest (e.g., mRNA) can also be used for delivery (see, e.g., Kiken et al, 2011; Su et al , 2011; see also US 8278036; Halabi et al. J Clin Oncol (2003) 21:1232-1237; Petsch et al, Nature Biotechnology 2012 Dec 7;30(12):1210-6).

Information concerning poxviruses that may be used in the practice of the invention, such as *Chordopoxvirinae* subfamily poxviruses (poxviruses of vertebrates), for instance, orthopoxviruses and avipoxviruses, e.g., vaccinia virus (e.g., Wyeth Strain, WR Strain (e.g., ATCC^{®} VR-1354), Copenhagen Strain, NYVAC, NYVAC.1, NYVAC.2, MVA, MVA-BN), canarypox virus (e.g., Wheatley C93 Strain, ALVAC), fowlpox virus (e.g., FP9 Strain, Webster Strain, TROVAC), dovepox, pigeonpox, quailpox, and raccoon pox, *inter alia,* synthetic or nonnaturally occurring recombinants thereof, uses thereof, and methods for making and using such recombinants may be found in scientific and patent literature, such as:
➢ US Patents Nos. 4,603,112, 4,769,330, 5,110,587, 5,174,993, 5,364,773, 5,762,938, 5,494,807, 5,766,597, 7,767,449, 6,780,407, 6,537,594, 6,265,189, 6,214,353, 6,130,066, 6,004,777, 5,990,091, 5,942,235, 5,833,975, 5,766,597, 5,756,101, 7,045,313, 6,780,417, 8,470,598, 8,372,622, 8,268,329, 8,268,325, 8,236,560, 8,163,293, 7,964,398, 7,964,396, 7,964,395, 7,939,086, 7,923,017, 7,897,156, 7,892,533, 7,628,980, 7,459,270, 7,445,924, 7,384,644, 7,335,364, 7,189,536, 7,097,842, 6,913,752, 6,761,893, 6,682,743, 5,770,212, 5,766,882, and 5,989,562, and
> Panicali, D. Proc. Natl. Acad. Sci. 1982; 79; 4927-493, Panicali D. Proc. Natl. Acad. Sci. 1983; 80(17): 5364-8, Mackett, M. Proc. Natl. Acad. Sci. 1982; 79: 7415-7419, Smith GL. Proc. Natl. Acad. Sci. 1983; 80(23): 7155-9, Smith GL. Nature 1983; 302: 490-5, Sullivan VJ. Gen. Vir. 1987; 68: 2587-98, Perkus M Journal of Leukocyte Biology 1995; 58:1-13, Yilma TD. Vaccine 1989; 7: 484-485, Brochier B. Nature 1991; 354: 520-22, Wiktor, TJ. Proc. Natl Acd. Sci. 1984; 81: 7194-8, Rupprecht, CE. Proc. Natl Acd. Sci. 1986; 83: 7947-50, Poulet, H Vaccine 2007; 25(Jul): 5606-12, Weyer J. Vaccine 2009; 27(Nov): 7198-201, Buller, RM Nature 1985; 317(6040): 813-5, Buller RM. J. Virol. 1988; 62(3):866-74, Flexner, C. Nature 1987; 330(6145): 259-62, Shida, H. J. Virol. 1988; 62(12): 4474-80, Kotwal, GJ. J. Virol. 1989; 63(2): 600-6, Child, SJ. Virology 1990; 174(2): 625-9, Mayr A. Zentralb1 Bakteriol 1978; 167(5,6): 375-9, Antoine G. Virology. 1998; 244(2): 365-96, Wyatt, LS. Virology 1998; 251(2): 334-42, Sancho, MC. J. Virol. 2002; 76(16); 8313-34, Gallego-Gomez, JC. J. Virol. 2003; 77(19); 10606-22), Goebel SJ. Virology 1990; (a,b) 179: 247-66, Tartaglia, J. Virol. 1992; 188(1): 217-32, Najera JL. J. Virol. 2006; 80(12): 6033-47, Najera, JL. J. Virol. 2006; 80: 6033-6047, Gomez, CE. J. Gen. Virol. 2007; 88: 2473-78, Mooij, P. Jour. Of Virol. 2008; 82: 2975-2988, Gomez, CE. Curr. Gene Ther. 2011; 11: 189-217, Cox,W. Virology 1993; 195: 845-50, Perkus, M. Jour. Of Leukocyte Biology 1995; 58: 1-13, Blanchard TJ. J Gen Virology 1998; 79(5): 1159-67, Amara R. Science 2001; 292: 69-74, Hel, Z., J. Immunol. 2001; 167: 7180-9, Gherardi MM. J. Virol. 2003; 77: 7048-57, Didierlaurent, A. Vaccine 2004; 22: 3395-3403, Bissht H. Proc. Nat. Aca. Sci. 2004; 101: 6641-46, McCurdy LH. Clin. Inf. Dis 2004; 38: 1749-53, Earl PL. Nature 2004; 428: 182-85, Chen Z. J. Virol. 2005; 79: 2678-2688, Najera JL. J. Virol. 2006; 80(12): 6033-47, Nam JH. Acta. Virol. 2007; 51: 125-30, Antonis AF. Vaccine 2007; 25: 4818-4827,B Weyer J. Vaccine 2007; 25: 4213-22, Ferrier-Rembert A. Vaccine 2008; 26(14): 1794-804, Corbett M. Proc. Natl. Acad. Sci. 2008; 105(6): 2046-51, Kaufman HL., J. Clin. Oncol. 2004; 22: 2122-32, Amato, RJ. Clin. Cancer Res. 2008; 14(22): 7504-10, Dreicer R. Invest New Drugs 2009; 27(4): 379-86, Kantoff PW.J. Clin. Oncol. 2010, 28, 1099-1105, Amato RJ. J. Clin. Can. Res. 2010; 16(22): 5539-47, Kim, DW. Hum. Vaccine. 2010; 6: 784-791, Oudard, S. Cancer Immunol. Immunother. 2011; 60: 261-71, Wyatt, LS. Aids Res. Hum. Retroviruses. 2004; 20: 645-53, Gomez, CE. Virus Research 2004; 105: 11-22, Webster, DP. Proc. Natl. Acad. Sci. 2005; 102: 4836-4, Huang, X. Vaccine 2007; 25: 8874-84, Gomez, CE. Vaccine 2007a; 25: 2863-85, Esteban M. Hum. Vaccine 2009; 5: 867-871, Gomez, CE. Curr. Gene therapy 2008; 8(2): 97-120, Whelan, KT. Plos one 2009; 4(6): 5934, Scriba, TJ. Eur. Jour. Immuno. 2010; 40(1): 279-90, Corbett, M. Proc. Natl. Acad. Sci. 2008; 105: 2046-2051, Midgley, CM. J. Gen. Virol. 2008; 89: 2992-97, Von Krempelhuber, A. Vaccine 2010; 28: 1209-16, Perreau, M. J. Of Virol. 2011; Oct: 9854-62, Pantaleo, G. Curr Opin HIV-AIDS. 2010; 5: 391-396,
each of which is incorporated herein by reference.

As to adenovirus vectors useful in the practice of the invention, mention is made of US Patent No. 6,955,808. The adenovirus vector used can be selected from the group consisting of the Ad5, Ad35, Ad11, C6, and C7 vectors. The sequence of the Adenovirus 5 ("Ad5") genome has been published. (Chroboczek, J., Bieber, F., and Jacrot, B. (1992) The Sequence of the Genome of Adenovirus Type 5 and Its Comparison with the Genome of Adenovirus Type 2, Virology 186, 280-285 ). Ad35 vectors are described in U.S. Pat. Nos. 6,974,695, 6,913,922, and 6,869,794. Ad11 vectors are described in U.S. Pat. No. 6,913,922. C6 adenovirus vectors are described in U.S. Pat. Nos. 6,780,407; 6,537,594; 6,309,647; 6,265,189; 6,156,567; 6,090,393; 5,942,235 and 5,833,975. C7 vectors are described in U.S. Pat. No. 6,277,558. Adenovirus vectors that are E1-defective or deleted, E3-defective or deleted, and/or E4-defective or deleted may also be used. Certain adenoviruses having mutations in the E1 region have improved safety margin because E1-defective adenovirus mutants are replication-defective in non-permissive cells, or, at the very least, are highly attenuated. Adenoviruses having mutations in the E3 region may have enhanced the immunogenicity by disrupting the mechanism whereby adenovirus down-regulates MHC class I molecules. Adenoviruses having E4 mutations may have reduced immunogenicity of the adenovirus vector because of suppression of late gene expression. Such vectors may be particularly useful when repeated re-vaccination utilizing the same vector is desired. Adenovirus vectors that are deleted or mutated in E1, E3, E4, E1 and E3, and E1 and E4 can be used in accordance with the present invention. Furthermore, "gutless" adenovirus vectors, in which all viral genes are deleted, can also be used in accordance with the present invention. Such vectors require a helper virus for their replication and require a special human 293 cell line expressing both E1a and Cre, a condition that does not exist in natural environment. Such "gutless" vectors are non-immunogenic and thus the vectors may be inoculated multiple times for re-vaccination. The "gutless" adenovirus vectors can be used for insertion of heterologous inserts/genes such as the transgenes of the present disclosure, and can even be used for co-delivery of a large number of heterologous inserts/genes.

As to lentivirus vector systems useful in the practice of the invention, mention is made of US Patents Nos. 6428953, 6165782, 6013516, 5994136, 6312682, and 7,198,784, and documents cited therein.

With regard to AAV vectors useful in the practice of the invention, mention is made of US Patent Nos. 5658785, 7115391, 7172893, 6953690, 6936466, 6924128, 6893865, 6793926, 6537540, 6475769 and 6258595, and documents cited therein.

Another vector is BCG (Bacille Calmette Guerin). BCG vectors are described in Stover et al. (Nature 351:456-460 (1991)). A wide variety of other vectors useful for therapeutic administration or immunization of the peptides of the disclosure, e.g., Salmonella typhi vectors and the like, is apparent to those skilled in the art from the description herein.

Vectors can be administered so as to have in vivo expression and response akin to doses and/or responses elicited by antigen administration

A preferred means of administering nucleic acids encoding the peptide of the disclosure uses minigene constructs encoding multiple epitopes. To create a DNA sequence encoding the selected CTL epitopes (minigene) for expression in human cells, the amino acid sequences of the epitopes are reverse translated. A human codon usage table is used to guide the codon choice for each amino acid. These epitope-encoding DNA sequences are directly adjoined, creating a continuous polypeptide sequence. To optimize expression and/or immunogenicity, additional elements can be incorporated into the minigene design. Examples of amino acid sequence that could be reverse translated and included in the minigene sequence include: helper T lymphocyte, epitopes, a leader (signal) sequence, and an endoplasmic reticulum retention signal. In addition, MHC presentation of CTL epitopes may be improved by including synthetic (e.g. poly-alanine) or naturally- occurring flanking sequences adjacent to the CTL epitopes.

The minigene sequence is converted to DNA by assembling oligonucleotides that encode the plus and minus strands of the minigene. Overlapping oligonucleotides (30-100 bases long) are synthesized, phosphorylated, purified and annealed under appropriate conditions using well known techniques. The ends of the oligonucleotides are joined using T4 DNA ligase. This synthetic minigene, encoding the CTL epitope polypeptide, can then cloned into a desired expression vector.

Standard regulatory sequences well known to those of skill in the art are included in the vector to ensure expression in the target cells. Several vector elements are required: a promoter with a down-stream cloning site for minigene insertion; a polyadenylation signal for efficient transcription termination; an E. coli origin of replication; and an E. coli selectable marker (e.g. ampicillin or kanamycin resistance). Numerous promoters can be used for this purpose, e.g., the human cytomegalovirus (hCMV) promoter. See, U.S. Patent Nos. 5,580,859 and 5,589,466 for other suitable promoter sequences.

Additional vector modifications may be desired to optimize minigene expression and immunogenicity. In some cases, introns are required for efficient gene expression, and one or more synthetic or naturally-occurring introns could be incorporated into the transcribed region of the minigene. The inclusion of mRNA stabilization sequences can also be considered for increasing minigene expression. It has recently been proposed that immuno stimulatory sequences (ISSs or CpGs) play a role in the immunogenicity of DNA' vaccines. These sequences could be included in the vector, outside the minigene coding sequence, if found to enhance immunogenicity.

In some embodiments, a bicistronic expression vector, to allow production of the minigene-encoded epitopes and a second protein included to enhance or decrease immunogenicity can be used. Examples of proteins or polypeptides that could beneficially enhance the immune response if co-expressed include cytokines (e.g., IL2, IL12, GM-CSF), cytokine-inducing molecules (e.g. LeIF) or costimulatory molecules. Helper (HTL) epitopes could be joined to intracellular targeting signals and expressed separately from the CTL epitopes. This would allow direction of the HTL epitopes to a cell compartment different than the CTL epitopes. If required, this could facilitate more efficient entry of HTL epitopes into the MHC class II pathway, thereby improving CTL induction. In contrast to CTL induction, specifically decreasing the immune response by co-expression of immunosuppressive molecules (e.g. TGF-β) may be beneficial in certain diseases.

Once an expression vector is selected, the minigene is cloned into the polylinker region downstream of the promoter. This plasmid is transformed into an appropriate E. coli strain, and DNA is prepared using standard techniques. The orientation and DNA sequence of the minigene, as well as all other elements included in the vector, are confirmed using restriction mapping and DNA sequence analysis. Bacterial cells harboring the correct plasmid can be stored as a master cell bank and a working cell bank.

Purified plasmid DNA can be prepared for injection using a variety of formulations. The simplest of these is reconstitution of lyophilized DNA in sterile phosphate-buffer saline (PBS). A variety of methods have been described, and new techniques may become available. As noted herein, nucleic acids are conveniently formulated with cationic lipids. In addition, glycolipids, fusogenic liposomes, peptides and compounds referred to collectively as protective, interactive, non-condensing (PINC) could also be complexed to purified plasmid DNA to influence variables such as stability, intramuscular dispersion, or trafficking to specific organs or cell types.

Target cell sensitization can be used as a functional assay for expression and MHC class I presentation of minigene-encoded CTL epitopes. The plasmid DNA is introduced into a mammalian cell line that is suitable as a target for standard CTL chromium release assays. The transfection method used is dependent on the final formulation. Electroporation can be used for "naked" DNA, whereas cationic lipids allow direct in vitro transfection. A plasmid expressing green fluorescent protein (GFP) can be co-transfected to allow enrichment of transfected cells using fluorescence activated cell sorting (FACS). These cells are then chromium-51 labeled and used as target cells for epitope- specific CTL lines. Cytolysis, detected by 51 Cr release, indicates production of MHC presentation of mini gene-encoded CTL epitopes.

In vivo immunogenicity is a second approach for functional testing of minigene DNA formulations. Transgenic mice expressing appropriate human MHC molecules are immunized with the DNA product. The dose and route of administration are formulation dependent (e.g. IM for DNA in PBS, IP for lipid-complexed DNA). Twenty-one days after immunization, splenocytes are harvested and restimulated for 1 week in the presence of peptides encoding each epitope being tested. These effector cells (CTLs) are assayed for cytolysis of peptide-loaded, chromium-51 labeled target cells using standard techniques. Lysis of target cells sensitized by MHC loading of peptides corresponding to minigene-encoded epitopes demonstrates DNA vaccine function for in vivo induction of CTLs.

Peptides may be used to elicit CTL ex vivo, as well. The resulting CTL, can be used to treat chronic tumors in patients in need thereof that do not respond to other conventional forms of therapy, or does not respond to a peptide vaccine approach of therapy. Ex vivo CTL responses to a particular tumor antigen are induced by incubating in tissue culture the patient's CTL precursor cells (CTLp) together with a source of antigen-presenting cells (APC) and the appropriate peptide. After an appropriate incubation time (typically 1-4 weeks), in which the CTLp are activated and mature and expand into effector CTL, the cells are infused back into the patient, where they destroy their specific target cell (i.e., a tumor cell). In order to optimize the in vitro conditions for the generation of specific cytotoxic T cells, the culture of stimulator cells are maintained in an appropriate serum-free medium.

Prior to incubation of the stimulator cells with the cells to be activated, e.g., precursor CD8+ cells, an amount of antigenic peptide is added to the stimulator cell culture, of sufficient quantity to become loaded onto the human Class I molecules to be expressed on the surface of the stimulator cells. In the present invention, a sufficient amount of peptide is an amount that allows about 200, and preferably 200 or more, human Class I MHC molecules loaded with peptide to be expressed on the surface of each stimulator cell. Preferably, the stimulator cells are incubated with >2µg/ml peptide. For example, the stimulator cells are incubates with > 3, 4, 5, 10, 15, or more µg/ml peptide.

Resting or precursor CD8+ cells are then incubated in culture with the appropriate stimulator cells for a time period sufficient to activate the CD8+ cells. Preferably, the CD8+ cells are activated in an antigen- specific manner. The ratio of resting or precursor CD8+ (effector) cells to stimulator cells may vary from individual to individual and may further depend upon variables such as the amenability of an individual's lymphocytes to culturing conditions and the nature and severity of the disease condition or other condition for which the withindescribed treatment modality is used. Preferably, however, the lymphocyte: stimulator cell ratio is in the range of about 30: 1 to 300: 1. The effector/stimulator culture may be maintained for as long a time as is necessary to stimulate a therapeutically useable or effective number of CD8+ cells.

The induction of CTL in vitro requires the specific recognition of peptides that are bound to allele specific MHC class I molecules on APC. The number of specific MHC/peptide complexes per APC is crucial for the stimulation of CTL, particularly in primary immune responses. While small amounts of peptide/MHC complexes per cell are sufficient to render a cell susceptible to lysis by CTL, or to stimulate a secondary CTL response, the successful activation of a CTL precursor (pCTL) during primary response requires a significantly higher number of MHC/peptide complexes. Peptide loading of empty major histocompatability complex molecules on cells allows the induction of primary cytotoxic T lymphocyte responses.

Since mutant cell lines do not exist for every human MHC allele, it is advantageous to use a technique to remove endogenous MHC- associated peptides from the surface of APC, followed by loading the resulting empty MHC molecules with the immunogenic peptides of interest. The use of non-transformed (non-tumorigenic), noninfected cells, and preferably, autologous cells of patients as APC is desirable for the design of CTL induction protocols directed towards development of ex vivo CTL therapies. This application discloses methods for stripping the endogenous MHC-associated peptides from the surface of APC followed by the loading of desired peptides.

A stable MHC class I molecule is a trimeric complex formed of the following elements: 1) a peptide usually of 8 - 10 residues, 2) a transmembrane heavy polymorphic protein chain which bears the peptide-binding site in its al and a2 domains, and 3) a non-covalently associated non-polymorphic light chain, p2microglobuiin. Removing the bound peptides and/or dissociating the p2microglobulin from the complex renders the MHC class I molecules nonfunctional and unstable, resulting in rapid degradation. All MHC class I molecules isolated from PBMCs have endogenous peptides bound to them. Therefore, the first step is to remove all endogenous peptides bound to MHC class I molecules on the APC without causing their degradation before exogenous peptides can be added to them.

Two possible ways to free up MHC class I molecules of bound peptides include lowering the culture temperature from 37°C to 26°C overnight to destablize p2microglobulin and stripping the endogenous peptides from the cell using a mild acid treatment. The methods release previously bound peptides into the extracellular environment allowing new exogenous peptides to bind to the empty class I molecules. The cold-temperature incubation method enables exogenous peptides to bind efficiently to the MHC complex, but requires an overnight incubation at 26°C which may slow the cell's metabolic rate. It is also likely that cells not actively synthesizing MHC molecules (e.g., resting PBMC) would not produce high amounts of empty surface MHC molecules by the cold temperature procedure.

Harsh acid stripping involves extraction of the peptides with trifluoroacetic acid, pH 2, or acid denaturation of the immunoaffinity purified class I-peptide complexes. These methods are not feasible for CTL induction, since it is important to remove the endogenous peptides while preserving APC viability and an optimal metabolic state which is critical for antigen presentation. Mild acid solutions of pH 3 such as glycine or citrate -phosphate buffers have been used to identify endogenous peptides and to identify tumor associated T cell epitopes. The treatment is especially effective, in that only the MHC class I molecules are destabilized (and associated peptides released), while other surface antigens remain intact, including MHC class II molecules. Most importantly, treatment of cells with the mild acid solutions do not affect the cell's viability or metabolic state. The mild acid treatment is rapid since the stripping of the endogenous peptides occurs in two minutes at 4°C and the APC is ready to perform its function after the appropriate peptides are loaded. The technique is utilized herein to make peptide-specific APCs for the generation of primary antigen- specific CTL. The resulting APC are efficient in inducing peptide- specific CD8+ CTL.

Activated CD8+ cells may be effectively separated from the stimulator cells using one of a variety of known methods. For example, monoclonal antibodies specific for the stimulator cells, for the peptides loaded onto the stimulator cells, or for the CD8+ cells (or a segment thereof) may be utilized to bind their appropriate complementary ligand. Antibodytagged molecules may then be extracted from the stimulator-effector cell admixture via appropriate means, e.g., via well-known immunoprecipitation or immunoassay methods.

Effective, cytotoxic amounts of the activated CD8+ cells can vary between in vitro and in vivo uses, as well as with the amount and type of cells that are the ultimate target of these killer cells. The amount can also vary depending on the condition of the patient and should be determined via consideration of all appropriate factors by the practitioner. Preferably, however, about 1 X 10⁶ to about 1 X 10¹², more preferably about 1 X 10⁸ to about 1 X 10¹¹, and even more preferably, about 1 X 10⁹ to about 1 X 10¹⁰ activated CD8+ cells are utilized for adult humans, compared to about 5 X 10⁶ - 5 X 10⁷ cells used in mice.

Preferably, as discussed herein, the activated CD8+ cells are harvested from the cell culture prior to administration of the CD8+ cells to the individual being treated. It is important to note, however, that unlike other present and proposed treatment modalities, the present method uses a cell culture system that is not tumorigenic. Therefore, if complete separation of stimulator cells and activated CD8+ cells are not achieved, there is no inherent danger known to be associated with the administration of a small number of stimulator cells, whereas administration of mammalian tumor-promoting cells may be extremely hazardous.

Methods of re-introducing cellular components are known in the art and include procedures such as those exemplified in U.S. Patent No. 4,844,893 to Honsik, et al. and U.S. Patent No. 4,690,915 to Rosenberg. For example, administration of activated CD8+ cells via intravenous infusion is appropriate.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", second edition (Sambrook, 1989); "Oligonucleotide Synthesis" (Gait, 1984); "Animal Cell Culture" (Freshney, 1987); "Methods in Enzymology" "Handbook of Experimental Immunology" (Wei, 1996); "Gene Transfer Vectors for Mammalian Cells" (Miller and Calos, 1987); "Current Protocols in Molecular Biology" (Ausubel, 1987); "PCR: The Polymerase Chain Reaction", (Mullis, 1994); "Current Protocols in Immunology" (Coligan, 1991). These techniques are applicable to the production of the polynucleotides and polypeptides of the disclosure, and, as such, may be considered in making and practicing the invention. Particularly useful techniques for particular embodiments are discussed in the sections that follow.

### Therapeutic Methods

The present disclosure provides methods of inducing a neoplasia/tumor specific immune response in a subject, vaccinating against a neoplasia/tumor, treating and or alleviating a symptom of cancer in a subject by administering the subject a neoplasia vaccine or a neoantigenic peptide or composition of the disclosure.

According to the invention, the herein-described neoplasia vaccine or immunogenic composition may be used for a patient that has been diagnosed as having cancer, or at risk of developing cancer. In one embodiment, the patient may have a solid tumor such as breast, ovarian, prostate, lung, kidney, gastric, colon, testicular, head and neck, pancreas, brain, melanoma, and other tumors of tissue organs and hematological tumors, such as lymphomas and leukemias, including acute myelogenous leukemia, chronic myelogenous leukemia, chronic lymphocytic leukemia, T cell lymphocytic leukemia, and B cell lymphomas.

The peptide or composition of the disclosure is administered in an amount sufficient to induce a CTL response.

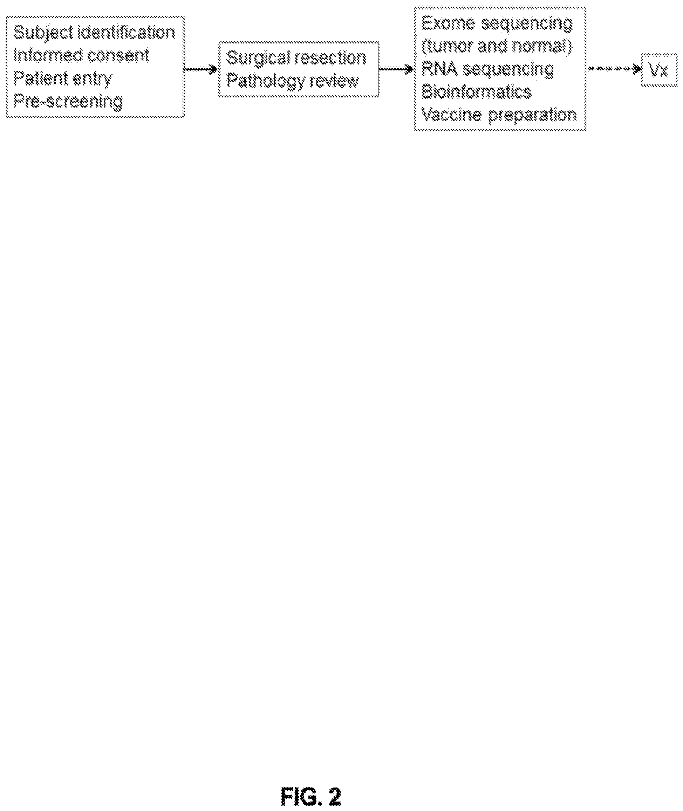

The herein-described compositions and methods may be used on patients in need thereof with any cancer according to the general flow process shown in FIG. 2. Patients in need thereof may receive a series of priming vaccinations with a mixture of personalized tumor-specific peptides. Additionally, over a 4 week period the priming may be followed by two boosts during a maintenance phase. All vaccinations are subcutaneously delivered. The vaccine or immunogenic composition is evaluated for safety, tolerability, immune response and clinical effect in patients and for feasibility of producing vaccine or immunogenic composition and successfully initiating vaccination within an appropriate time frame. The first cohort can consist of 5 patients, and after safety is adequately demonstrated, an additional cohort of 10 patients may be enrolled. Peripheral blood is extensively monitored for peptide-specific T-cell responses and patients are followed for up to two years to assess disease recurrence.

### Vaccine or Immunogenic Composition Kits and Co-Packaging

In an instance, the disclosure provides kits containing any one or more of the elements discussed herein to allow administration of the immunogenic composition or vaccine. Elements may be provided individually or in combinations, and may be provided in any suitable container, such as a vial, a bottle, or a tube. In some embodiments, the kit includes instructions in one or more languages, for example in more than one language. In some instances , a kit comprises one or more reagents for use in a process utilizing one or more of the elements described herein. Reagents may be provided in any suitable container. For example, a kit may provide one or more delivery or storage buffers. Reagents may be provided in a form that is usable in a particular process, or in a form that requires addition of one or more other components before use (e.g. in concentrate or lyophilized form). A buffer can be any buffer, including but not limited to a sodium carbonate buffer, a sodium bicarbonate buffer, a borate buffer, a Tris buffer, a MOPS buffer, a HEPES buffer, and combinations thereof. In some embodiments, the buffer is alkaline. In some embodiments, the buffer has a pH from about 7 to about 10. In some instances, the kit comprises one or more of the vectors, proteins and/or one or more of the polynucleotides described herein. The kit may advantageously allow the provision of all elements of the systems of the disclosure. Kits can involve vector(s) and/or particle(s) and/or nanoparticle(s) containing or encoding RNA(s) for 1-50 or more neoantigen mutations to be administered to an animal, mammal, primate, rodent, etc., with such a kit including instructions for administering to such a eukaryote, as well as instructions for use with any of the methods of the present invention.

In one instance the kit contains at least one vial with an immunogenic composition or vaccine. In one instance kits may comprise ready to use components that are mixed and ready to use. The ready to use immunogenic or vaccine composition may comprise separate vials containing different pools of immunogenic compositions. The immunogenic compositions may comprise one vial containing a viral vector or DNA plasmid and the other vial may comprise immunogenic protein. In another instance a kit may contain an immunogenic composition or vaccine in a ready to be reconstituted form. The immunogenic or vaccine composition may be freeze dried or lyophilized. The kit may comprise a separate vial with a reconstitution buffer that can be added to the lyophilized composition so that it is ready to administer. The buffer may advantageously comprise an adjuvant or emulsion according to the present invention. In another embodiment the kit may comprise single vials containing a dose of immunogenic composition. In another aspect multiple vials are included so that one vial is administered according to a treatment timeline. In a further embodiment the vials are labeled for their proper administration to a patient in need thereof. The immunogen may be in a lyophilized form, a dried form or in aqueous solution as described herein. The immunogen may be a live attenuated virus, protein, or nucleic acid as described herein.

In another instance the kit may comprise separate vials for an immunogenic composition for use in priming an immune response and another immunogenic composition to be used for boosting. In one instance the priming immunogenic composition could be DNA or a viral vector and the boosting immunogenic composition may be protein. Either composition may be lyophilized or ready for administering.

### Examples

### Example 1

### Cancer Vaccine Testing Protocol

The herein-described compositions and methods may be tested on 15 patients with high-risk melanoma (fully resected stages IIIB, IIIC and IVM1a,b) according to the general flow process shown in FIG. 2. Patients may receive a series of priming vaccinations with a mixture of personalized tumor-specific peptides and poly-ICLC over a 4 week period followed by two boosts during a maintenance phase. All vaccinations are subcutaneously delivered. The vaccine or immunogenic composition is evaluated for safety, tolerability, immune response and clinical effect in patients and for feasibility of producing vaccine or immunogenic composition and successfully initiating vaccination within an appropriate time frame. The first cohort can consist of 5 patients, and after safety is adequately demonstrated, an additional cohort of 10 patients may be enrolled. Peripheral blood is extensively monitored for peptide-specific T-cell responses and patients are followed for up to two years to assess disease recurrence.

As described herein, there is a large body of evidence in both animals and humans that mutated epitopes are effective in inducing an immune response and that cases of spontaneous tumor regression or long term survival correlate with CD8+ T-cell responses to mutated epitopes (Buckwalter and Srivastava PK. "It is the antigen(s), stupid" and other lessons from over a decade of vaccitherapy of human cancer. Seminars in immunology 20:296-300 (2008); Karanikas et al, High frequency of cytolytic T lymphocytes directed against a tumor-specific mutated antigen detectable with HLA tetramers in the blood of a lung carcinoma patient with long survival. Cancer Res. 61:3718-3724 (2001); Lennerz et al, The response of autologous T cells to a human melanoma is dominated by mutated neoantigens. Proc Natl Acad Sci U S A.102:16013 (2005)) and that "immunoediting" can be tracked to alterations in expression of dominant mutated antigens in mice and man (Matsushita et al, Cancer exome analysis reveals a T-cell-dependent mechanism of cancer immunoediting Nature 482:400 (2012); DuPage et al, Expression of tumor-specific antigens underlies cancer immunoediting Nature 482:405 (2012); and Sampson et al, Immunologic escape after prolonged progression-free survival with epidermal growth factor receptor variant III peptide vaccination in patients with newly diagnosed glioblastoma J Clin Oncol. 28:4722-4729 (2010)).

Next-generation sequencing can now rapidly reveal the presence of discrete mutations such as coding mutations in individual tumors, most commonly single amino acid changes (e.g., missense mutations) and less frequently novel stretches of amino acids generated by frame-shift insertions/deletions/gene fusions, read-through mutations in stop codons, and translation of improperly spliced introns (e.g., neoORFs). NeoORFs are particularly valuable as immunogens because the entirety of their sequence is completely novel to the immune system and so are analogous to a viral or bacterial foreign antigen. Thus, neoORFs: (1) are highly specific to the tumor (i.e. there is no expression in any normal cells); (2) can bypass central tolerance, thereby increasing the precursor frequency of neoantigen-specific CTLs. For example, the power of utilizing analogous foreign sequences in a therapeutic anti-cancer vaccine was recently demonstrated with peptides derived from human papilloma virus (HPV). ~50% of the 19 patients with pre-neoplastic, viral-induced disease who received 3 - 4 vaccinations of a mix of HPV peptides derived from the viral oncogenes E6 and E7 maintained a complete response for ≥24 months (Kenter et a, Vaccination against HPV-16 Oncoproteins for Vulvar Intraepithelial Neoplasia NEJM 361:1838 (2009)).

Sequencing technology has revealed that each tumor contains multiple, patient-specific mutations that alter the protein coding content of a gene. Such mutations create altered proteins, ranging from single amino acid changes (caused by missense mutations) to addition of long regions of novel amino acid sequence due to frame shifts, read-through of termination codons or translation of intron regions (novel open reading frame mutations; neoORFs). These mutated proteins are valuable targets for the host's immune response to the tumor as, unlike native proteins, they are not subject to the immune-dampening effects of self-tolerance. Therefore, mutated proteins are more likely to be immunogenic and are also more specific for the tumor cells compared to normal cells of the patient.

Utilizing recently improved algorithms for predicting which missense mutations create strong binding peptides to the patient's cognate MHC molecules, a set of peptides representative of optimal mutated epitopes (both neoORF and missense) for each patient is identified and prioritized and up to 20 or more peptides are prepared for immunization (Zhang et al, Machine learning competition in immunology - Prediction of HLA class I binding peptides J Immunol Methods 374:1 (2011); Lundegaard et al Prediction of epitopes using neural network based methods J Immunol Methods 374:26 (2011)). Peptides ~20-35 amino acids in length is synthesized because such "long" peptides undergo efficient internalization, processing and crosspresentation in professional antigen-presenting cells such as dendritic cells, and have been shown to induce CTLs in humans (Melief and van der Burg, Immunotherapy of established (pre) malignant disease by synthetic long peptide vaccines Nature Rev Cancer 8:351 (2008)).

In addition to a powerful and specific immunogen, an effective immune response advantageously includes a strong adjuvant to activate the immune system (Speiser and Romero, Molecularly defined vaccines for cancer immunotherapy, and protective T cell immunity Seminars in Immunol 22:144 (2010)). For example, Toll-like receptors (TLRs) have emerged as powerful sensors of microbial and viral pathogen "danger signals", effectively inducing the innate immune system, and in turn, the adaptive immune system (Bhardwaj and Gnjatic, TLR AGONISTS: Are They Good Adjuvants? Cancer J. 16:382-391 (2010)). Among the TLR agonists, poly-ICLC (a synthetic double-stranded RNA mimic) is one of the most potent activators of myeloid-derived dendritic cells. In a human volunteer study, poly-ICLC has been shown to be safe and to induce a gene expression profile in peripheral blood cells comparable to that induced by one of the most potent live attenuated viral vaccines, the yellow fever vaccine YF-17D (Caskey et al, Synthetic double-stranded RNA induces innate immune responses similar to a live viral vaccine in humans J Exp Med 208:2357 (2011)). Hiltonol^{®}, a GMP preparation of poly-ICLC prepared by Oncovir, Inc, is utilized as the adjuvant.

### Example 2

### Target Patient Population

Patients with stage IIIB, IIIC and IVM1a,b, melanoma have a significant risk of disease recurrence and death, even with complete surgical resection of disease (Balch et al, Final Version of 2009 AJCC Melanoma Staging and Classification J Clin Oncol 27:6199 - 6206 (2009)). An available systemic adjuvant therapy for this patient population is interferon-a (IFNα) which provides a measurable but marginal benefit and is associated with significant, frequently dose-limiting toxicity (Kirkwood et al, Interferon alfa-2b Adjuvant Therapy of High-Risk Resected Cutaneous Melanoma: The Eastern Cooperative Oncology Group Trial EST 1684 J Clin Oncol 14:7-17 (1996); Kirkwood et al, High- and Low-dose Interferon Alpha-2b in High-Risk Melanoma: First Analysis of Intergroup Trial E1690/S9111/C9190 J Clin Oncol 18:2444 - 2458 (2000)). These patients are not immuno-compromised by previous cancer-directed therapy or by active cancer and thus represent an excellent patient population in which to assess the safety and immunological impact of the vaccine. Finally, current standard of care for these patients does not mandate any treatment following surgery, thus allowing for the 8 - 10 week window for vaccine preparation.

The target population is cutaneous melanoma patients with clinically detectable, histologically confirmed nodal (local or distant) or in transit metastasis, who have been fully resected and are free of disease (most of stage IIIB (because of the need to have adequate tumor tissue for sequencing and cell line development, patients with ulcerated primary tumor but micrometastatic lymph nodes (T1-4b, N1a or N2a) is excluded.), all of stage IIIC, and stage IVM1a, b). These may be patients at first diagnosis or at disease recurrence after previous diagnosis of an earlier stage melanoma.

Tumor harvest: Patients can undergo complete resection of their primary melanoma (if not already removed) and all regional metastatic disease with the intent of rendering them free of melanoma. After adequate tumor for pathological assessment has been harvested, remaining tumor tissue is placed in sterile media in a sterile container and prepared for disaggregation. Portions of the tumor tissue is used for whole-exome and transcriptome sequencing and cell line generation and any remaining tumor is frozen.

Normal tissue harvest: A normal tissue sample (blood or sputum sample ) is taken for whole exome sequencing.

Patients with clinically evident locoregional metastatic disease or fully resectable distant nodal, cutaneous or lung metastatic disease (but absence of unresectable distant or visceral metastatic disease) is identified and enrolled on the study. Entry of patients prior to surgery is necessary in order to acquire fresh tumor tissue for melanoma cell line development (to generate target cells for in vitro cytotoxicity assays as part of the immune monitoring plan).

### Example 3

### Dose and Schedule

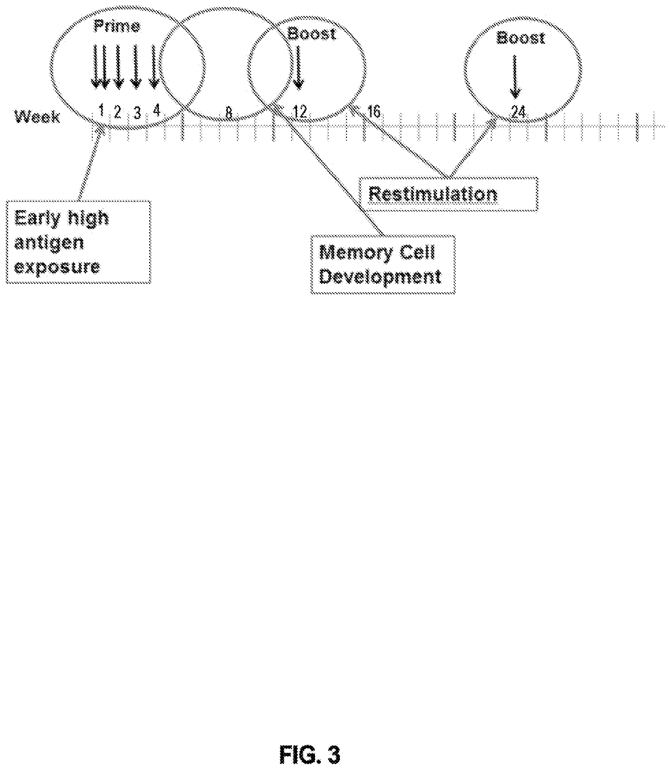

For patients who have met all pre-treatment criteria, vaccine administration can commence as soon as possible after the study drug has arrived and has met incoming specifications. For each patient, there is four separate study drugs, each containing 5 of 20 patient-specific peptides. Immunizations may generally proceed according to the schedule shown in FIG. 3.

Patients are treated in an outpatient clinic. Immunization on each treatment day can consist of four 1 ml subcutaneous injections, each into a separate extremity in order to target different regions of the lymphatic system to reduce antigenic competition. If the patient has undergone complete axillary or inguinal lymph node dissection, vaccines are administered into the right or left midriff as an alternative. Each injection can consist of 1 of the 4 study drugs for that patient and the same study drug is injected into the same extremity for each cycle. The composition of each 1 ml injection is:
0.75 ml study drug containing 300 µg each of 5 patient-specific peptides
0.25 ml (0.5 mg) of 2 mg/ml poly-ICLC (Hiltonol^{®})

During the induction/priming phase, patients are immunized on days 1, 4, 8, 15 and 22. In the maintenance phase, patients can receive booster doses at weeks 12 and 24.

Blood samples may be obtained at multiple time points: pre- (baseline; two samples on different days); day 15 during priming vaccination; four weeks after the induction/priming vaccination (week 8); pre- (week 12) and post- (week 16) first boost; pre- (week 24) and post-(week 28) second boost 50 - 150 ml blood is collected for each sample (except week 16). The primary immunological endpoint is at week 16, and hence patients can undergo leukapheresis (unless otherwise indicated based on patient and physician assessment).

### Example 4

### Immune Monitoring

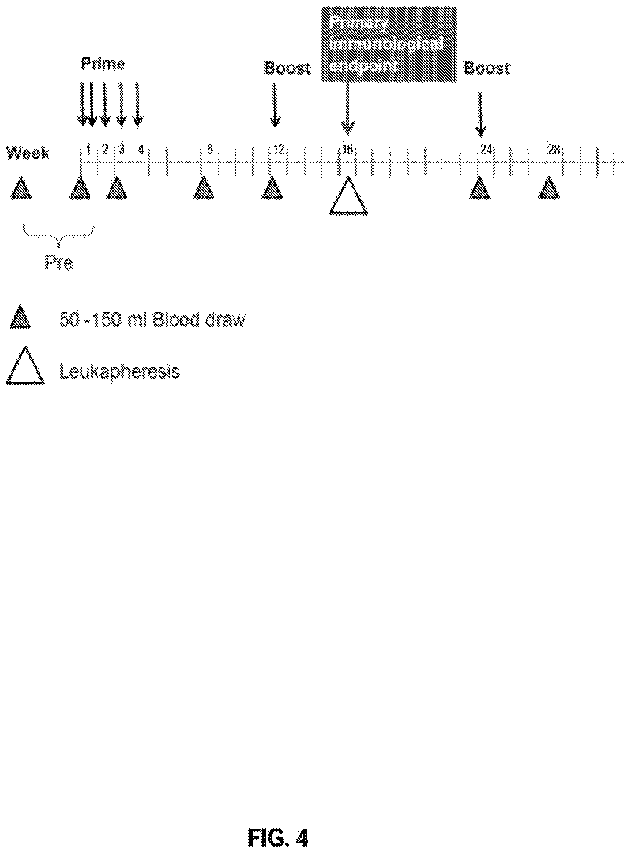

The immunization strategy is a "prime-boost" approach, involving an initial series of closely spaced immunizations to induce an immune response followed by a period of rest to allow memory T-cells to be established. This is followed by a booster immunization, and the T-cell response 4 weeks after this boost is expected to generate the strongest response and is the primary immunological endpoint. Global immunological response is initially monitored using peripheral blood mononuclear cells from this time point in an 18 hr ex vivo ELISPOT assay, stimulating with a pool of overlapping 15mer peptides (11 aa overlap) comprising all the immunizing epitopes. Pre-vaccination samples are evaluated to establish the baseline response to this peptide pool. As warranted, additional PBMC samples are evaluated to examine the kinetics of the immune response to the total peptide mix. For patients demonstrating responses significantly above baseline, the pool of all 15mers are de-convoluted to determine which particular immunizing peptide(s) were immunogenic. In addition, a number of additional assays are conducted on a case-by-case basis for appropriate samples:
- The entire 15mer pool or sub-pools are used as stimulating peptides for intracellular cytokine staining assays to identify and quantify antigen-specific CD4+, CD8+, central memory and effector memory populations
- Similarly, these pools are used to evaluate the pattern of cytokines secreted by these cells to determine the TH1 vs TH2 phenotype
- Extracellular cytokine staining and flow cytometry of unstimulated cells are used to quantify Treg and myeloid-derived suppressor cells (MDSC).
- If a melanoma cell line is successfully established from a responding patient and the activating epitope can be identified, T-cell cytotoxicity assays are conducted using the mutant and corresponding wild type peptide
- PBMC from the primary immunological endpoint is evaluated for "epitope spreading" by using known melanoma tumor associated antigens as stimulants and by using several additional identified mutated epitopes that were not selected to be among the immunogens, as shown in FIG. 4.

Immuno-histochemistry of the tumor sample is conducted to quantify CD4+, CD8+, MDSC, and Treg infiltrating populations.

### Example 5

### Neoantigen Preparation

Following surgical resection of the tumor, a portion of the tumor tissue and a blood sample is transferred immediately to the facility where it is assigned a unique identification code for further tracking. The tumor tissue is disaggregated with collagenase and separate portions are frozen for nucleic acid (DNA and RNA) extraction. The blood sample is immediately transferred to a facility for nucleic acid extraction. DNA and/or RNA extracted from the tumor tissue is used for whole-exome sequencing (e.g., by using the Illumina HiSeq platform) and to determine HLA typing information. It is contemplated within the scope of the invention that missense or neoORF neoantigenic peptides may be directly identified by protein-based techniques (e.g., mass spectrometry).

Bioinformatics analysis are conducted as follows. Sequence analysis of the Exome and RNA - SEQ fast Q files leverage existing bioinformatic pipelines that have been used and validated extensively in large-scale projects such as the TCGA for many patient samples (e.g., Chapman et al, 2011, Stransky et al, 2011, Berger et al, 2012). There are two sequential categories of analyses: data processing and cancer genome analysis.

Data processing pipeline: The Picard data processing pipeline (picard.sourceforge.net/) was developed by the Sequencing Platform. Raw data extracted from (e.g., Illumina) sequencers for each tumor and normal sample is subjected to the following processes using various modules in the Picard pipeline:
(i) Data conversion: Raw Illumina data is converted to the standard BAM format and basic QC metrics pertaining to the distribution of bases exceeding different quality thresholds are generated.
(ii) Alignment: The Burrows-Wheeler Alignment Tool (BWA) is used to align read pairs to the human genome (hg19).
(iii) Mark Duplicates: PCR and optical duplicates are identified based on read pair mapping positions and marked in the final BAM file.
(iv) Indel Realignment: Reads that align to known insertion and deletion polymorphic sites in the genome is examined and those sites where the log odds (LOD) score for improvement upon realignment is at least 0.4 is corrected.
(v) Quality Recalibration: Original base quality scores reported by the Illumina pipeline is recalibrated based on the read-cycle, the lane, the flow cell tile, the base in question and the preceding base. The recalibration assumes that all mismatches in non-dbSNP positions are due to errors which enable recalibration of the probability of error in each category of interest as the fraction of mismatches amongst the total number of observations.
(vi) Quality Control: The final BAM file is processed to generate extensive QC metrics including read quality by cycle, distribution of quality scores, summary of alignment and the insert size distribution. Data that fails quality QC is blacklisted.
(vii) Identity Verification: Orthogonally collected sample genotype data at ~100 known SNP positions are checked against the sequence data to confirm the identity of the sample. A LOD score of ≥ 10 is used as a threshold for confirmation of identity. Data that fails identity QC is blacklisted.
(viii) Data Aggregation: All data from the same sample is merged and the mark duplicates step is repeated. Novel target regions containing putative short insertions and deletion regions are identified and the indel realignment step is performed at these loci.
(ix) Local realignment around putative indels in aggregated data: Novel target regions containing putative short insertions and deletions are identified and a local realignment step is performed at these loci (e.g., using the GATK RealignerTargetCreator and IndelRealigner modules) to ensure consistency and correctness of indel calls.
(x) Quality Control on Aggregated Data: QC metrics such as alignment summary and insert size distribution is recomputed. Additionally a set of metrics that evaluate the rate of oxidative damage in the early steps of the library constructions process caused by acoustic shearing of DNA in the presence of reactive contaminants from the extraction process are generated.

The output of Picard is a bam file (Li et al, 2009) (see, e.g., http://samtools.sourceforge.net/SAM1.pdf) that stores the base sequences, quality scores, and alignment details for all reads for the given sample.

Cancer Mutation Detection Pipeline: Tumor and matched normal bam files from the Picard pipeline is analyzed as described herein:
1. Quality Control
   (i). The Capseg program is applied to tumor and matched normal exome samples to get the copy number profiles. The CopyNumberQC tool can then be used to manually inspect the generated profiles and assess tumor/normal sample mix-ups. Normal samples that have noisy profiles as well as cases where the tumor sample has lower copy number variation than the corresponding normal is flagged and tracked through the data generation and analysis pipelines to check for mix-ups.
   (ii). Tumor purity and ploidy is estimated by the ABSOLUTE tool 15 based on Capseg-generated copy number profiles. Very noisy profiles might result from sequencing of highly degraded samples. No tumor purity and ploidy estimates would be possible in such cases and the corresponding sample is flagged.
   (iii). ContEst (Cibulskis et al, 2011) is used to determine the level of crosssample contamination in samples. Samples with greater than 4% contamination is discarded.
2. Identification of somatic single nucleotide variations (SSNVs)
   Somatic base pair substitutions are identified by analyzing tumor and matched normal bams from a patient using a Bayesian statistical framework called muTect (Cibulskis et al, 2013). In the preprocessing step, reads with a preponderance of low quality bases or mismatches to the genome are filtered out. Mutect then computes two log-odds (LOD) scores which encapsulate confidence in presence and absence of the variant in the tumor and normal samples respectively. In the post-processing stage candidate mutations are filtered by six filters to account for artifacts of capture, sequencing and alignment:
   (i) Proximal gap: removes false positives that arise due to the presence of misaligned indels in the vicinity of the event. Samples with ≥ 3 reads with insertions or deletions in a 11-bp window around the candidate mutation are rejected.
   (ii) Poor mapping: discards false positives that arise by virtue of ambiguous placement of reads in the genome. Rejects candidates if ≥ 50% reads in tumor and normal samples have mapping quality zero or if there are no reads harboring the mutant allele with mapping quality ≥ 20.
   (iii) Trialleleic sites: discards sites that are heterozygous in the normal since these have a tendency to generate many false positives.
   (iv) Strand bias: removes false positives caused by context-specific sequencing errors where a large fraction of reads harboring the mutation have the same orientation. Rejects candidates where the strand-specific LOD is < 2 where the sensitivity to pass that threshold is ≥ 90%.
   (v) Clustered position: rejects false positives due to alignment errors characterized by the alternative allele occurring at a fixed distance from the start or end of the read alignment. Rejects if the median distance from the start and end of the reads are ≤ 10 which implies that the mutation is at the start or end of the alignment, or if the median absolute deviation of the distances are ≤ 3 which implies that the mutations are clustered.
   (vi) Observed in control: discards false positives in the tumor where there is evidence of occurrence of the alternate allele in the normal sample beyond what is expected by random sequencing errors. Rejects if there are ≥ 2 reads containing the alternate allele in the normal sample or if they are in ≥ 3% of the reads, and if the sum of their quality scores are > 20.
   In addition to these 6 filters, candidates are compared against a panel of normal samples and those that are found to be present as germline variants in two or more normal samples are rejected. The final set of mutations can then be annotated with the Oncotator tool by several fields including genomic region, codon, cDNA and protein changes.
3. Identification of somatic small insertions and deletions
   The local realignment output described herein (see "Local realignment around putative indels in aggregated data", supra) is used to predict candidate somatic and germline indels based on assessment of reads supporting the variant exclusively in tumor or both in tumor and normal bams respectively. Further filtering based on number and distribution of mismatches and base quality scores are done (McKenna et al, 2010, DePristo et al, 2011). All indels are manually inspected using the Integrated Genomics Viewer (Robinson et al, 2011) (www.broadinstitute.org/igv) to ensure high-fidelity calls.
4. Gene fusion detection
   The first step in the gene fusion detection pipeline is alignment of tumor RNA-Seq reads to a library of known gene sequences following by mapping of this alignment to genomic coordinates. The genomic mapping helps collapse multiple read pairs that map to different transcript variants that share exons to common genomic locations. The DNA aligned bam file is queried for read pairs where the two mates map to two different coding regions that are either on different chromosomes or at least 1 MB apart if on the same chromosome. It can also be required that the pair ends aligned in their respective genes be in the direction consistent with coding->coding 5'-> 3' direction of the (putative) fusion mRNA transcript. A list of gene pairs where there are at least two such 'chimeric' read pairs are enumerated as the initial putative event list subject to further refinement. Next, all unaligned reads are extracted from the original bam file, with the additional constraint that their mates were originally aligned and map into one of the genes in the gene pairs obtained as described herein. An attempt can then be made to align all such originally unaligned reads to the custom "reference" built of all possible exon-exon junctions (full length, boundary-to-boundary, in coding 5'-> 3' direction) between the discovered gene pairs. If one such originally unaligned read maps (uniquely) onto a junction between an exon of gene X and an exon of gene Y, and its mate was indeed mapped to one of the genes X or Y, then such a read is marked as a "fusion" read. Gene fusion events are called in cases where there is at least one fusion read in correct relative orientation to its mate, without excessive number of mismatches around the exon:exon junction and with a coverage of at least 10 bp in either gene. Gene fusions between highly homologous genes (ex. HLA family) are likely spurious and is filtered out.
5. Estimation of clonality
   Bioinformatic analysis may be used to estimate clonality of mutations. For example, the ABSOLUTE algorithm (Carter et al, 2012, Landau et al, 2013) may be used to estimate tumor purity, ploidy, absolute copy numbers and clonality of mutations. Probability density distributions of allelic fractions of each mutation is generated followed by conversion to cancer cell fractions (CCFs) of the mutations. Mutations are classified as clonal or subclonal based on whether the posterior probability of their CCF exceeds 0.95 is greater or lesser than 0.5 respectively.
6. Quantification of expression
   The TopHat suite (Langmead et al, 2009) is used to align RNA-Seq reads for the tumor and matched normal bams to the hg19 genome. The quality of RNA-Seq data is assessed by the RNA-SeQC (DeLuca et al., 2012) package. The RSEM tool (Li et al., 2011) can then be used to estimate gene and isoform expression levels. The generated reads per kilobase per million and tau estimates are used to prioritize neoantigens identified in each patient as described elsewhere.
7. Validation of mutations in RNA-Seq
8. Confirmation of the somatic mutations identified by analysis of whole exome data as described herein (including single nucleotide variations, small insertions and deletions and gene fusions) are assessed by examining the corresponding RNA-Seq tumor BAM file of the patient. For each variant locus, a power calculation based on the beta-binomial distribution is performed to ensure that there is at least 95% power to detect it in the RNA-Seq data. A capture identified mutation is considered validated if there are at least 2 reads harboring the mutation for adequately powered sites.

Selection of Tumor-Specific Mutation-Containing Epitopes: All missense mutations and neoORFs are analyzed for the presence of mutation-containing epitopes using the neuralnetwork based algorithm netMHC, provided and maintained by the Center for Biological Sequence Analysis, Technical University of Denmark, Netherlands. This family of algorithms were rated the top epitope prediction algorithms based on a competition recently completed among a series of related approaches (ref). The algorithms were trained using an artificial neural network based approach on 69 different human HLA A and B alleles covering 99% of the HLA-A alleles and 87% of the HLA-B alleles found in the Caucasian population, the major ethnic group in the target patient population in the local area. The most up-do-date version is utilized (v2.4 ).

The accuracy of the algorithms were evaluated by conducting predictions from mutations found in CLL patients for whom the HLA allotypes were known. The included allotypes were A0101, A0201, A0310, A1101, A2402, A6801, B0702, B0801, B1501. Predictions were made for all 9mer and 10 mer peptides spanning each mutation using netMHCpan in mid-2011. Based on these predictions, seventy-four (74) 9mer peptides and sixty-three (63) 10mer peptides, most with predicted affinities below 500 nM, were synthesized and the binding affinity was measured using a competitive binding assay (Sette).

The predictions for these peptides were repeated in March 2013 using each of the most up to date versions of the netMHC servers (netMHCpan, netMHC and netMHCcons). These three algorithms were the top rated algorithms among a group of 20 used in a competition in 2012 (Zhang et al). The observed binding affinities were then evaluated with respect to each of the new predictions. For each set of predicted and observed values, the % of correct predictions for each range is given, as well as the number of samples. The definition for each range is as follows:
0 - 150: Predicted to have an affinity equal to or lower than 150 nM and measured to have an affinity equal to or lower than 150 nM.
0 - 150^{∗}: Predicted to have an affinity equal to or lower than 150 nM and measured to have an affinity equal to or lower than 500 nM.
151 - 500 nM: Predicted to have an affinity greater than 150 nM but equal to or lower than 500 nM and measured to have an affinity equal to or below 500 nM.
FN (> 500 nM): False Negatives - Predicted to have an affinity greater than 500 nM but measured to have an affinity equal to or below 500 nM.

For 9mer peptides (Table 1) , there was little difference between the algorithms, with the slightly higher value for the 151- 500 nM range for netMHC cons not judged to be significant because of the low number of samples.

**Table 1**

| **Range (nM)** | **9mer PAN** | **9mer netMHC** | **9mer CONS** |
|---|---|---|---|
| **0-150** | 76% (33) | 78% (37) | 76% (34) |
| **0-150**^{∗} | 91% (33) | 89% (37) | 88% (34) |
| **151-500** | 50% (28) | 50% (14) | 62% (13) |
| **FN (>500)** | 38% (13) | 39% (23) | 41% (27) |

For 10mer peptides (Table 2), again there was little difference between the algorithms except that netMHC produced significantly more false positives than netMHCpan or netMMHCcons. However, the precision of the 10mer predictions are slightly lower in the 0 - 150 nM and 0 - 150^{∗} nM ranges and significantly lower in the 151-500 nM range, compared to the 9mers.

**Table 2**

| **Range (nM)** | **10mer PAN** | **10mer netMHC** | **10mer CONS** |
|---|---|---|---|
| **0-150** | 53% (19) | 50% (16) | 59% (17) |
| **0-150^{∗}** | 68% (19) | 69% (16) | 76% (17) |
| **151-500** | 35% (26) | 42% (12) | 35% (23) |
| **FN (>500)** | 11% (18) | 23% (35) | 13% (23) |

For 10mers, only predictions in the 0 - 150 nM range is utilized due to the lower than 50% precision for binders in the 151-500 nM range.

The number of samples for any individual HLA allele was too small to draw any conclusions regarding accuracy of the prediction algorithm for different alleles. Data from the largest available subset (0 - 150^{∗} nM; 9mer) is shown in Table 3 as an example.

**Table 3**

| Allele | Fraction correct |
|---|---|
| A0101 | 2/2 |
| A0201 | 9/11 |
| A0301 | 5/5 |
| A1101 | 4/4 |
| A2402 | 0/0 |
| A6801 | 3/4 |
| B0702 | 4/4 |
| B0801 | 1/2 |
| B1501 | 2/2 |

Only predictions for HLA A and B alleles are utilized as there is little available data on which to judge accuracy of predictions for HLA C alleles (Zhang et al).

An evaluation of melanoma sequence information and peptide binding predictions was conducted using information from the TCGA database. Information for 220 melanomas from different patients revealed that on average there were approximately 450 missense and 5 neoORFs per patient. 20 patients were selected at random and the predicted binding affinities were calculated for all the missense and neoORF mutations using netMHC (Lundegaard et al Prediction of epitopes using neural network based methods J Immunol Methods 374:26 (2011)). As the HLA allotypes were unknown for these patients, the number of predicted binding peptides per allotype were adjusted based on the frequency of that allotype (Bone Marrow Registry dataset for the expected affected dominant population in the geographic area [Caucasian for melanoma]) to generate a predicted number of actionable mutant epitopes per patient. For each of these mutant epitopes (MUT), the corresponding native (NAT) epitope binding was also predicted.

### Utilizing the prioritization described herein:

- 90% (18 of 20) of patients were predicted to have at least 20 peptides appropriate for vaccination;
- For nearly a quarter of the patients, neoORF peptides could constitute half to all of the 20 peptides;
- For just over half of the patients, only peptides in categories 1 and 2 would be used;
- For 80% of the patients, only peptides in categories 1, 2, and 3 would be utilized.

Thus, there is a sufficient number of mutations in melanoma to expect a high proportion of patients to generate an adequate number of immunogenic peptides.

### Example 6

### Peptide Production and Formulation

GMP neoantigenic peptides for immunization is prepared by chemical synthesis Merrifield RB: Solid phase peptide synthesis. I. The synthesis of a tetrapeptide. J. Am. Chem. Soc. 85:2149-54, 1963) in accordance with FDA regulations. Three development runs have been conducted of 20 ~20-30mer peptides each. Each run was conducted in the same facility and utilized the same equipment as is used for the GMP runs, utilizing draft GMP batch records. Each run successfully produced > 50 mg of each peptide, which were tested by all currently planned release tests (e.g., Appearance, Identify by MS, Purity by RP-HPLC, Content by Elemental Nitrogen, and TFA content by RP-HPLC) and met the targeted specification where appropriate. The products were also produced within the timeframe anticipated for this part of the process (approximately 4 weeks). The lyophilized bulk peptides were placed on a long term stability study and is evaluated at various time points up to 12 months.

Material from these runs has been used to test the planned dissolution and mixing approach. Briefly, each peptide is dissolved at high concentration (50 mg/ml) in 100% DMSO and diluted to 2 mg/ml in an aqueous solvent. Initially, it was anticipated that PBS would be used as a diluent, however, a salting out of a small number of peptides caused a visible cloudiness. D5W (5% dextrose in water) was shown to be much more effective; 37 of 40 peptides were successfully diluted to a clear solution. 10% sucrose or 10% Trehalose in water also is effective. The formulation containing 10% sucrose or 10% trehalose is lyophilizable unlike the formulation containing 5% Dextrose. The only problematic peptides are very hydrophobic peptides.

Table 4 shows the results of solubility evaluations of 60 potential neoantigen peptides, sorted based on the calculated fraction of hydrophobic amino acids. As shown, almost all peptides with a hydrophobic fraction lower than 0.4 are soluble in DMSO/D5W, but a number of peptides with a hydrophobic fraction greater than or equal to 0.4 were not soluble in DMSO/D5W (indicated by red highlighting in the column labeled "Solubility in DMSO/D5W"). A number of these can be solubilized by addition of succinate (indicated by green highlighting in the column "Solubility in DMSO/D5W/Succinate"). 3 of 4 of these peptides had hydrophobic fractions between 0.4 and 0.43. Four peptides became less soluble upon addition of succinate; 3 of 4 of these peptides had a hydrophobic fraction greater than or equal to 0.45.

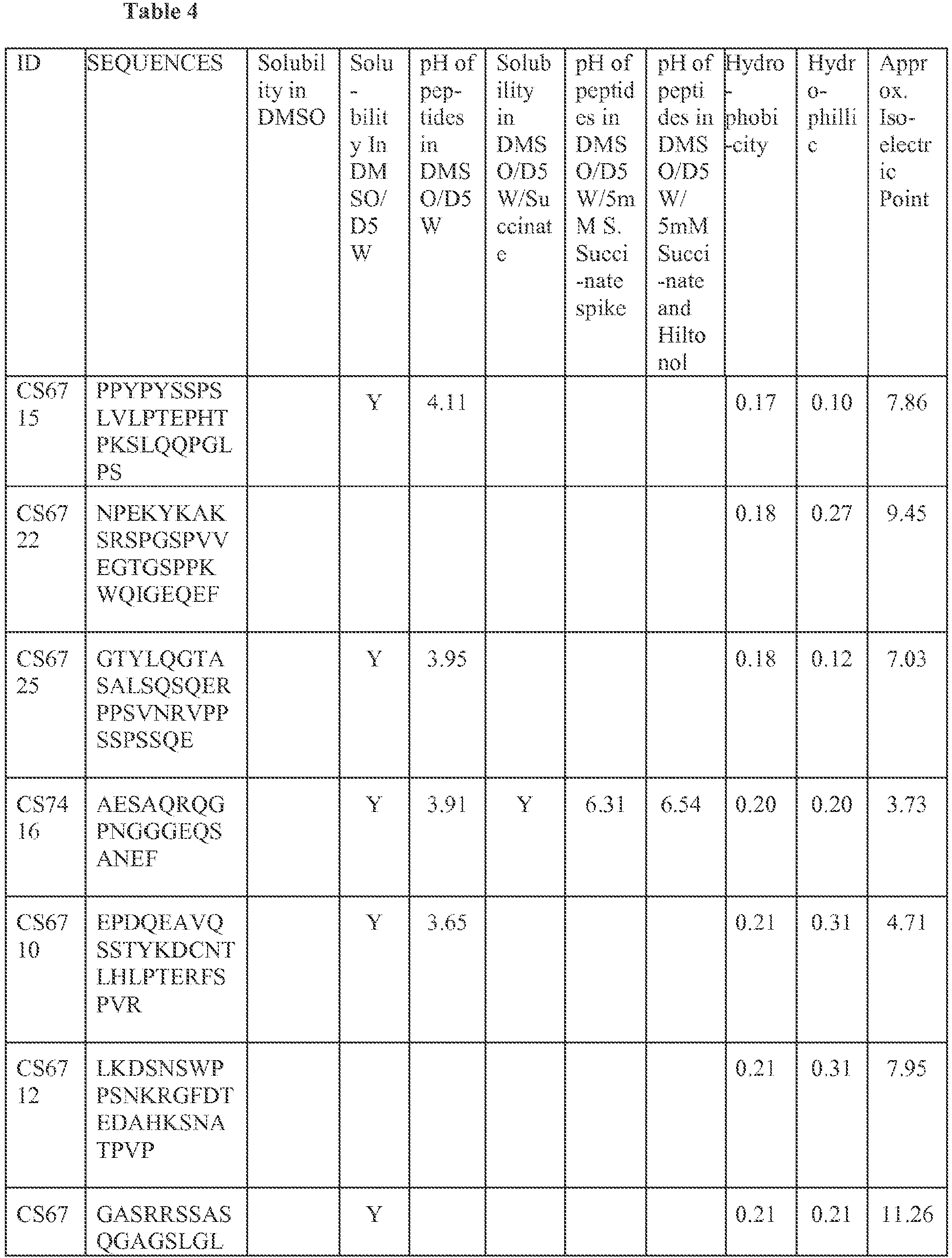

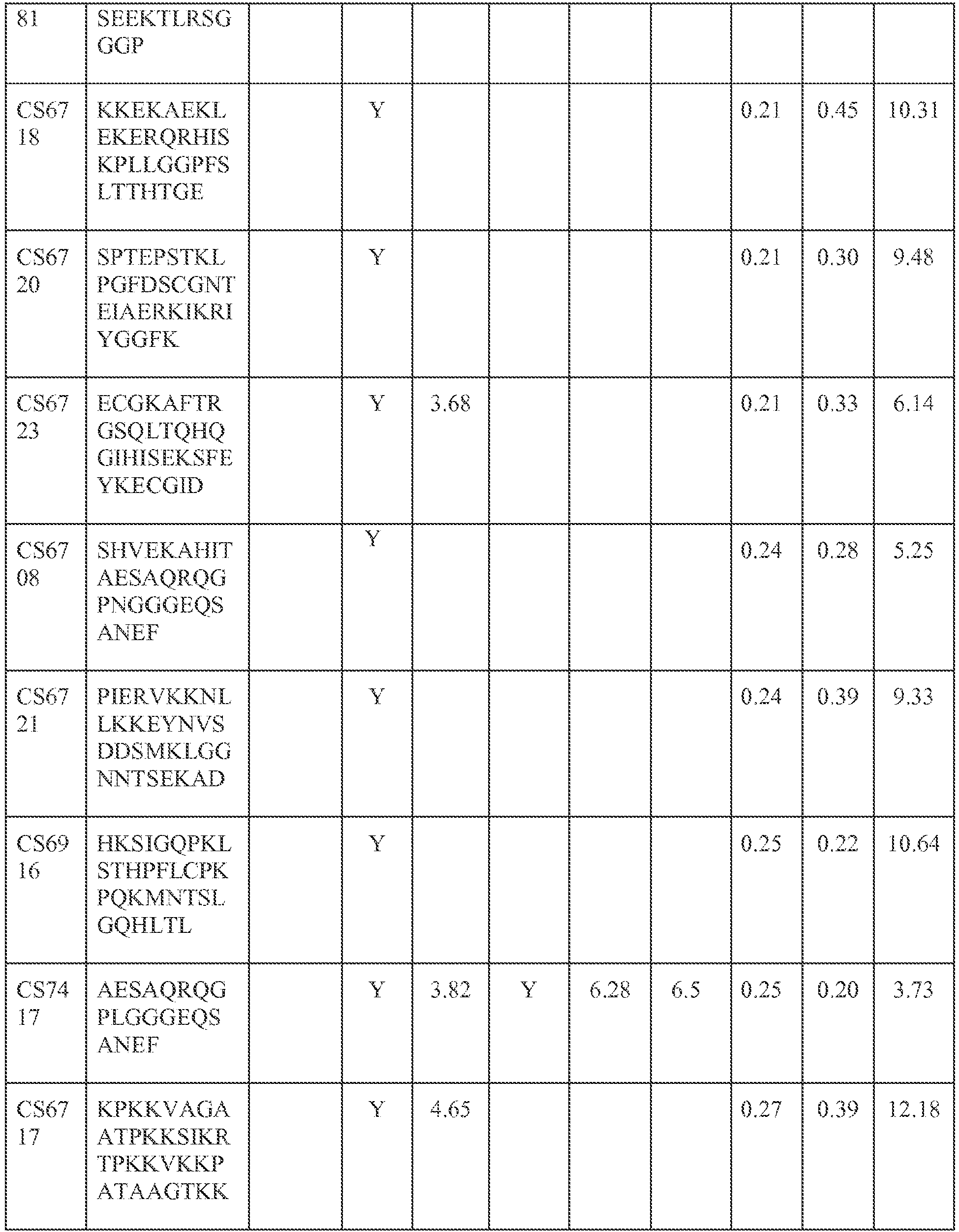

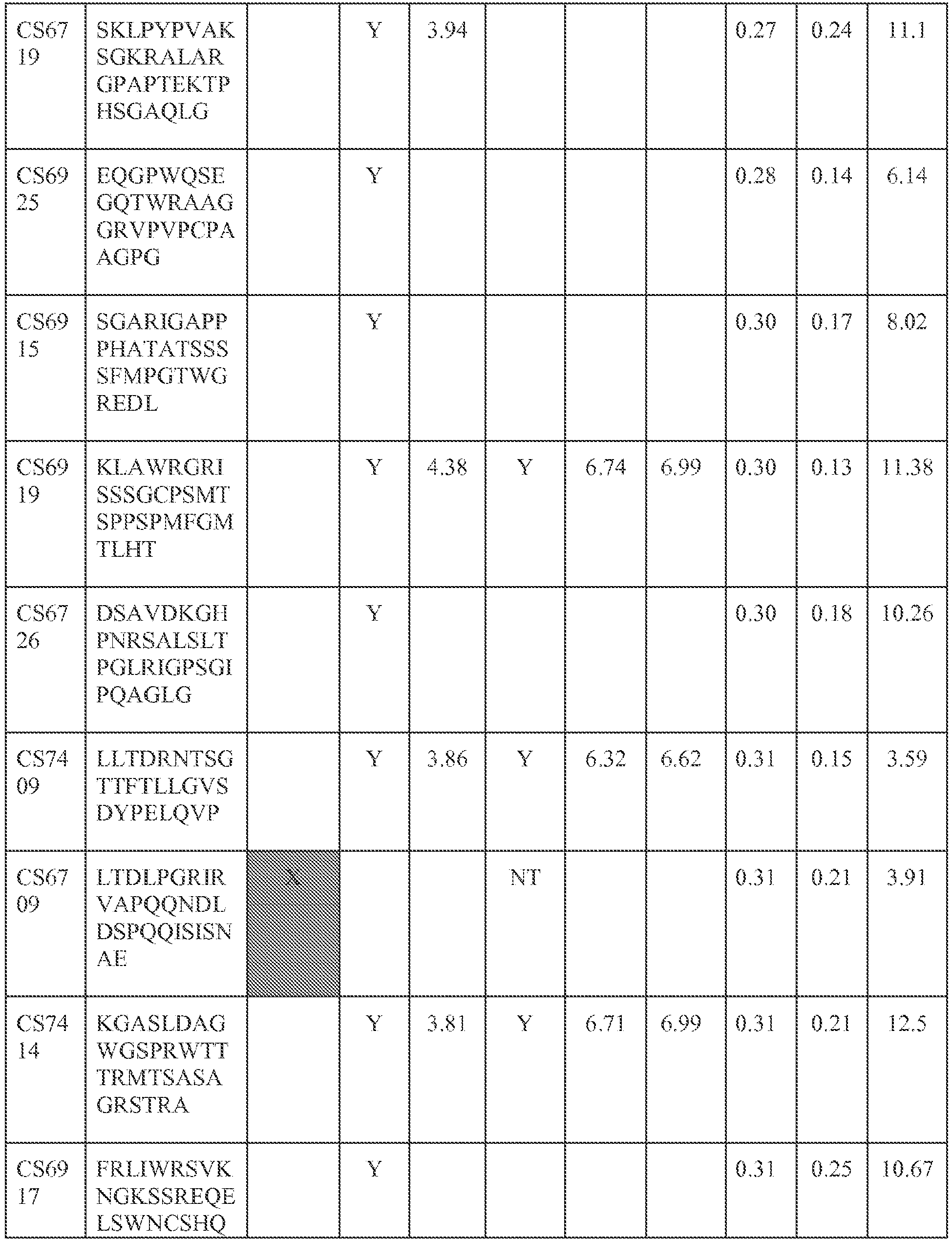

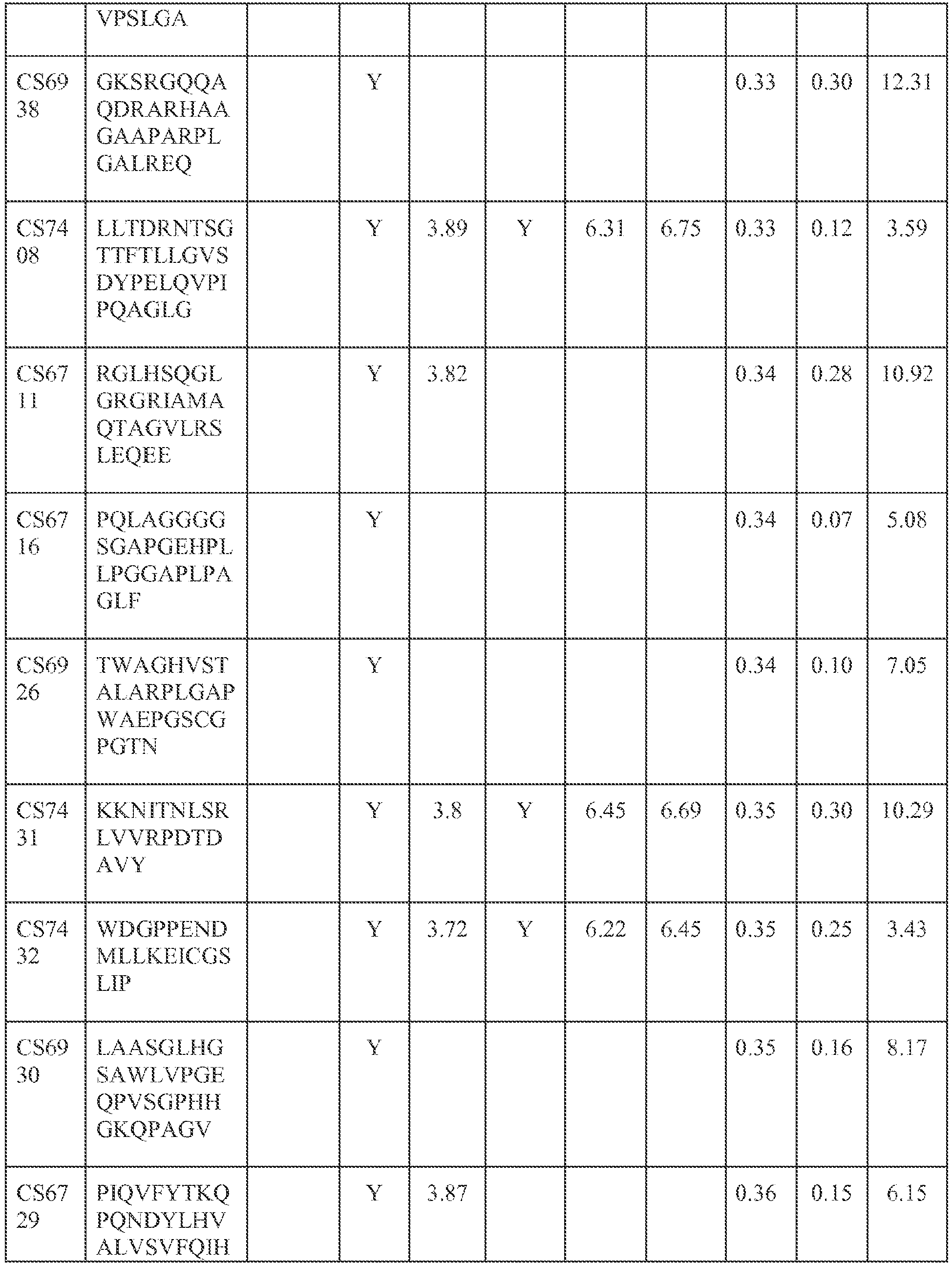

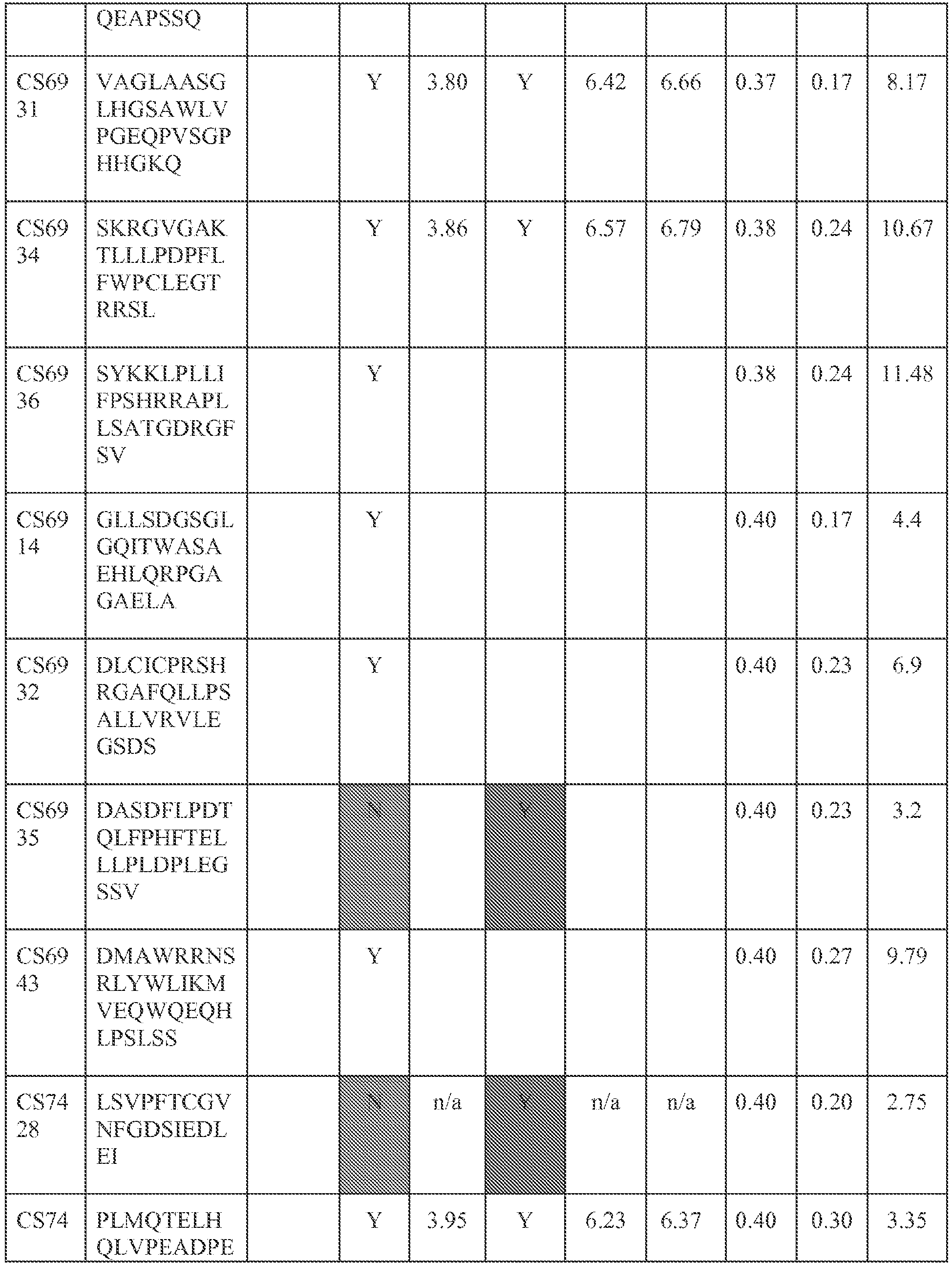

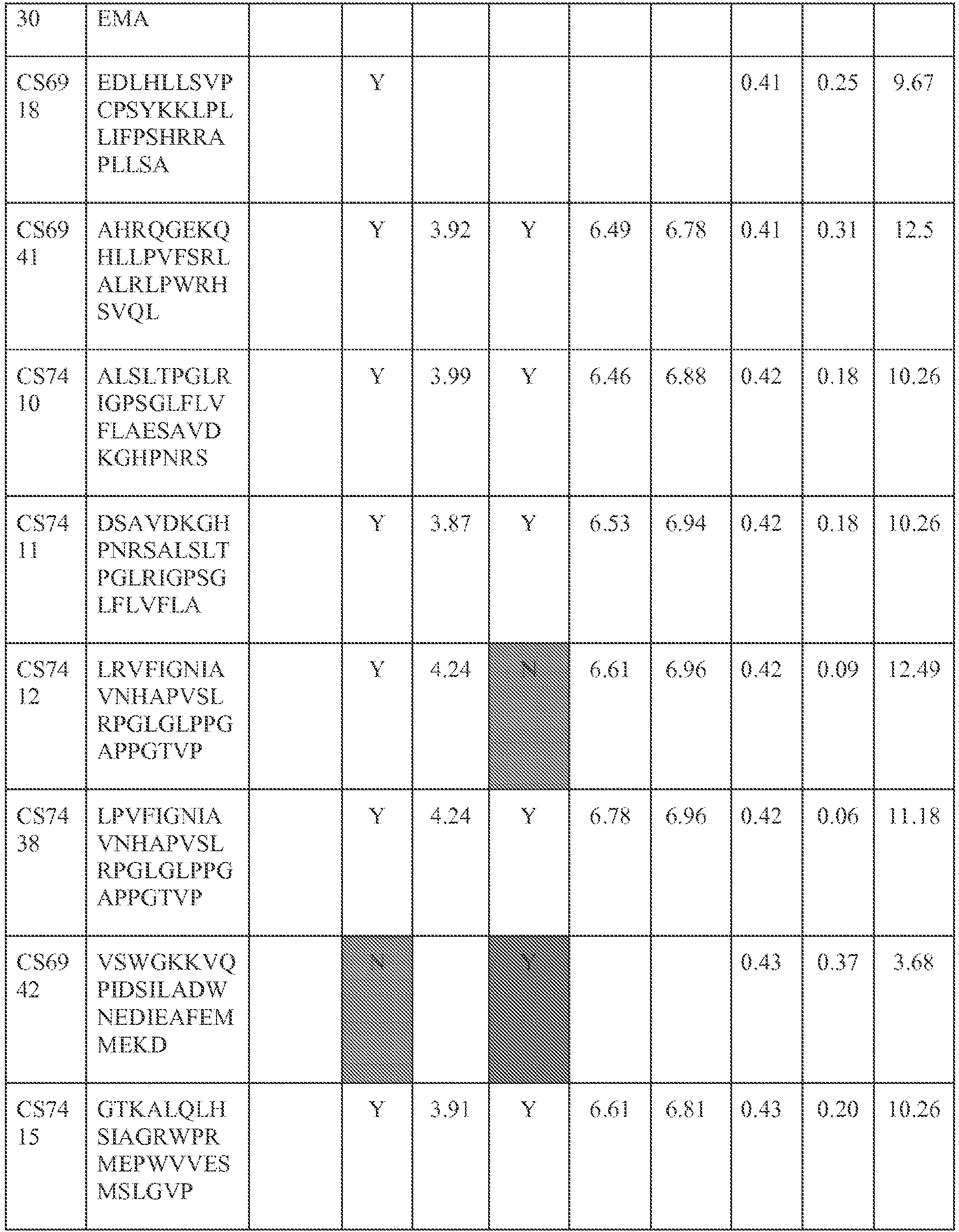

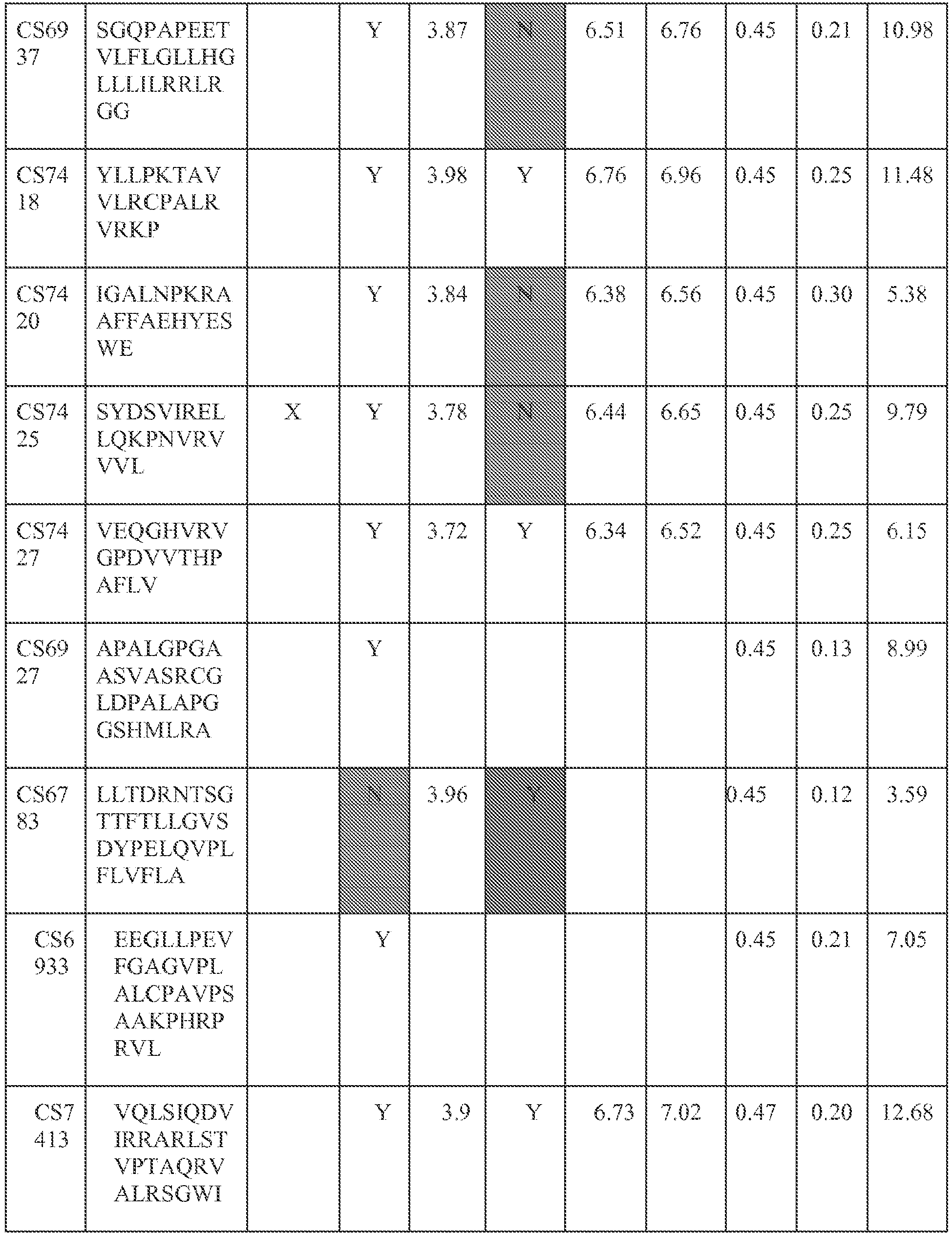

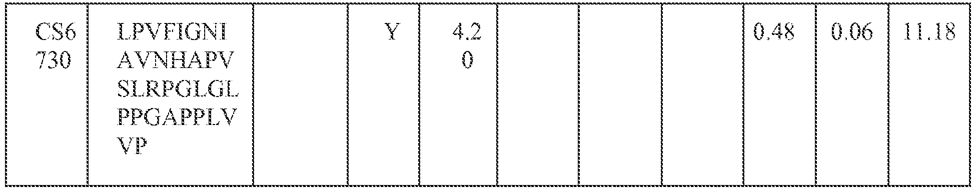

The predicted biochemical properties of planned immunizing peptides are evaluated and synthesis plans may be altered accordingly (using a shorter peptide, shifting the region to be synthesized in the N- or C-terminal direction around the predicted epitope, or potentially utilizing an alternate peptide) in order to limit the number of peptides with a high hydrophobic fraction.

Ten separate peptides in DMSO/D5W were subjected to two freeze/thaw cycles and showed full recovery. Two individual peptides were dissolved in DMSO/D5W and placed on stability at two temperatures (-20°C and -80°C). These peptides were evaluated (RP-HPLC and pH and visual inspection) for up to 24 weeks. Both peptides are stable for up to 24 weeks; the percent impurities detected by the RP-HPLC assay did not change significantly for either peptide when stored at either -20°C or -80° C. Any small changes appear to be due to assay variability as no trends were noted to be evaluated.

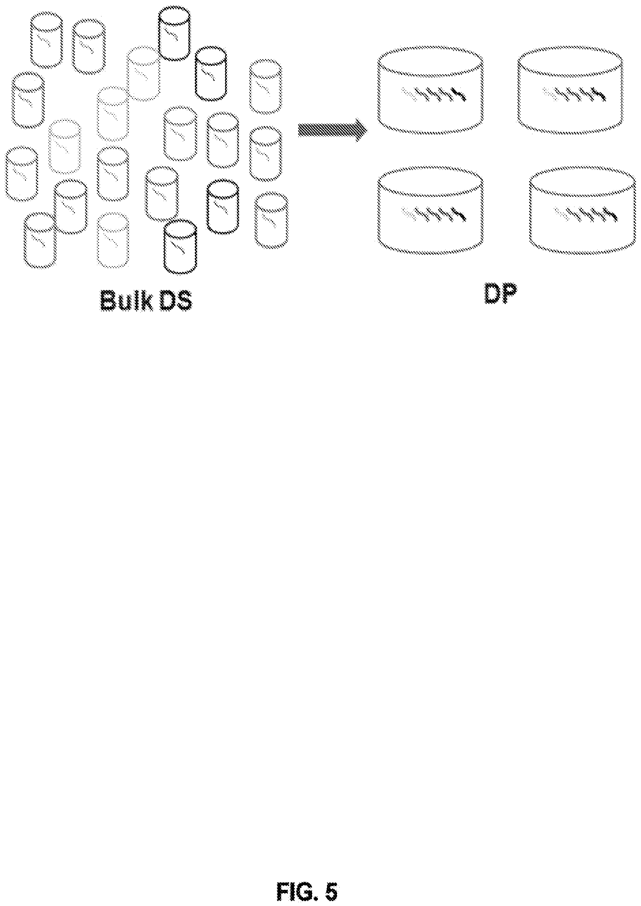

As shown in FIG. 5, the design of the dosage form process are to prepare 4 pools of patient-specific peptides consisting of 5 peptides each. A RP-HPLC assay has been prepared and qualified to evaluate these peptide mixes. This assay achieves good resolution of multiple peptides within a single mix and can also be used to quantitate individual peptides.

Membrane filtration (0.2 µm pore size) is used to reduce bioburden and conduct final filter sterilization. Four different appropriately sized filter types were initially evaluated and the Pall, PES filter (# 4612) was selected. To date, 4 different mixtures of 5 different peptides each have been prepared and individually filtered sequentially through two PES filters. Recovery of each individual peptide was evaluated utilizing the RP-HPLC assay. For 18 of the 20 peptides, the recovery after two filtrations was >90%. For two highly hydrophobic peptides, the recovery was below 60% when evaluated at small scale but were nearly fully recovered (87 and 97%) at scale. As stated herein, approaches are undertaken to limit the hydrophobic nature of the sequences selected.

A peptide pool (Pool 4) consisting of five peptides was prepared by dissolution in DMSO, dilution with D5W/Succinate (5 mM) to 2 mg/ml and pooling to a final peptide concentration of 400 µg per ml and a final DMSO concentration of 4%. After preparation, peptides were filtered with a 25mm Pall PES filter (Cat # 4612) and dispensed into Nunc Cryo vials (# 375418) in one ml aliquots. Samples were analyzed at time zero and at 2 and 4 weeks to date. Additional samples are analyzed at 8 and 24 weeks. At -80°C, no significant change in the HPLC profiles or impurity profile of the peptide Pool 4 was observed at the four-week time point. Through the 4 week time point, visual observation and pH for the peptide pool did not change.

### Example 7

### Peptide synthesis

GMP peptides are synthesized by standard solid phase synthetic peptide chemistry (e.g., using CS 536 XT peptide synthesizers) and purified by RP-HPLC. Each individual peptide is analyzed by a variety of qualified assays to assess appearance (visual), purity (RP-HPLC), identity (by mass spectrometry), quantity (elemental nitrogen), and trifluoroacetate counterion (RP-HPLC) and released.

The personalized neoantigen peptides may be comprised of up to 20 distinct peptides unique to each patient. Each peptide may be a linear polymer of ~20 - ~30 L-amino acids joined by standard peptide bonds. The amino terminus may be a primary amine (NH2-) and the carboxy terminus is a carbonyl group (-COOH). The standard 20 amino acids commonly found in mammalian cells are utilized (alanine, arginine, asparagine, aspartic acid, cysteine, glutamine, glutamic acid , glycine, histidine, isoleucine, leucine lysine, methionine, phenylalanine, proline, serine, threonine, tryptophan, tyrosine, valine). The molecular weight of each peptide varies based on its length and sequence and is calculated for each peptide.

Fmoc (9-fluorenylmethoyloxycarbnyl )-N-terminal protected amino acids are utilized for all synthesis reactions. The side chains of the amino acids are protected by 2,2,4,6,7-pentamethyl-dihydrobenzofuran-5-sulfonyl (Pbf), triphenylmethyl (Trt), t-butyloxycarbonyl (Boc) or t-butyl ether (tBu) group as appropriate. All bulk amino acids are dissolved in dimethylformamide (DMF). Condensation utilizes the following two catalyst combinations in separate reactions:
Diisopylcarbodiimide/1-Hydroxybenzotriazole (DIC/HOBT)
Diisoproplyethylamine/2-(1H-Benzotriazol-1-yl)-1,1,3,3-tetramethyluronium hexafluorophosphate (DIEA/HBTU)

Each amino acid is coupled twice in order to ensure high level of incorporation. The first coupling utilizes DIC/HOBT for 2 - 6 hours and the second coupling utilizes DIEA/HBTU for 1 - 2 hours. Each of the two couplings are monitored by UV absorbance and the resin is washed extensively with DMF in between coupling cycles to improve efficiency. After two cycles of coupling, calculated coupling efficiency must be at least 95% to continue to the next cycle. Further synthesis of any peptides that do not meet that minimal coupling efficiency is stopped.

After all amino acids have been coupled, the resin is washed twice with DMF and subsequently three times with methanol. The resin is then vacuum dried briefly while still in the reaction vessel and then transferred to a new, tared vessel for vacuum drying (greater than 12 hours) until it is freely flowing. The mass of crude peptide synthesized is determined by weighing the vessel containing dried resin, subtracting the mass of the tared vessel and adjusting for the resin mass. Expected mass yields range from 60% - 90%. Any synthesis that failed to produce at least 200 mg crude peptide is terminated. The dried resin may be stored at 4oC for up to 28 days prior to initiation of cleavage.

The cleavage reaction is conducted in a single room. Prior to transfer of the set of patient-specific dried resins from the synthesis room to the cleavage room, the cleavage room is fully qualified by QA for synthesis of a new GMP product. Qualification includes line clearance inspection, verification of GMP suite cleaning, staging of all required materials and glassware, verification of equipment suitability and labeling, and verification that all required personnel are properly trained and qualified to conduct the work and are properly gowned and free of apparent illness.

Room readiness operations initiates with verification of the equipment to be used (rotary evaporator, vacuum pump, balance) and inspection of documentation indicating that the equipment has been properly cleaned and calibrated (if appropriate). A complete list of all raw materials (TFA, triisopropylsilane (TIS) and 1,2-ethanedithiol) required is issued by QA and manufacturing identifies lot number to be utilized, retest or expiration date and quantity of material dispensed for each day's reactions.

Cleavage of the peptide chain from the resin and cleavage of the side chain protecting groups are accomplished under acidic conditions (95% TFA) in the presence of 2 % triisopropylsilane (TIS) and 1% 1,2-ethanedithiol as scavengers of acid-generated free-radicals for 3 to 4 hours at room temperature.

Resin is separated from free crude peptide by filtration. The final solution of released and de-protected peptide undergoes precipitation with ether and the precipitate is freeze-dried for 12 hours. The yield of released crude peptide is determined by weighing the freeze-dried powder and calculating the ratio of released crude peptide/resin-bound peptide. Expected yields of crude peptide are 200 mg to 1000 mg. Any cleavage reaction that fails to yield at least 200 mg crude peptide is terminated. The crude peptide is then transferred to the purification suite.

The purification is conducted in a single room. Prior to transfer of the set of dried crude peptide from the cleavage room to the purification room, the purification room is fully qualified by Quality Assurance for synthesis of a new GMP product. Qualification includes line clearance inspection, verification of GMP suite cleaning, staging of all required materials and glassware, verification of equipment suitability and labeling, and verification that all required personnel are properly trained and qualified to conduct the work and are properly gowned and free of apparent illness.

Room readiness operations initiates with verification of the equipment to be used (preparative Reverse-Phase High-Performance Liquid Chromatography [RP-HPLC], balance, analytical Liquid Chromatography/Mass Spectrometer (LC/MS), lyophilizer, balance) and inspection of documentation indicating that the equipment has been properly cleaned and calibrated (if appropriate). A complete list of all raw materials (trifluoroacetic acid [TFA], acetonitrile [ACN], water) required is issued by QA and Manufacturing identifies lot number to be utilized, retest or expiration date and quantity of material dispensed for each day's reactions.

Purification is initiated by dissolving no more than 200 mg of the freeze-dried released peptide in ACN. The sample is then further diluted with water to 5% -10% ACN. TFA is added to a final concentration of 0.1%. One C-18 RP-HPLC column (10 cm x 250 cm) is freshly packed prior to the initiation of each set of patient specific peptides. Columns are extensively washed with 5% acetonitrile containing 0.1% TFA prior to loading patient peptide. Maximum amount of peptide loaded onto a single column is 200 mgs. Columns are monitored by UV observance at 220 nm. Following loading of single peptide, the sample is allowed to enter the column and column is washed with 5% acetonitrile/0.1% TFA. A 10% - 50% gradient of acetonitrile with 0.1% TFA is used to elute the peptide. Fractions is collected (50 ml each) beginning at the point UV observance is at 20% above baseline. Fractions continue to be collected until no further UV absorbing material is eluting from the column or the gradient is complete. Typically, the main elution peak is separated into 4 to 8 fractions.

Each individual fraction is assessed by analytical LC/MS. Analytical conditions chosen is based on the percent acetonitrile associated with the peak eluted product. Fractions with the expected mass and purity greater than or equal to 95% is pooled as peptide product. Typically 2 to 4 fractions meet this pooling requirement. The pooled peptide is placed into a tared jar for freeze-drying and freeze-dried for 24 to 72 hours. The mass of lyophilized peptide is determined by determining the mass of the jar containing freeze-dried peptide and subtracting the mass of the tared jar.

Portions of the freeze-dried peptide is transferred to quality control for analysis and disposition. The remaining is stored at -20oC prior to further processing.

Any peptides for which none of the fractions meet the requirement of 95% purity is discarded. No reprocessing of RP-HPLC fractions can occur. If sufficient unpurified freeze-dried and cleaved peptide is available, a second sample of the peptide may be purified over the column, adjusting the gradient conditions to improve purity of the eluted peptide.

The column can then be stripped of any remaining peptide by washing extensively with 4 column volumes of 100% ACN/0.1 % TFA and then re-equilibrated with 5% ACN/0.1% TFA prior to loading the next peptide.

Peptides for an individual patient is sequentially processed over the same column. No more than 25 peptides are processed over a single column.

Unit operations for drug substance manufacturing thus constitute:
Synthesis:
   Condensation, wash and re-condensation for each amino acid
   Resin washing and vacuum drying
   Transfer to the cleavage suite
Cleavage:
   Acid cleavage from the resin
   Separation of released peptide from the resin and peptide precipitation
   Transfer to the purification suite
Purification:
   Dissolution in acetonitrile and RP- HPLC purification
   Freeze-drying of peak fractions for 24 to 72 hours
   Removal of aliquots for QC testing and storage of remaining lyophilized product.

Personalized neoantigen peptides may be supplied as a box containing 2 ml Nunc Cryo vials with color-coded caps, each vial containing approximately 1.5 ml of a frozen DMSO/D5W solution containing up to 5 peptides at a concentration of 400 ug/ml. There may be 10 - 15 vials for each of the four groups of peptides. The vials are to be stored at -80°C until use. Ongoing stability studies support the storage temperature and time.

Storage and Stability: The personalized neoantigen peptides are stored frozen at - 80oC. The thawed, sterile filtered, in process intermediates and the final mixture of personalized neoantigen peptides and poly-ICLC can be kept at room temperature but should be used within 4 hours.

Compatibility: The personalized neoantigen peptides are mixed with 1/3 volume poly-ICLC just prior to use.

### Example 8

### Formulation testing

Cloudiness or precipitation was seen with certain peptides in the peptide pool solution under some conditions. The effect of weak buffers on peptide solubility and stability was therefore evaluated.

It was found that the mixing of poly-ICLC and the peptide pool (in D5W with DMSO) sometimes resulted in cloudiness or precipitation, possibly due to the low pH of the poly-ICLC solution, particularly for hydrophibic peptides. In order to raise the pH of the peptide solution, buffers were tested and the effect on peptide solubility was evaluated. Based on initial testing, citrate and succinate buffers were tested.

It was found that improved solubility was seen for 3 of 4 peptides which had solubility issues in D5W alone. Based on this initial observation, 19 additional peptides were evaluated with citrate or succinate, and 4 further peptides with succinate alone. It was found that solutions of 18 of the 19 tested peptides were clear when using either sodium citrate (where tested) or sodium succinate as buffer (none of the four peptides evaluated in succinate alone demonstrated cloudiness).

Concentrations of 2 mM to 5 mM succinate were found to be effective. Recovery of peptide was improved for one peptide in succinate buffer but not in citrate buffer. Depending on the peptide pool and the concentration of succinate buffer used, pH for the peptide solutions in D5W /succinate ranged from about 4.64 to about 6.96.

After evaluation of a total of 27 peptides (including initial difficult to solubilize group of 4 peptides), it was found that one peptide reproducibly showed cloudiness in all conditions, and one additional peptide showed slight cloudiness but was fully recoverable upon filtration. Both of these two peptides had high hydrophobicity.

In general, it was found that peptides that are clear upon dilution to 2 mg/ml in D5W with succinate buffer retain clarity upon mixing with other peptides (this is generally true for peptides in D5W alone).

In a representative procedure, peptides were weighed and corrected for % peptide content, and then dissolved in DMSO to a concentration of 50 mg/mL. The DMSO/Peptide solution was then diluted with 5mM sodium succinate in D5W to a 2mg/mL peptide concentration.

Additional peptide solubility conditions were tested. Peptides CS6709, CS6712, CS6720, CS6726, and CS6783 were weighed at approximately 10 mg each. The peptides were then dissolved in approximately 200 µL USP grade DMSO to obtain a 50 mg/mL concentration for each peptide. Applicants observed that peptide CS6709 at 10.02 mg did not fully dissolve in the 200 µL amount of DMSO that was calculated to provide 50 mg/mL. The sample appeared to be cloudy. Additional 50 µL increments of DMSO were added to Peptide CS6709 up to 400µL; for a total of 600 µL of DMSO. CS6709 went into solution (clear) when the amount of DMSO reached 600 µL, the concentration was at 16.67 mg/mL.

To dilute the peptides to 400µg, a PBS pH 7.4 solution without potassium was prepared. All 5 DMSO peptide samples (50 mg/mL) were placed in a single vial for dilution to 400 µg/mL. Each DMSO peptide was added to the vial at 40 µL, except for CS6709 which was at a concentration of 16.67mg/mL. The volume of CS6709 added to the single vial was 120µL. The samples were diluted to 400 µg by adding 4.72 mL PBS pH 7.4. Upon addition of the PBS pH 7.4, it was observed that one or more of the peptides had precipitated out.

To determine which of the peptides precipitated, Applicants followed the matrix in Table 5 below using very small amounts (10-20µL) of the DMSO dissolved peptides and adding these peptides to the various liquids.

**Table 5: Peptide Diluent Matrix**

| Peptide | Liposome | PBS pH 7.4 | Water | 10% Sucrose | D5W (5% Dextrose USP Grade Inj) |
|---|---|---|---|---|---|
| CS6709 | NP | NP | NP | NP | NP |
| CS6712 | NP | NP | NP | NP | NP |
| CS6720 | NP | NP | NP | NP | NP |
| CS6726 | NP | NP | NP | NP | NP |
| CS6783 | P | P | NP | NP | NP |

| | | | | | |
|---|---|---|---|---|---|
| P = precipitation; NP = no precipitation | | | | | |

CS6783 was found to precipitate when PBS pH 7.4 was added as a diluent to the peptide mixture. The Injectable USP grade D5W is a diluent substitute for the PBS pH 7.4.

In addition, Applicants tested a small amount of each peptide (< 1 mg) to see if any of the 5 peptides could be dissolved in D5W without using DMSO. Peptides CS6709, CS6712, CS6720, and CS6726 could be dissolved directly in D5W. CS6783 could not be dissolved using D5W.

### Example 9

### Formulation

Formulations for each patient include up to 20 peptides produced individually as immunogens. For vaccination, four pools (up to 5 peptides each) are prepared for injection into separate sites targeting distinct parts of the lymphatic system as discussed herein. The individual peptides are weighed, dissolved in DMSO at high concentration, diluted with 5% dextrose in water (D5W) and sodium succinate (4.8- 5 mM) and mixed in four pools. The individual pools are filtered through a 0.2 µm filter to reduce bioburden, aliquoted into vials and frozen. The frozen vials are stored frozen until use.

As described herein, the set of patient-specific peptides constituting the drug substances are individually prepared, lyophilized, tested and released, and stored following manufacture. To prepare these peptides for injection, four groups comprised of up to 5 different peptides each are identified for pooling.

### Example 10

### Preparation of vaccines

**Weighing and Dissolution:** Based on gross weight and peptide content, 15 mg (net weight) or slightly more of each individual peptide are weighed and 100% USP Grade DMSO (2:250 µl) is added to achieve a final peptide concentration of 50 mg/ml. Based on developmental studies, >95% of the dissolved peptides demonstrate clarity at this point.

**Dilution and Mixing:** USP Grade D5W containing 5 mM Sodium Succinate (D5W /Succ) is prepared and filtered (0.2 J..tm) for use as diluent. 250 µl each dissolved peptide is diluted with D5W /Succ to reduce the peptide concentration to 2 mg peptide/ml and adjust pH to approximately ~6.0. Any peptides that do not demonstrate a clear solution are replaced with another peptide (or D5W/Succinate solution only if no additional peptides are available). 5.5 ml of each diluted peptide solution is then combined into a single 5-peptide containing pool with each peptide at a concentration of 400 µg peptide/ml. The first of two 0.2 µm membrane filtration steps are then performed. Each pool is drawn into a 60 ml Becton Dickson (or equivalent) syringe fitted with a leur lock tip and an 18 gauge blunt needle. The needle is removed and replaced with a 25 mm PALL PES (Polyether sulfone) 0.2 µm membrane filter (PALL Catalog HP1002). The contents of the syringe are transferred through the filter into a 50 ml sterile polypropylene tube (Falcon# 352070 or equivalent). An aliquot of each pool is removed for testing and the remainder frozen at -80°C. The remainder of each individual diluted peptide is stored at -20°C until all analysis is complete.

**Shipping:** The frozen peptide pools are shipped using validated shipping containers and overnight air.

**Filtration and Storage:** The frozen pools are thawed and transferred to a biosafety cabinet. A 2 ml sample from the thawed pool is tested for sterility and endotoxin testing. The remaining bulk solution are processed for a second of two 0.2 µm membrane filtration steps. The bulk pooled peptide is drawn into a Becton Dickinson (or equivalent) 60 ml syringe fitted with a luer-lock tip and an 18 gauge blunt needle. The needle is removed and replaced with a 25 mm PALL PES (Polyether sulfone) 0.2 µm membrane filter (PALL Catalog HP1002). The contents of the syringe are transferred through the filter into a 50 ml sterile polypropylene tube (Falcon# 352070 or equivalent). 1.5 ml aliquots of the peptide solution are then transferred aseptically into fifteen pre-labeled sterile 1.8 ml Nunc Cryo vials (Cat #375418). The vials are capped with one of 4 color-coded caps. A different color-coded cap is used for each of the 4 pools of peptides for a single patient to assist identification. The vials are labeled with the patient's name, medical record number study number, original product/sample alphanumeric identifier and the unique alphanumeric identifier (A-D). All vials are frozen at -80°C. The remaining frozen vials are stored until all release testing has passed acceptance criteria. Patients are not scheduled for immunization until all release testing is complete and product is released to the pharmacy.

Alternatively, on each day of immunization, one set (four) of vials which have not yet been subjected to sterilizing filtration within a biosafety cabinet as described herein are thawed and transferred to a biosafety cabinet. The contents of each vial are withdrawn into separate syringes. A 0.2 µm sterilizing filter is attached and the contents transferred through the filter into a sterile vial. The filter is removed and checked for integrity. 0.75 ml of the peptide mixture is then withdrawn using a sterile syringe and mixed by syringe-to-syringe transfer with 0.25 ml poly-ICLC (Hiltonol^{®}).

**Analysis:** Three tests (Appearance, Identity and Residual Solvents) are conducted as in-process tests on an aliquot of the pooled peptides. Endotoxin is tested on an aliquot of the thawed peptide pool prior to final filtration. Sterility is analyzed on the combined samples from two vials of the final product. This approach is taken to assure that the key biochemical information (peptide solubility, identity of each peak in each pool and levels of any residual solvents) is available prior to conducting the final filtration. Upon receipt of pooled and filtered bulk peptide pools, endotoxin testing and culturing for microorganisms is performed to evaluate microbiological purity. Meeting the endotoxin specification is required for product use. Any positive results in the microbial culture test is investigated for impact on product use. The key safety test, sterility, is conducted on vialed samples after final filtration and vialing, the samples closest to patient use.

### Example 11

### Administration

Following mixing with the personalized neo-antigenic peptides/polypeptides, the vaccine (e.g., peptides + poly-ICLC) is to be administered subcutaneously.

**Preparation of personalized neo-antigenic peptides/polypeptides pools:** peptides are mixed together in 4 pools of up to 5 peptides each. The selection criteria for each pool is based on the particular MHC allele to which the peptide is predicted to bind.

**Pool Composition:** The composition of the pools will be selected on the basis of the particular HLA allele to which each peptide is predicted to bind. The four pools are injected into anatomic sites that drain to separate lymph node basins. This approach was chosen in order to potentially reduce antigenic competition between peptides binding to the same HLA allele as much as possible and involve a wide subset of the patient's immune system in developing an immune response. For each patient, peptides predicted to bind up to four different HLA A and B alleles are identified. Some neoORF derived peptides are not associated with any particular HLA allele. The approach to distributing peptides to different pools is to spread each set of peptides associated with a particular HLA allele over as many of the four pools as possible. It is highly likely there are situations where there are more than 4 predicted peptides for a given allele, and in these cases it is necessary to allocate more than one peptide associated with a particular allele to the same pool. Those neoORF peptides not associated with any particular allele are randomly assigned to the remaining slots. An example is shown below:

| | | |
|---|---|---|
| A1 | HLAA0101 | 3 peptides |
| A2 | HLA A1101 | 5 peptides |
| B1 | HLA B0702 | 2 peptides |
| B2 | HLA B6801 | 7 peptides |
| X | NONE (neoORF) | 3 peptides |

| Pool # | 1 | 2 | 3 | 4 |
|---|---|---|---|---|
| | B2 | B2 | B2 | B2 |
| | B2 | B2 | B2 | A2 |
| | A2 | A2 | A2 | A2 |
| A1 | A1 | A1 | B1 | |
| B1 | X | X | X | |

Peptides predicted to bind to the same MHC allele are placed into separate pools whenever possible. Some of the neoORF peptides may not be predicted to bind to any MHC allele of the patient. These peptides are still utilized however, primarily because they are completely novel and therefore not subject to the immune-dampening effects of central tolerance and therefore have a high probability of being immunogenic. NeoORF peptides also carry a dramatically reduced potential for autoimmunity as there is no equivalent molecule in any normal cell. In addition, there can be false negatives arising from the prediction algorithm and it is possible that the peptide contains a HLA class II epitope (HLA class II epitopes are not reliably predicted based on current algorithms). All peptides not identified with a particular HLA allele are randomly assigned to the individual pools. The amounts of each peptide are predicated on a final dose of 300 µg of each peptide per injection.

For each patient, four distinct pools (labeled "A", "B", "C" and "D") of 5 synthetic peptides each are prepared by the manufacturer and stored at -80°C. On the day of immunization, the complete vaccine consisting of the peptide component(s) and poly-ICLC is prepared in the research pharmacy. One vial each (A, B, C and D) is thawed at room temperature and moved into a biosafety cabinet for the remaining steps. 0.75 ml of each peptide pool is withdrawn from the vial into separate syringes. Separately, four 0.25 ml (0.5 mg) aliquots of poly-ICLC is withdrawn into separate syringes. The contents of each peptide pool containing syringe is then gently mixed with a 0.25 ml aliquot of poly-ICLC by syringe-to-syringe transfer. The entire one ml of the mixture is used for injection. These 4 preparations are labeled "study drug A", "study drug B", "study drug C", and "study drug D".

On each day of immunization, patients are subcutaneously injected with up to four pools of personalized neoantigen peptides mixed with poly-ICLC (Hiltonol^{®}),

The injection volume for each mixture of peptides and Hiltonol^{®} is 1 ml.

Each pool of peptides consists of up to 5 peptides, each at a concentration of 400 µg/ml.

The composition of the peptide pool is:
Up to five peptides each at a concentration of 400 µg/ml.
4% DMSO
4.8- 5% dextrose in water
4.8 - 5 mM Sodium Succinate

Hiltonol^{®}consists of:
2 mg/ml poly I: poly C
1.5 mg/ml poly-L-Lysine
5 mg/ml sodium carboxymethylcellulose
0.9% sodium chloride

Each 1 ml injection volume consists of 0.75 ml of one of the four peptide pools mixed with 0.25 ml Hiltonol^{®}. Following mixing, the composition is:
Up to five peptides each at a concentration of 300 µg/ml.
≤3% DMSO
3.6-3.7% dextrose in water
3.6-3.7 mM Sodium Succinate
0.5 mg/ml poly I: poly C
0.375 mg/ml poly-L-Lysine
1.25 mg/ml sodium carboxymethylcellulose
0.225% sodium chloride

**Injections:** At each immunization, each of the 4 study drugs is injected subeutaneously into one extremity. Each individual study drug is administered to the same extremity at each immunization for the entire duration of the treatment (i.e. study drug A will be injected into left arm on day 1, 4, 8 etc., study drug B will be injected into right arm on days 1, 4, 8 etc.). Alternative anatomical locations for patients who axe status post complete axillary or inguinal lymph node dissection are the left and right midriff, respectively.

Vaccine is administered following a prime/boost schedule. Priming doses of vaccine is administered on days 1, 4, 8, 15, and 22 as shown herein. In the boost phase, vaccine is administered on days 85 (week 13) and 169 (week 25).

All patients receiving at least one dose of vaccine is evaluated for toxicity. Patients are evaluated for immunologic activity if they have received all vaccinations during the induction phase and the first vaccination (boost) during the maintenance phase.

### Example 12

### Short-term Room Temperature Stability of Final Dosage Form

**Peptide Stability.** A peptide pool (Pool 3) consisting of the five peptides shown in Table 6 below was prepared by dissolution in DMSO and dilution with D5W /Succinate (2 mM) to 2 mg/ml and pooling to a final peptide concentration of 400 µg per ml and a final DMSO concentration of 4%. After preparation, peptides were filtered with a 25mm Pall PES filter (Cat# 4612) and dispensed into Nunc Cryo vials(# 375418) in one ml aliquots.

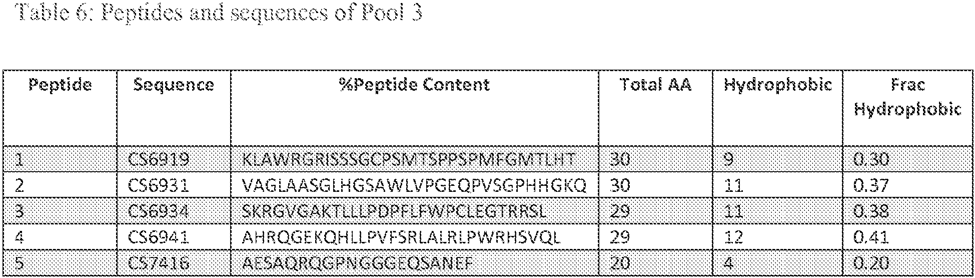

Three samples were prepared by mixing 0.75 ml of Pool3 with 0.25 ml Hiltonol^{®}as planned for the dosage form preparation. The samples were then left at room temperature for 0, 4 and 6 hours and analyzed by RP-HPLC (Table 7). No change was noted for 4 of the 5 peptides. As slight increase in a second peak associated with peptide CS6919 was noted, increasing from 14% to 17% and 18% at 4 and 6 hours, respectively. As noted in a -20°C stability study, peptides CS6919 and CS6934 (both represented in Pool4) can form a heterodimer (as shown by mass spectrometry) which elutes at the position of this impurity. Recovery of all peptides was above 90%, indicating no breakdown and loss of any peptides in the final dosage form after 6 hour room temperature incubation.

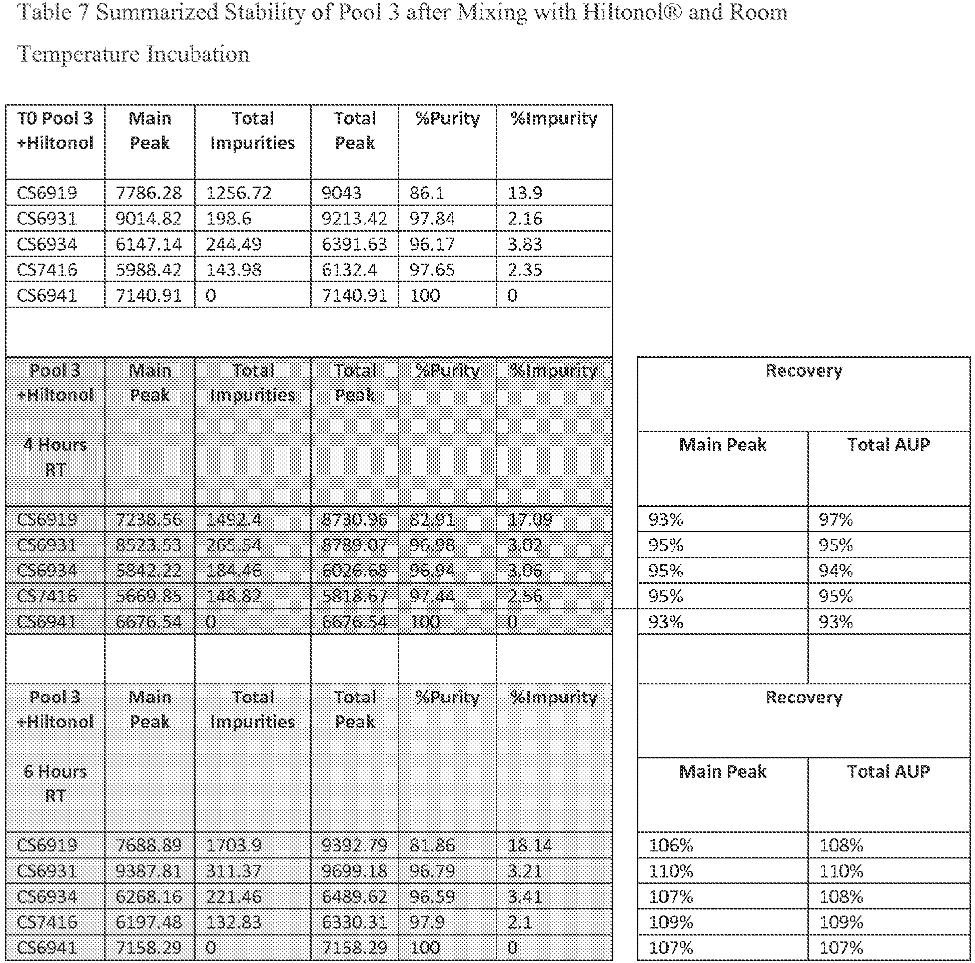

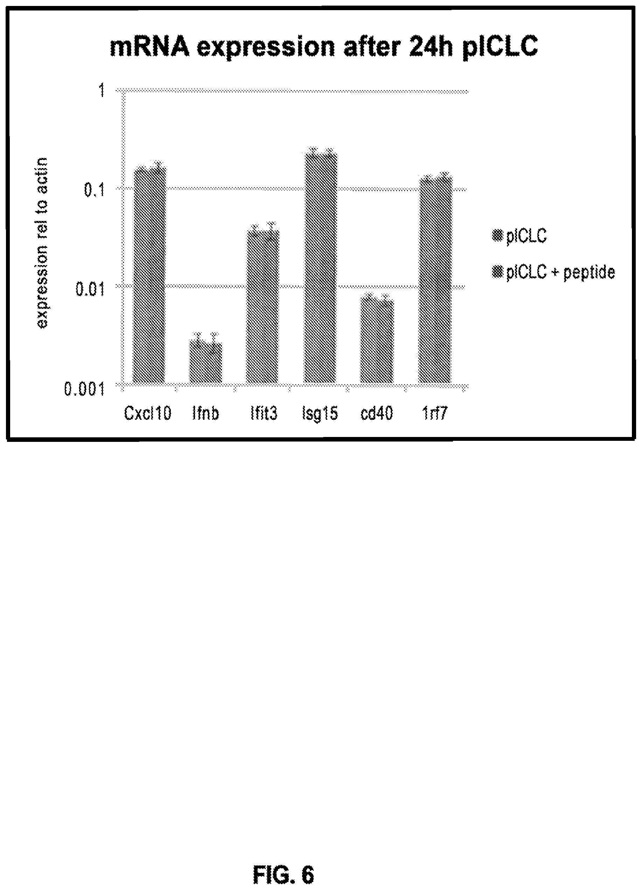

**Poly-ICLC Stability.** In a second study, another peptide pool (Pool4) was used, mixed with Hiltonol^{®}(0.75 ml peptide pool + 0.25 ml Hiltonol^{®}) and stored at room temperature for 6 hours. The room temperature incubated peptide + Hiltonol^{®} mix and Hiltonol^{®} alone (that was stored continuously at 4°C), were then diluted to 20 ug/ml poly-ICLC and assayed for TLR stimulation using mouse dendritic cells according to published methods. After 24 hour stimulation, quantitative PCR was used to assess the levels of induction of a number of key immune markers as shown in FIG. 6. There was no difference in the stimulatory capability of poly-ICLC after 6 hour room temperature with peptide pools in the final formulation, indicating that Hiltonol^{®} was not affected by any formulation components (DMS0[4%], D5W, 5 mM Succinate, peptides) and was stable in the final dosage form for up to 6 hours at room temperature.

### Example 13

### Lyophilization of the Final Formulation Form

The formulation for peptides is as following: Each pool of peptides consists of up to 5 peptides, each at a concentration of 400 µg/ml. The composition of the peptide pool is:
Up to five peptides each at a concentration of 400 µg/ml
4 - 8% DMSO
4.6 - 4.8% dextrose in water
5mM Sodium Succinate

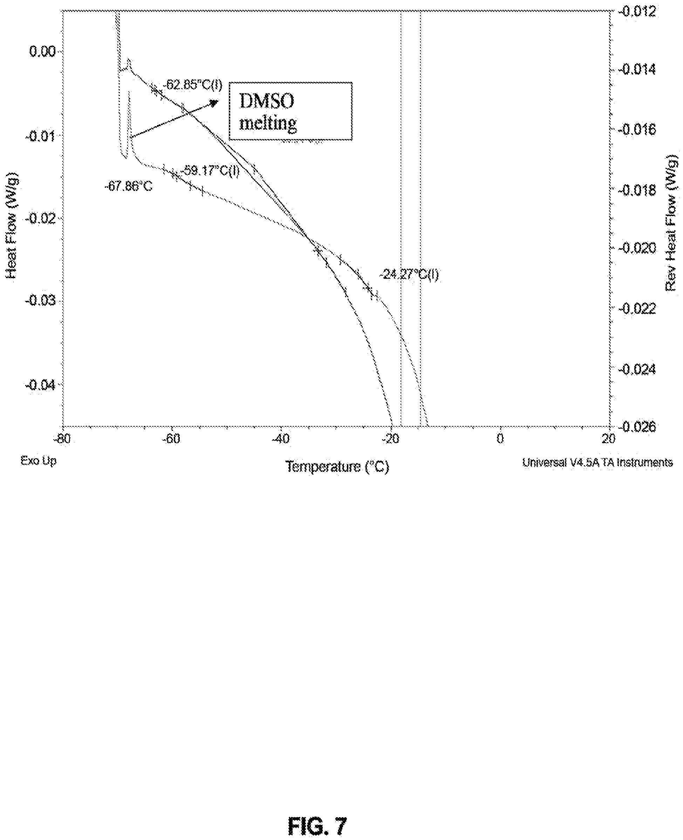

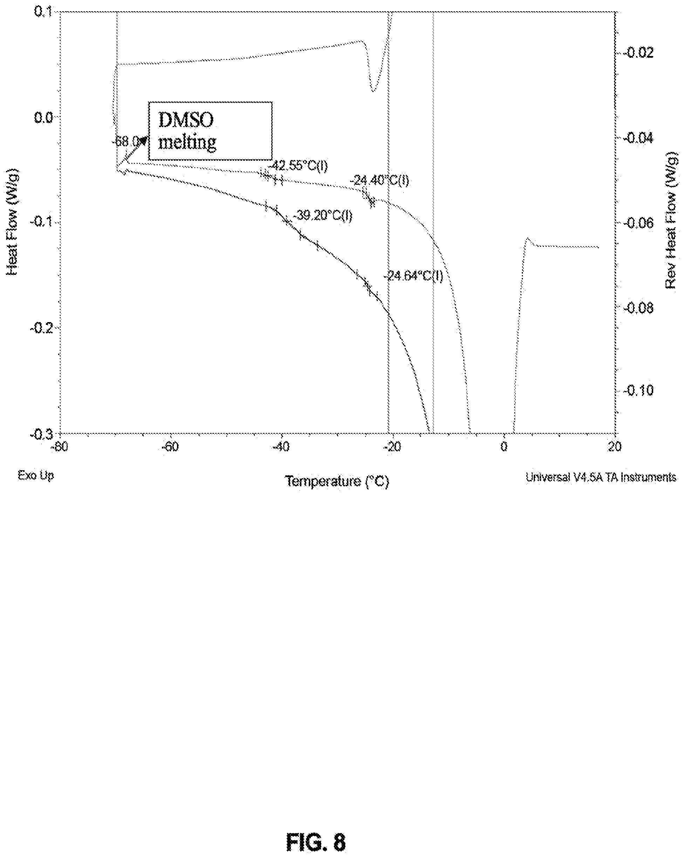

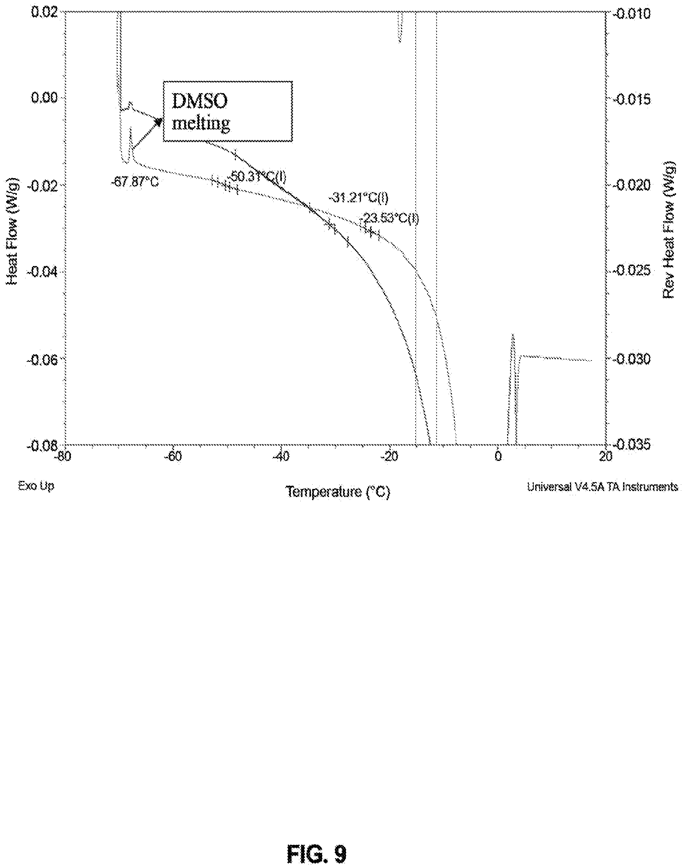

The bulking agent that is used for stabilization is Dextrose in water (D5W). The final formulation is based on the thermal properties of the formulation matrix. Modulated differential scanning calorimetry (MDSC) data suggested the presence of two glass transition temperatures (Tg') at -24°C and -56°C respectively and an exothermic reaction at -67°C due to melting of DMSO. Based on the literature, the glass transition of D5W is -43°C. The MDSC data suggests that the presence of DMSO further reduces the glass transition temperature. Based on this information, the lyophilization feasibility of peptides was checked using two additional bulking agents, Sucrose and Trehalose. The following formulations were assessed with MDSC analysis (FIG. 7-9):
1. 5% D5W and 0.8% DMSO
2. 10% Sucrose and 0.8% DMSO
3. 10% Trehalose and 0.8% DMSO

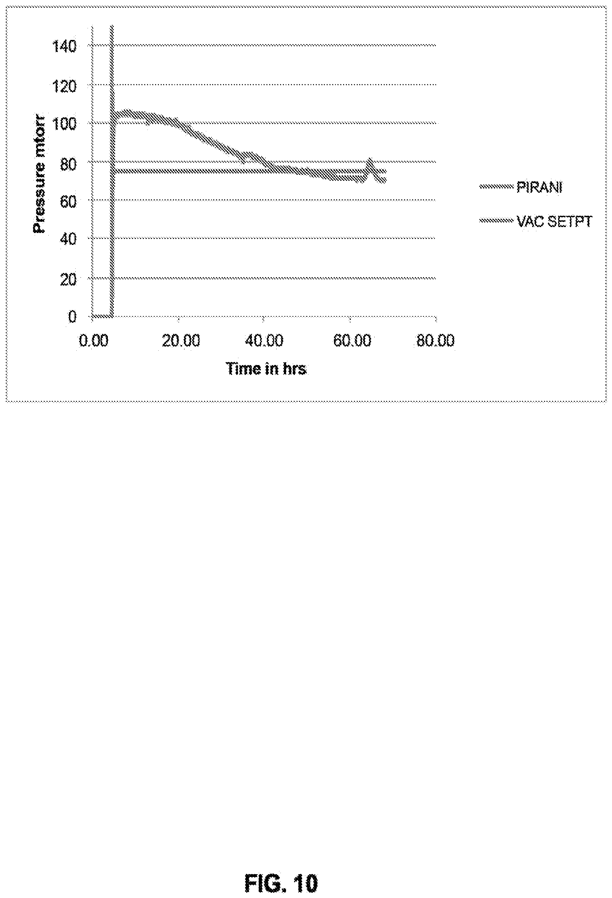

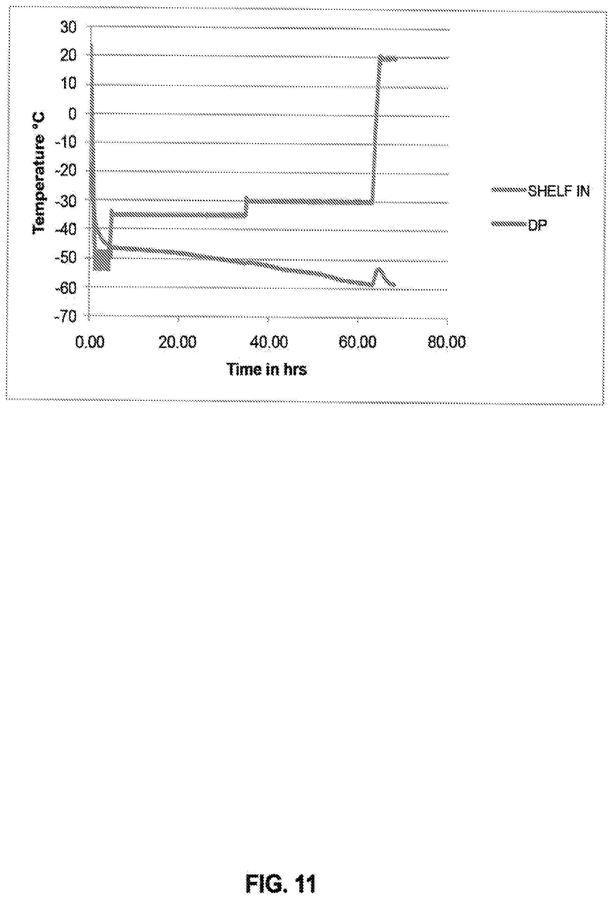

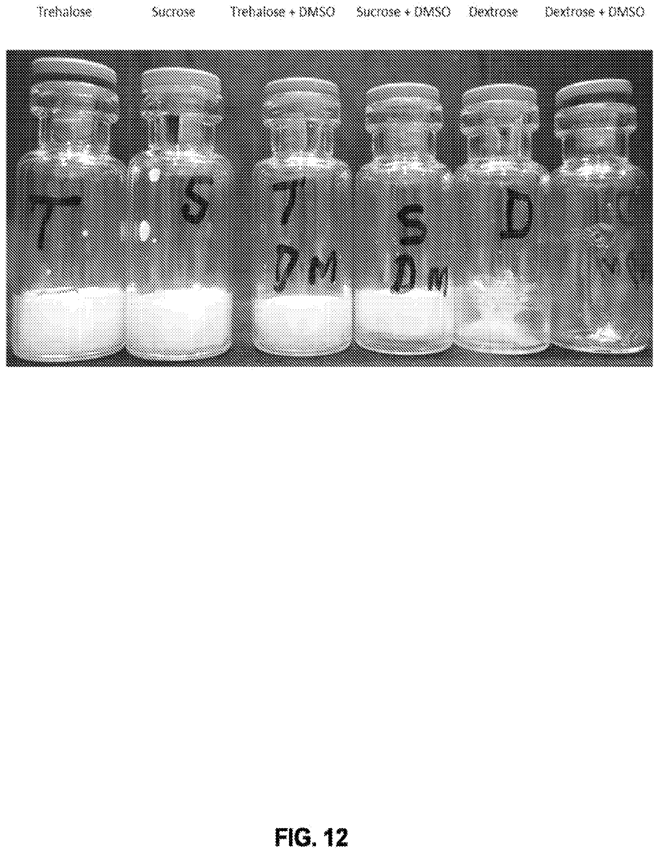

The above formulations were lyophilized using a conservative lyo cycle by freezing - 50°C for 3 hrs, primary drying at -35°C at 75 mtorr for 30 hrs and at-30°C for 30 hrs (FIG. 10 and 11). The formulation containing D5W-DMSO collapsed completely, though partial cake is seen for the formulation containing D5W alone. The lyophilization results suggest that in presence of 0.8 % DMSO, the formulation containing trehalose or sucrose is more compatible for lyophilization than formulation containing dextrose (FIG. 12).

The samples (25 µL) were analyzed by MDSC using the following program. The following parameters were used to monitor thermal events:
1. Equilibrate at 20.00 °C
2. Isothermal for 5.00 min
3. Modulate +/- 1.00 °C every 60 seconds
4. Data storage: ON
5. Ramp 1.00 °C/min to -70 °C
6. Equilibrate at -70 °C
7. Isothermal for 5.00 min
8. Ramp 1.00 °C/min to 20.00 °C
9. Equilibrate at 20.00 °C
10. Data storage: OFF
11. Isothermal for 5 minutes
12. End of Method

**Lyophilization.** MDSC was used to determine the glass transition temperature (T_{g}) which is used to select the primary drying and freezing temperature of the products (Table 8 and FIG. 7-9). The data indicates that melting of DMSO occurs around -68°C in all formulations. There were two glass transitions for all 3 formulations. The formulation containing dextrose, trehalose or sucrose has the lowest heat flow glass transition of -59°C, -42°C and -50°C respectively suggesting that it is difficult to lyophilize the formulation containing D5W-DMSO without collapse/melt.

**Table 8: MDSC analysis of 10% Sucrose and 0.8% DMSO**

| Formulations | Freezing temp(°C) | melting(°C) | DMSO melting(°C) | Tg'1(°C) | Tg'2(°C) |
|---|---|---|---|---|---|
| 5% D5W-0.8% DMSO heat | -18.2 | -0.38 | -67.86 | -24.27 | -59.17 |
| flow | | | | | |
| 5% D5W-0.8% DMSO reverse heat flow | N/A | N/A | NA | -33.31 | -62.86 |
| 10% Trehalose-0.8% DMSO heat flow | -12.64 | -1.25 | -68.06 | -24.4 | -42.55 |
| 10% Trehalose-0.8% DMSO reverse heat flow | N/A | N/A | NA | -24.4 | -39.2 |
| 10% Sucrose-0.8% DMSO heat flow | -11.36 | -0.26 | -67.87 | -23.53 | -50.31 |
| 10% Sucrose-0.8% DMSO reverse heat flow | N/A | N/A | NA | -31.21 | N/A |

Lyophilization was initially tried with Nunc vials, and it was found that the configuration of the nunc vials was not adequate to lyophilize the formulation matrix. One ml of a 5% D5W and 0.8% DMSO formulation in four 1.8 mL sterile Nunc vials (Thermo Scientific) was lyophilized using the lyophilization cycle (Freezing to -50°C and hold for 2 hrs, primary drying at -15°C for 20 hrs at 75 m torr and 8 hrs of secondary drying at 20°C with 75 m torr pressure). It was observed that there was no cake in the vials and the liquid residual DMSO and D5W in the form of small liquid droplets were noticed at the bottom of the Nunc vials.

The flint vial suitable for lyophilization was chosen to determine the feasibility of lyophilization of the lead formulation. Five vials containing 1.5 ml of each formulation were filled in 3 mL 13 mm flint vials and partially closed with a 13 mm lyo stopper and kept in the middle shelf of Lyostar II for lyophilization

It is difficult to lyophilize a formulation having a glass transition below -50°C. Based on the glass transition temperature, the following conservative lyophilization parameters were set for lyophilization (Table 9). The results obtained on pressure profile and temperature profiles are presented in FIG. 10 and 11 respectively. The pirani pressure reached below shelf set pressure during primary and secondary drying suggesting there is no moisture in the chamber (FIG. 10) and the lyophilization cycle is complete.

**Table 9: Lyophilization parameters of placebo formulation of peptides containing DMSO and Tehalose, Sucrose or D5W.**

| **Step** | **Temperature** | **Pressure** | **Hold Time(minutes)** | **Ramp Rate** | **Ramp/Hold Time(minor hr)** |
|---|---|---|---|---|---|
| Load | 20°C | atmosphere | N/A | N/A | |
| Freezing | -50°C | N/A | N/A | 1°C/min | 70 (Ramp) |
| Freezing | -50°C | N/A | 120 | N/A | 180 (Hold) |
| Primary drying | -35°C | 75 m torr | 1800 | 1°C/min | 15 (Ramp) |
| Primary drying | -30°C | 75 m torr | Till pirani reaches 75 m torr (1800 min) | 1°C/min | 5 ramp |
| Secondary drying | 20°C | 75 m torr | N/A | 1°C/min | 50 (ramp) |
| Secondary drying | 20°C | 75 m torr | Till pirani reaches 75 m torr (1800 min) | NA | Till pirani reaches 75 m torr (220 min) |
| Backfill to 600 Torr under Nitrogen, and stopper, Bring to 760 (Atmos), crimp and seal, | | | | | |

**Physical appearance of the cake.** The formulation containing D5W and DMSO is completely collapsed and melted, whereas the formulation containing Trehalose-DMSO or Sucrose-DMSO has white amorphous cake with slight collapse (FIG. 12).

## Claims

1. A pharmaceutical composition comprising:
(a) at least one neo-antigenic peptide or a pharmaceutically acceptable salt thereof, wherein the at least one neo-antigenic peptide ranges from 5 to 50 amino acids in length;
(b) a pH modifier that is a base, wherein the pH modifier is succinate or citrate and wherein the pH modifier is present in the composition at a concentration from 1 mM to 10 mM; and
(c) a pharmaceutically acceptable carrier comprising
i. water;
ii. dextrose, sucrose or trehalose; and
iii. 1-4% dimethylsulfoxide.

2. The pharmaceutical composition according to claim 1, wherein the pharmaceutical composition is a vaccine composition.

3. The pharmaceutical composition according to claim 1 or claim 2, wherein the pharmaceutical composition is for intravenous or subcutaneous administration.

4. The pharmaceutical composition according to any one of claims 1-3, wherein the pharmaceutical composition comprises at least two, three, four or five neo-antigenic peptides.

5. The pharmaceutical composition according to any one of claims 1-4, wherein the at least one neoantigenic peptide ranges from 15 to 35 amino acids in length.

6. The pharmaceutical composition according to any one of claims 1-5, wherein the pH modifier is present in the composition at a concentration from 1.5 mM to 7.5 mM, 2.0 to 6.0 mM, or 3.75 to 5.0 mM.

7. The pharmaceutical composition according to any one of claims 1-6, wherein the pH modifier is succinate.

8. The pharmaceutical composition according to any one of claims 1-7, wherein the pH modifier is sodium succinate, preferably disodium succinate.

9. The pharmaceutical composition according to any one of claims 1-8, wherein the pharmaceutically acceptable carrier comprises dextrose, preferably wherein the pharmaceutically acceptable carrier comprises 5% dextrose.

10. The pharmaceutical composition according to any one of claims 1-8, wherein the pharmaceutically acceptable carrier comprises trehalose, preferably wherein the pharmaceutically acceptable carrier comprises 10% trehalose.

11. The pharmaceutical composition according to any one of claims 1-8, wherein the pharmaceutically acceptable carrier comprises sucrose, preferably wherein the pharmaceutically acceptable carrier comprises 10% sucrose.

12. The pharmaceutical composition according to any one of claims 1-11, wherein the pharmaceutical composition comprises between 50/µg and 1.5mg of the at least one neoantigenic peptide or a pharmaceutically acceptable salt thereof.

13. The pharmaceutical composition according to any one of claims 1-12, wherein the pharmaceutical composition further comprises an immunomodulator or adjuvant, preferably wherein the immunomodulator or adjuvant is selected from the group consisting of 1018 ISS, aluminum salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, Juvlmmune, LipoVac, MF59, monophosphoryl lipid A, Montanide IMS 1312, Montanide ISA 206, Montanide ISA 50V, Montanide ISA-51, OK-432, OM-174, OM-197-MP-EC, ONTAK, PepTel^{®}, vector system, PLGA microparticles, resiquimod, SRL172, Virosomes and other Virus-like particles, YF-17D, VEGF trap, R848, beta-glucan, Pam3Cys, and Aquila's QS21 stimulon.

14. The pharmaceutical composition according to any one of claims 1-13, wherein the pharmaceutical composition is lyophilizable.

15. A method of preparing a neo-antigenic peptide solution for a neoplasia vaccine, the method comprising:
(a) preparing a solution comprising at least one neo-antigenic peptide or a pharmaceutically acceptable salt thereof, wherein the at least one neo-antigenic peptide ranges from 5 to 50 amino acids in length; and
(b) combining the solution comprising at least one neo-antigenic peptide or a pharmaceutically acceptable salt thereof with a solution comprising a pharmaceutically acceptable salt of succinic acid or citric acid, thereby preparing a peptide solution for a neoplasia vaccine,
wherein the pharmaceutically acceptable salt of succinic acid or citric acid is present in the peptide solution at a concentration from 1 mM to 10 mM and wherein the peptide solution comprises water; dextrose, sucrose or trehalose; and 1-4% dimethylsulfoxide; and
preferably
(i) wherein the solution comprising at least one neo-antigenic peptide or a pharmaceutically acceptable salt thereof comprises at least two or at least three, or four, or five neo-antigenic peptides;
(ii) wherein the peptide solution for a neoplasia vaccine comprises water, dextrose, 1 mM to 10 mM succinate, and 1-4% dimethylsulfoxide; or
(iii) further comprising, after the step of combining, filtering the peptide solution for a neoplasia vaccine; and
preferably wherein the peptide solution for a neoplasia vaccine is lyophilizable.

16. A method of preparing a neoplasia vaccine, the method comprising:
(a) preparing a neo-antigenic peptide solution according to the method of claim 15; and
(b) combining the neo-antigenic peptide solution with a solution of an immunomodulator or adjuvant, thereby preparing a neoplasia vaccine,
preferably wherein the immunomodulator or adjuvant is selected from the group consisting of 1018 ISS, aluminum salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, Juvlmmune, LipoVac, MF59, monophosphoryl lipid A, Montanide IMS 1312, Montanide ISA 206, Montanide ISA 50V, Montanide ISA-51, OK-432, OM-174, OM-197-MPEC, ONTAK, PepTel^{®}, vector system, PLGA microparticles, resiquimod, SRL172, Virosomes and other Virus-like particles, YF-17D, VEGF trap, R848, beta-glucan, Pam3Cys, and Aquila's QS21 stimulon.

17. A pharmaceutical composition according to any one of claims 1-14 for use in treating a neoplasia, wherein said treating comprises administering the pharmaceutical composition to a subject diagnosed as having a neoplasia, preferably further comprising administering a second, third or fourth pharmaceutical composition according to any one of claims 1-14 to the subj ect.

18. A vaccination or immunization kit comprising:
(a) a composition according to any one of claims 1-12, wherein the composition is separately packaged and freeze-dried; and
(b) a solution for the reconstitution of the freeze-dried composition, preferably wherein the solution contains an adjuvant.

## Patentansprüche

1. Eine pharmazeutische Zusammensetzung, die Folgendes beinhaltet:
(a) mindestens ein neoantigenes Peptid oder ein pharmazeutisch akzeptables Salz davon, wobei das mindestens eine neoantigene Peptid eine Länge im Bereich von 5 bis 50 Aminosäuren hat;
(b) einen pH-Modifikator, der eine Base ist, wobei der pH-Modifikator Succinat oder Citrat ist und wobei der pH-Modifikator in der Zusammensetzung in einer Konzentration von 1 mM bis 10 mM vorhanden ist; und
(c) einen pharmazeutisch akzeptablen Träger, der Folgendes beinhaltet:
i. Wasser;
ii. Dextrose, Saccharose oder Trehalose; und
iii. 1-4% Dimethylsulfoxid.

2. Pharmazeutische Zusammensetzung nach Anspruch 1, wobei die pharmazeutische Zusammensetzung eine Impfstoffzusammensetzung ist.

3. Pharmazeutische Zusammensetzung nach Anspruch 1 oder Anspruch 2, wobei die pharmazeutische Zusammensetzung zur intravenösen oder subkutanen Verabreichung ist.

4. Pharmazeutische Zusammensetzung nach einem der Ansprüche 1-3, wobei die pharmazeutische Zusammensetzung mindestens zwei, drei, vier oder fünf neoantigene Peptide beinhaltet.

5. Pharmazeutische Zusammensetzung nach einem der Ansprüche 1-4, wobei das mindestens eine neoantigene Peptid eine Länge im Bereich von 15 bis 35 Aminosäuren hat.

6. Pharmazeutische Zusammensetzung nach einem der Ansprüche 1-5, wobei der pH-Modifikator in der Zusammensetzung in einer Konzentration von 1,5 mM bis 7,5 mM, 2,0 bis 6,0 mM oder 3,75 bis 5,0 mM vorhanden ist.

7. Pharmazeutische Zusammensetzung nach einem der Ansprüche 1-6, wobei der pH-Modifikator Succinat ist.

8. Pharmazeutische Zusammensetzung nach einem der Ansprüche 1-7, wobei der pH-Modifikator Natriumsuccinat, bevorzugt Dinatriumsuccinat, ist.

9. Pharmazeutische Zusammensetzung nach einem der Ansprüche 1-8, wobei der pharmazeutisch akzeptable Träger Dextrose beinhaltet, wobei der pharmazeutisch akzeptable Träger bevorzugt 5% Dextrose beinhaltet.

10. Pharmazeutische Zusammensetzung nach einem der Ansprüche 1-8, wobei der pharmazeutisch akzeptable Träger Trehalose beinhaltet, wobei der pharmazeutisch akzeptable Träger bevorzugt 10% Trehalose beinhaltet.

11. Pharmazeutische Zusammensetzung nach einem der Ansprüche 1-8, wobei der pharmazeutisch akzeptable Träger Saccharose beinhaltet, wobei der pharmazeutisch akzeptable Träger bevorzugt 10% Saccharose beinhaltet.

12. Pharmazeutische Zusammensetzung nach einem der Ansprüche 1-11, wobei die pharmazeutische Zusammensetzung zwischen 50 µg und 1,5 mg des mindestens einen neoantigenen Peptids oder eines pharmazeutisch akzeptablen Salzes davon beinhaltet.

13. Pharmazeutische Zusammensetzung nach einem der Ansprüche 1-12, wobei die pharmazeutische Zusammensetzung ferner einen Immunmodulator oder ein Adjuvans beinhaltet,
wobei der Immunmodulator oder das Adjuvans bevorzugt aus der Gruppe ausgewählt ist, die aus Folgenden besteht: 1018 ISS, Aluminiumsalzen, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, Juvlmmune, LipoVac, MF59, Monophosphoryllipid A, Montanide IMS 1312, Montanide ISA 206, Montanide ISA 50V, Montanide ISA-51, OK-432, OM-174, OM-197-MP-EC, ONTAK, PepTel^{®}, Vektorsystem, PLGA-Mikropartikeln, Resiquimod, SRL172, Virosomen und anderen virusähnlichen Partikeln, YF-17D, VEGF-Trap, R848, beta-Glucan, Pam3Cys und Aquila's QS21-Stimulon.

14. Pharmazeutische Zusammensetzung nach einem der Ansprüche 1-13, wobei die pharmazeutische Zusammensetzung lyophilisierbar ist.

15. Ein Verfahren zur Herstellung einer neoantigenen Peptidlösung für einen Neoplasieimpfstoff, wobei das Verfahren Folgendes beinhaltet:
(a) Herstellen einer Lösung, die mindestens ein neoantigenes Peptid oder ein pharmazeutisch akzeptables Salz davon beinhaltet, wobei das mindestens eine neoantigene Peptid eine Länge im Bereich von 5 bis 50 Aminosäuren hat; und
(b) Kombinieren der Lösung, die das mindestens eine neoantigene Peptid oder ein pharmazeutisch akzeptables Salz davon beinhaltet, mit einer Lösung, die ein pharmazeutisch akzeptables Salz von Bernsteinsäure oder Citronensäure beinhaltet, und somit Herstellen einer Peptidlösung für einen Neoplasieimpfstoff,
wobei das pharmazeutisch akzeptable Salz von Bernsteinsäure oder Citronensäure in der Peptidlösung in einer Konzentration von 1 mM bis 10 mM vorhanden ist und wobei die Peptidlösung Folgendes beinhaltet: Wasser; Dextrose, Saccharose oder Trehalose; und 1-4% Dimethylsulfoxid; und
bevorzugt
(i) wobei die Lösung, die mindestens ein neoantigenes Peptid oder ein pharmazeutisch akzeptables Salz davon beinhaltet, mindestens zwei oder mindestens drei oder vier oder fünf neoantigene Peptide beinhaltet;
(ii) wobei die Peptidlösung für einen Neoplasieimpfstoff Wasser, Dextrose, 1 mM bis 10 mM Succinat und 1-4% Dimethylsulfoxid beinhaltet; oder
(iii) wobei es nach dem Schritt des Kombinierens ferner das Filtrieren der Peptidlösung für einen Neoplasieimpfstoff beinhaltet; und
wobei die Peptidlösung bevorzugt für einen Neoplasieimpfstoff lyophilisierbar ist.

16. Ein Verfahren zur Herstellung eines Neoplasieimpfstoffs, wobei das Verfahren Folgendes beinhaltet:
(a) Herstellen einer neoantigenen Peptidlösung nach dem Verfahren von Anspruch 15; und
(b) Kombinieren der neoantigenen Peptidlösung mit einer Lösung eines Immunmodulators oder Adjuvans, und somit Herstellen eines Neoplasieimpfstoffs,
wobei der Immunmodulator oder das Adjuvans bevorzugt aus der Gruppe ausgewählt ist, die aus Folgenden besteht: 1018 ISS, Aluminiumsalzen, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, Juvlmmune, LipoVac, MF59, Monophosphoryllipid A, Montanide IMS 1312, Montanide ISA 206, Montanide ISA 50V, Montanide ISA-51, OK-432, OM-174, OM-197-MP-EC, ONTAK, PepTel^{®}, Vektorsystem, PLGA-Mikropartikeln, Resiquimod, SRL172, Virosomen und anderen virusähnlichen Partikeln, YF-17D, VEGF-Trap, R848, beta-Glucan, Pam3Cys und Aquila's QS21-Stimulon.

17. Eine pharmazeutische Zusammensetzung nach einem der Ansprüche 1-14 zur Verwendung beim Behandeln einer Neoplasie, wobei das Behandeln das Verabreichen der pharmazeutischen Zusammensetzung an ein Individuum beinhaltet, bei dem das Vorliegen einer Neoplasie diagnostiziert wurde, das bevorzugt ferner das Verabreichen einer zweiten, dritten oder vierten pharmazeutischen Zusammensetzung nach einem der Ansprüche 1-14 an das Individuum beinhaltet.

18. Ein Impf- oder Immunisierungskit, das Folgendes beinhaltet:
(a) eine Zusammensetzung nach einem der Ansprüche 1-12, wobei die Zusammensetzung separat verpackt und gefriergetrocknet ist; und
(b) eine Lösung zum Rekonstituieren der gefriergetrockneten Zusammensetzung, wobei die Lösung bevorzugt ein Adjuvans enthält.

## Revendications

1. Composition pharmaceutique comprenant :
(a) au moins un peptide néo-antigénique ou un sel pharmaceutiquement acceptable de celui-ci, l'au moins un peptide néo-antigénique ayant une longueur de 5 à 50 acides aminés ;
(b) un modificateur de pH qui est une base, le modificateur de pH étant un succinate ou un citrate et le modificateur de pH étant présent dans la composition à une concentration de 1 mM à 10 mM ; et
(c) un support pharmaceutiquement acceptable comprenant
i. de l'eau ;
ii. du dextrose, du saccharose ou du tréhalose ; et
iii. 1 à 4 % de diméthylsulfoxyde.

2. Composition pharmaceutique selon la revendication 1, la composition pharmaceutique étant une composition vaccinale.

3. Composition pharmaceutique selon la revendication 1 ou la revendication 2, la composition pharmaceutique étant destinée à une administration intraveineuse ou souscutanée.

4. Composition pharmaceutique selon l'une quelconque des revendications 1 à 3, la composition pharmaceutique comprenant au moins deux, trois, quatre ou cinq peptides néo-antigéniques.

5. Composition pharmaceutique selon l'une quelconque des revendications 1 à 4, dans laquelle l'au moins un peptide néo-antigénique a une longueur de 15 à 35 acides aminés.

6. Composition pharmaceutique selon l'une quelconque des revendications 1 à 5, dans laquelle le modificateur de pH est présent dans la composition à une concentration de 1,5 mM à 7,5 mM, de 2,0 à 6,0 mM, ou de 3,75 à 5,0 mM.

7. Composition pharmaceutique selon l'une quelconque des revendications 1 à 6, dans laquelle le modificateur de pH est un succinate.

8. Composition pharmaceutique selon l'une quelconque des revendications 1 à 7, dans laquelle le modificateur de pH est le succinate de sodium, préférablement le succinate de disodium.

9. Composition pharmaceutique selon l'une quelconque des revendications 1 à 8, dans laquelle le support pharmaceutiquement acceptable comprend du dextrose, préférablement dans laquelle le support pharmaceutiquement acceptable comprend 5 % de dextrose.

10. Composition pharmaceutique selon l'une quelconque des revendications 1 à 8, dans laquelle le support pharmaceutiquement acceptable comprend du tréhalose, préférablement dans laquelle le support pharmaceutiquement acceptable comprend 10 % de tréhalose.

11. Composition pharmaceutique selon l'une quelconque des revendications 1 à 8, dans laquelle le support pharmaceutiquement acceptable comprend du saccharose, préférablement dans laquelle le support pharmaceutiquement acceptable comprend 10 % de saccharose.

12. Composition pharmaceutique selon l'une quelconque des revendications 1 à 11, la composition pharmaceutique comprenant entre 50 µg et 1,5 mg de l'au moins un peptide néo-antigénique ou d'un sel pharmaceutiquement acceptable de celui-ci.

13. Composition pharmaceutique selon l'une quelconque des revendications 1 à 12, la composition pharmaceutique comprenant en outre un immunomodulateur ou un adjuvant,
préférablement dans laquelle l'immunomodulateur ou l'adjuvant est sélectionné dans le groupe constitué du 1018 ISS, de sels d'aluminium, de l'Amplivax, de l'AS15, du BCG, du CP-870,893, du CpG7909, du CyaA, du dSLIM, du GM-CSF, de l'IC30, de l'IC31, de l'Imiquimod, de l'ImuFact IMP321, de l'IS Patch, de l'ISS, de l'ISCOMATRIX, du JuvImmune, du LipoVac, du MF59, du monophosphoryl-lipide A, du Montanide IMS 1312, du Montanide ISA 206, du Montanide ISA 50V, du Montanide ISA-51, de l'OK-432, de l'OM-174, de l'OM-197-MP-EC, de l'ONTAK, du PepTel^{®}, d'un système vectoriel, de microparticules de PLGA, du resiquimod, du SRL 172, de virosomes et d'autres particules de type virus, de l'YF-17D, de VEGF trap, du R848, du bêta-glucane, du Pam3Cys et du stimulon QS21 d'Aquila.

14. Composition pharmaceutique selon l'une quelconque des revendications 1 à 13, la composition pharmaceutique étant lyophilisable.

15. Procédé de préparation d'une solution de peptide néo-antigénique pour un vaccin contre la néoplasie, le procédé comprenant :
(a) la préparation d'une solution comprenant au moins un peptide néo-antigénique ou un sel pharmaceutiquement acceptable de celui-ci, dans laquelle l'au moins un peptide néo-antigénique a une longueur de 5 à 50 acides aminés ; et
(b) la combinaison de la solution comprenant au moins un peptide néo-antigénique ou un sel pharmaceutiquement acceptable de celui-ci avec une solution comprenant un sel pharmaceutiquement acceptable d'acide succinique ou d'acide citrique, en préparant ainsi une solution peptidique pour un vaccin contre la néoplasie,
dans lequel le sel pharmaceutiquement acceptable d'acide succinique ou d'acide citrique est présent dans la solution peptidique à une concentration de 1 mM à 10 mM et dans lequel la solution peptidique comprend de l'eau ; du dextrose, du saccharose ou du tréhalose ; et 1 à 4 % de diméthylsulfoxyde ; et
préférablement
(i) dans lequel la solution comprenant au moins un peptide néo-antigénique ou un sel pharmaceutiquement acceptable de celui-ci comprend au moins deux ou au moins trois, ou quatre, ou cinq peptides néo-antigéniques ;
(ii) dans lequel la solution peptidique pour un vaccin contre la néoplasie comprend de l'eau, du dextrose, 1 mM à 10 mM de succinate et 1 à 4% de diméthylsulfoxyde ; ou
(iii) comprenant en outre, après l'étape de combinaison, un filtrage de la solution peptidique pour un vaccin contre la néoplasie ; et
préférablement dans lequel la solution peptidique pour un vaccin contre la néoplasie est lyophilisable.

16. Procédé de préparation d'un vaccin contre la néoplasie, le procédé comprenant :
(a) la préparation d'une solution de peptide néo-antigénique par le procédé selon la revendication 15 ; et
(b) la combinaison de la solution de peptide néo-antigénique avec une solution d'un immunomodulateur ou d'un adjuvant, en préparant ainsi un vaccin contre la néoplasie,
préférablement dans lequel l'immunomodulateur ou l'adjuvant est sélectionné dans le groupe constitué du 1018 ISS, de sels d'aluminium, de l'Amplivax, de l'AS15, du BCG, du CP-870,893, du CpG7909, du CyaA, du dSLIM, du GM-CSF, de l'IC30, de l'IC31, de l'Imiquimod, de l'ImuFact IMP321, de l'IS Patch, de l'ISS, de l'ISCOMATRIX, du JuvImmune, du LipoVac, du MF59, du monophosphoryl-lipide A, du Montanide IMS 1312, du Montanide ISA 206, du Montanide ISA 50V, du Montanide ISA-51, de l'OK-432, de l'OM-174, de l'OM-197-MP-EC, de l'ONTAK, du PepTel^{®}, d'un système vectoriel, de microparticules de PLGA, du resiquimod, du SRL 172, de virosomes et d'autres particules de type virus, de l'YF-17D, de VEGF trap, du R848, du bêta-glucane, du Pam3Cys et du stimulon QS21 d'Aquila.

17. Composition pharmaceutique selon l'une quelconque des revendications 1 à 14, destinée à une utilisation dans le traitement d'une néoplasie, ledit traitement comprenant l'administration de la composition pharmaceutique à un sujet diagnostiqué comme présentant une néoplasie, comprenant préférablement en outre l'administration d'une deuxième, d'une troisième ou d'une quatrième composition pharmaceutique selon l'une quelconque des revendications 1 à 14 au sujet.

18. Trousse de vaccination ou d'immunisation comprenant :
(a) une composition selon l'une quelconque des revendications 1 à 12, la composition étant emballée séparément et lyophilisée ; et
(b) une solution pour la reconstitution de la composition lyophilisée, la solution contenant préférablement un adjuvant.
